(19) Europäisches Patentamt
European Patent Office
Office européen des brevets

(11) EP 2 638 916 A1

(12) **EUROPEAN PATENT APPLICATION**

(43) Date of publication:
**18.09.2013 Bulletin 2013/38**

(21) Application number: **12159936.9**

(22) Date of filing: **16.03.2012**

(51) Int Cl.:
***A61K 39/395*** *(2006.01)* ***C07K 16/32*** *(2006.01)*
***A61P 35/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Covagen AG**
**8952 Zürich-Schlieren (CH)**

(72) Inventors:
- **Brack, Simon**
**8400 Winterthur (CH)**
- **Mourlane, Frédéric**
**4600 Olten (CH)**
- **Toller, Isabella**
**8037 Zürich (CH)**
- **Woods, Richard**
**8049 Zürich (CH)**
- **Bertschinger, Julian**
**8634 Hombrechtikon (CH)**
- **Grabulovski, Dragan**
**8049 Zürich (CH)**

(74) Representative: **Vossius & Partner**
**Siebertstrasse 4**
**81675 München (DE)**

(54) **Novel binding molecules with antitumoral activity**

(57) The present invention relates to a HER2-specific binding molecule that specifically binds to two different epitopes of HER2 comprising: (a) a first binding (poly) peptide that specifically binds to a first epitope of HER2; and (b) a second binding (poly)peptide that specifically binds to a second epitope of HER2. The present invention further relates to a nucleic acid molecule encoding the HER2-specific binding molecule of the invention, a vector comprising said nucleic acid molecule as well as a host cell or a non-human host transformed with said vector. The invention also relates to a method of producing a HER2-specific binding molecule of the invention as well as to pharmaceutical and diagnostic compositions. Moreover, the present invention also relates to the HER2-specific binding molecule, the nucleic acid molecule, the vector or the host cell of the invention for use in the treatment of tumors.

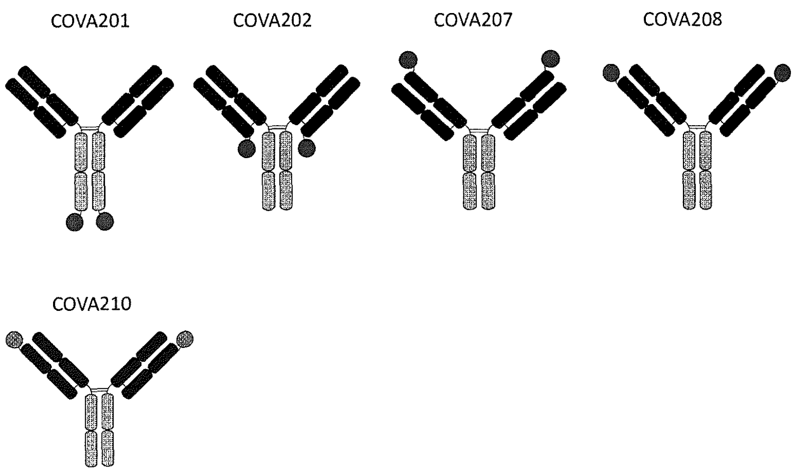


Figure 8

EP 2 638 916 A1

## Description

The present invention relates to a HER2-specific binding molecule that specifically binds to two different epitopes of HER2 comprising: (a) a first binding (poly)peptide that specifically binds to a first epitope of HER2; and (b) a second binding (poly)peptide that specifically binds to a second epitope of HER2. The present invention further relates to a nucleic acid molecule encoding the HER2-specific binding molecule of the invention, a vector comprising said nucleic acid molecule as well as a host cell or a non-human host transformed with said vector. The invention also relates to a method of producing a HER2-specific binding molecule of the invention as well as to pharmaceutical and diagnostic compositions. Moreover, the present invention also relates to the HER2-specific binding molecule, the nucleic acid molecule, the vector or the host cell of the invention for use in the treatment of tumors.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Non- immunoglobulin- derived binding reagents (collectively designated "scaffolds"; see, for example, Skerra (2000) J. Mol. Recognit. 13, 167- 187) have been suggested for use as diagnostic and therapeutic agents. More than 50 different protein scaffolds have been proposed over the past 10 to 15 years, the most advanced approaches in this field being (as summarized in Gebauer and Skerra (2009) Curr Opinion in Chemical Biology 13: 245- 255) : affibodies (based on the Z- domain of staphylococcal protein A) , Kunitz type domains, adnectins (based on the 10th domain of human fibronectin) , anticalins (derived from lipocalins) , DARPins (derived from ankyrin repeat proteins) , avimers (based on multimerized LDLR- A) , and Fynomers, which are derived from the human Fyn SH3 domain.

In general, SH3 domains are present in a large variety of proteins participating in cellular signal transduction (Musacchio et al. (1994) Prog. Biophys. Mol. Biol. 61; 283- 297) . These domains do not occupy a fixed position within proteins and can be expressed and purified independently. More than 1000 occurrences of the domain are presently known including about 300 human SH3 domains (Musacchio (2003) Advances in Protein Chemistry. 61; 211- 268) . Although there is great sequence diversity among SH3 domains, they all share a conserved fold: a compact beta barrel formed by two anti- parallel beta- sheets (Musacchio (2003) Advances in Protein Chemistry. 61; 211- 268) . Typically, SH3 domains bind to proline- rich peptides containing a PXXP core- binding motif (Ren et al. (1993) Science 259; 1157- 1161) , but examples of unconventional SH3 binding sites have also been described (Karkkainen et al. (2006) EMBO Rep. 7; 186- 191) . Most of the SH3 domains sequenced so far have an overall length of approximately 60 to 65 amino acids, but some of them may feature as many as 85 amino acids due to inserts into the loops connecting the main conservative elements of the secondary structure (Koyama et al. (1993) Cell 72 (6) ; 945- 952) . An alignment of different SH3 domains revealed conserved amino acid residues responsible for the proper structure formation as well as for the canonical proline- rich motif recognition (Larson et al. (2000) Protein Science 9; 2170- 2180) .

Upregulation of the HER2 protein is observed in breast and ovarian cancers and correlates with a poor prognosis (Slamon et al. (1987) Science, 235: 177- 182; Slamon et al. (1989) Science 244: 707- 712) . Overexpression of HER2 (frequently but not uniformly due to gene amplification) has also been observed in a range of other tumor types including carcinomas of the bladder, salivary gland, endometrium, pancreas, thyroid, kidney, lung, upper gastrointestinal tract and colon (Scholl et al. (2001) Ann Oncol, 12 Suppl 1: S81- 87; Ross JS (2011) Biomark Med 3: 307- 318; Fukushige et al. (1986) Mol Cell Biol 3: 955- 958; Cohen et al. (1989) Oncogene 1: 81- 88; Weiner et al (1990) Cancer Res 50: 421- 425; Park et al (1989) Cancer Res 23: 6605- 6609; Zhau et al (1990) Mol. Carcinog. 5: 254- 257; Aasland et al. (1988) Br J Cancer 4: 358- 363; Seliger et al (2000) Int J Cancer 87 (3) : 349- 359) . In addition, HER2 has been found to be overexpressed in prostate cancer, although at lower levels compared to breast cancer tissues (Minner et al. (2010) Clin Cancer Res 16 (5) : 1553- 1560) .

In the past, several HER2 binding proteins have been described, such as affibodies and DARPins (Wikman et al (2004) Protein Eng Des Sel 17 (5) : 455- 462; Zahnd et al (2007) 369 (4) : 1015- 1028) . Moreover, Hudziak et al. describe the generation of a panel of murine anti- HER2 antibodies (including the antibodies 4D5 and 2C4) which were characterized using a human breast tumor cell line (Hudziak et al. (1989) Mol Cell Biol 9 (3) : 1165- 1172; see also U.S. Patent No. 5, 677, 171) . Relative cell proliferation of the cells following exposure to the antibodies was determined. The authors demonstrated that the antibody 4D5 most effectively inhibited cellular proliferation. A recombinant humanized version of the murine anti- HER2 antibody 4D5 (huMAb4D5- 8, trastuzumab, Herceptin®, see also U.S. Patent No. 5, 821, 337) was approved by the US Food and Drug Administration in 1998 for the management of HER2- positive metastatic breast cancer (MBC) in combination with chemotherapy. Currently, trastuzumab is recommended as first-line treatment for patients with metastatic HER2- positive tumors, either as a single agent (limited group of patients) or in combination with endocrine therapy or chemotherapy, as well as in the adjuvant setting (Awada et al. (2012) Cancer Treat Rev, 106 (1) : 6- 13) . However, primary (intrinsic) or secondary (acquired while under treatment) resistance is frequently encountered during treatment with trastuzumab (Tsang et al. (2012) Br J Cancer 106: 6- 13) . For example, it has been observed that the rate of primary resistance to single- agent trastuzumab for HER2- overexpressing metastatic breast cancer is 66- 89%. In addition, the majority of patients who achieve an initial response to trastuzumab- based regimens develop resistance within 1 year (Nahta et al (2006) Nat Clin Pract Oncol 3 (5) : 269- 280) . Several strategies to overcome resistance to trastuzumab therapy have been described in the literature (see review Tsang et al. (2012) Br J Cancer 106: 6- 13; Awada et al. (2012) Cancer Treat Rev 106 (1) : 6- 13) .

One attractive avenue to overcome resistance is represented by using combinations of HER2 binding agents. In this context, several groups showed that the combination of two anti- HER2 antibodies inhibited the growth of human tumor cell lines *in vitro* and/or *in vivo* better than the treatment with either single antibody alone (Yamashita- Kashima et al (2011) Clin Cancer Res 17 (15) : 5060- 5070; Scheuer et al. (2009) Cancer Res 69 (24) : 9330- 9336; Lee- Hoeflich et al (2008) Cancer Res 68 (14) : 5878- 5887; Kasprzyk et al (1992) Cancer Res 52: 2771- 2776; Ben- Kasus et al. (2009) Proc Natl Acad Sci U S A 106 (9) : 3294- 3299) . Notably, increased efficacy was observed in preclinical studies where trastuzumab was combined with pertuzumab (pertuzumab is the humanized form of the mouse antibody 2C4 described in Hudziak et al. (Hudziak et al. (1989) Mol Cell Biol 9 (3) : 1165- 1172; U.S. Patent No. 5, 677, 171) ; Adams C.W. et al. (2006) Cancer Immunol Immunother, 55: 717- 727; WO2001/00245) ) , and Phase 2 clinical trials showed that the coadministration of trastuzumab and pertuzumab produced anti- tumor responses in patients who had previously experienced disease progression while receiving trastuzumab- based therapy (Baselga et al. (2010) J Clin Oncol 28: 1138- 1144) . Pertuzumab binds to domain 11 of the extracellular part of HER2, whereas trastuzumab binds to a site in domain IV of HER2, which is proximal to the membrane. Due to its binding specificity, pertuzumab was shown to prevent HER2 to form active heterodimers with other HER receptors (such as HER1, HER3 and HER4) (Agus et al (2002) Cancer Cell 2: 127- 137; Fendly et al (1990) Cancer Res 50 (5) : 1550- 1558) .

Recently, it has been shown in a Phase III clinical study that the combination of pertuzumab plus trastuzumab plus docetaxel, as compared with placebo plus trastuzumab plus docetaxel, significantly prolonged progression- free survival when used as first- line treatment for HER2- positive metastatic breast cancer patients (Baselga et al (2012) N Engl J Med 366 (2) : 109- 119) . However, the median independently assessed progression- free survival was prolonged by only 6.1 months (from 12.4 months in the control group to 18.5 months in the pertuzumab group) . Moreover, the combination of two or more biological compounds necessitates the dosing of two molecules which usually renders regulatory and clinical procedures more difficult. Furthermore, differences in pharmacokinetics and in tissue concentrations could reduce efficacy of the two antibodies. Therefore, developing improved HER2- targeting agents for the treatment of HER2- positive cancers remains a significant challenge.

Based on the improved therapeutic efficacy of antibody combinations targeting different epitopes on HER2 or EGFR (HER1) (Perera et al. (2005) Clin. Cancer Res. 11: 6390- 6399) , efforts have been undertaken to engineer multispecific EGFR and HER2 targeting proteins, which bind to different epitopes of either EGFR (WO2011/020033) or HER2 (oral presentation of Woisetschläger M., Bispecific Antibody Summit 2011, September 27, 2011, Boston, USA; slides number 5 & 15) . In WO2011/020033 fibronectin domain- based EGFR binding proteins were isolated and fused to the C- or N- terminus of either the heavy and/or light chain of the anti- EGFR monoclonal antibody 225 (also known as cetuximab (Erbitux®) ) . The EGFR binding fibronectin domains were shown to recognize a different epitope than the antibody 225. Some of the resulting fibronectin- antibody fusions were found to induce EGFR clustering and downregulation more effectively than the antibody 225 on its own.

In the presentation of Woisetschläger M. (oral presentation of Woisetschläger M., Bispecific Antibody Summit 2011, September 27, 2011, Boston, USA; slides number 5 & 15), the bispecific trastuzumab-based HER2-targeting antibody trastuzumab-Her2-1 binding to two different epitopes on HER2 was described. However, no enhanced activity of the bispecific trastuzumab-based antibody as compared to unmodified trastuzumab was observed.

Thus, despite the fact that a lot of effort has been invested into improving antitumor- therapies, there is still a need to identify novel therapeutic compounds for an improved treatment of cancer that overcome the above described disadvantages.

This need is addressed by the provision of the embodiments characterized in the claims.

Accordingly, the present invention relates to a HER2- specific binding molecule that specifically binds to two different epitopes of HER2 comprising: (a) a first binding (poly) peptide that specifically binds to a first epitope of HER2; and (b) a second binding (poly) peptide that specifically binds to a second epitope of HER2.

The term "HER2-specific binding molecule", in accordance with the present invention, relates to a binding molecule having two different binding specificities for HER2. In other words, the HER2-specific binding molecule of the present invention is capable of specifically binding to two distinct binding sites (i.e. epitopes) within said HER2 antigen. Moreover, the HER2-specific binding molecule of the present invention is capable of binding to said two different epitopes at the same time.

HER2 is defined in accordance with the pertinent art and relates to a human epidermal growth factor receptor type 2 (also referred to as HER2/neu or ErbB-2, see above), a 185-kDa receptor first described in 1984 (Schlechter et al (1984) Nature 312:513-516). Human HER2 is represented by the NCBI reference (NCBI Reference Sequence: NP_ 004439 publication date 26 Feb 2012 ) and has been described in the art, for example in Robinson et al. (2000) Oncogene 19:5548-5557 as well as the references cited herein above.

The term "(poly) peptide", as used in accordance with the present invention, describes linear molecular chains of amino acids, including single chain proteins or their fragments. The term refers to a group of molecules which comprises the group of peptides, consisting of up to 30 amino acids, as well as the group of polypeptides (also referred to herein as proteins) , consisting of more than 30 amino acids.

Furthermore, peptidomimetics of such (poly) peptide are also encompassed by the present invention, wherein amino acid (s) and/or peptide bond (s) have been replaced by functional analogues. Such functional analogues include all known amino acids other than the 20 gene- encoded amino acids, such as selenocysteine. The term (poly) peptide also refers to naturally modified (poly) peptides, where the modification is effected e.g. by glycosylation, acetylation, phosphorylation and similar modifications which are well known in the art.

In accordance with the present invention, a molecule is considered to bind specifically (also referred to herein as interacting specifically) when the respective molecule does not or essentially does not cross-react with an epitope of similar structure. Cross-reactivity of a panel of molecules under investigation may be tested, for example, by assessing binding of said panel of molecules under conventional conditions to the epitope of interest as well as to a number of more or less (structurally and/or functionally) closely related epitopes. Only those molecules that bind to the epitope of interest in its relevant context (e.g. a specific motif in the structure of a protein) but do not or do not essentially bind to any of the other epitope are considered specific for the epitope of interest. Corresponding methods are described e.g. in Harlow and Lane, Antibodies: A Laboratory Manual, Cold Spring Harbor Laboratory Press, 1988 or Harlow and Lane, Using Antibodies: A Laboratory Manual, Cold Spring Harbor Laboratory Press, 1999). The term "a molecule that essentially does not cross-react with an epitope of similar structure", as used herein, refers to a molecule that binds to the target antigen with at least 5-times higher affinity than to an epitope of similar structure, more preferably at least 10-times higher affinity, such as e.g. at least 50-times higher affinity, more preferably at least 100-times higher affinity, such as e.g. at least 500-times higher affinity. Even more preferably, it binds with at least 1.000-times higher affinity to the target antigen than to an epitope of similar structure, such as e.g. at least 10.000-times higher affinity and most preferably at least 100.000-times higher affinity.

In accordance with the present invention, the first binding (poly)peptide specifically binds to a first epitope of HER2.

An epitope may be a conformational or a continuous epitope. In polypeptide antigens, a conformational (or discontinuous) epitope is characterized by the presence of two or more discrete amino acid residues which are separated in the primary sequence, but are located near each other on the surface of the molecule when the polypeptide folds into the native three-dimensional structure to constitute the epitope (Sela, (1969) Science 166, 1365 and Laver, (1990) Cell 61, 553-6). The two or more discrete amino acid residues contributing to the epitope are present in separate sections or even in one or more (poly)peptide chain(s) of the antigen. In contrast, a linear or continuous epitope consist of two or more discrete amino acid residues which are located near each other in a single linear segment of a (poly)peptide chain.

The second binding (poly) peptide as defined in (b) specifically binds to a second epitope of HER2. Accordingly, and as described herein above, this means that the same antigen, namely HER2, is bound by this second binding (poly) peptide, however a different epitope on HER2 is bound. The term "different" epitope refers to an epitope that does not overlap with the epitope to which the first binding (poly) peptide specifically binds. Accordingly, binding of one of the binding (poly) peptide in accordance with the invention does not block the binding of the second binding (poly) peptide, thus enabling the simultaneous binding of both binding (poly) peptides.

The first and second binding (poly)peptide may be any (poly)peptide capable of specifically binding to a target epitope, such as e.g. an antibody or any of the above described non-immunoglobulin-derived binding reagents or scaffolds, preferably a scaffold selected from the group consisting of affibodies (based on the Z-domain of staphylococcal protein A), Kunitz type domains, adnectins (based on the 10th domain of human fibronectin), anticalins (derived from lipocalins), DARPins (derived from ankyrin repeat proteins), avimers (based on multimerized LDLR-A). All these scaffolds are well known in the art and have been described in the references cited herein above.

The binding (poly)peptides comprised in the HER2-specific binding molecule of the invention may form a single polypeptide chain or may be present in the HER2-specific binding molecule of the invention as several polypeptide chains, which may be covalently or non-covalently bound to each other. Where the binding (poly)peptides form a single polypeptide chain, they may be arranged in any order within said molecule, such as for example (a)-(b) or (b)-(a). More preferably, the binding (poly)peptides of (a) and (b) are arranged in the recited order, e.g. in the order (a) to (b) in the N-terminus to C-terminus direction. Where the binding (poly)peptides do not form a single polypeptide chain, they may still form a linear chain in which they are bound to each other. In that case, they may also be arranged in any order within said linear chain, such as for example (a)-(b) or (b)-(a). More preferably, the binding (poly)peptides of (a) and (b) are arranged in the recited order, e.g. in the order (a) to (b). The binding (poly)peptides may be arranged towards each other in a head-to-tail order, i.e. one binding (poly)peptide is (covalently or non-covalently) bound with its N-terminus to the C-terminus of the other binding (poly)peptide or they may be arranged in a head-to-head or a tail-to-tail order, i.e. one binding (poly)peptide is coupled (covalently or non-covalently) bound either with its N-terminus to the N-terminus of the other binding (poly)peptide or with its C-terminus to the C-terminus of the other binding (poly)peptide. It will be appreciated that the binding (poly)peptides may also form a non-linear arrangement. It will also be appreciated that one or more copies of said first and/or binding (poly)peptide may be present in the binding molecule of the invention, such as e.g. two, three or four copies of the first and/or binding (poly)peptide. Furthermore, it is a requirement for all the binding molecules described herein that the binding activity of the two binding (poly)peptides to their respective epitope is retained or essentially retained as defined herein below after formation of the binding molecules, i.e. by the covalent or non-covalent association of the two binding (poly)peptides. Where the binding molecule is formed of several (poly)peptide chains, it is preferred that these chains are covalently bound to each other.

Preferably, the binding of the HER2- specific binding molecule to tumor cells expressing HER2 on their surface results in a synergistic inhibition of tumor activity that is higher than the inhibition of tumor activity obtained by the combined binding of two mono- specific HER2- binding molecules, wherein the first mono- specific HER2- binding molecule comprises the binding (poly) peptide of (a) and the second mono- specific HER2- binding molecule comprises the binding (poly) peptide of (b)

The skilled person is well aware of methods to test the inhibition of tumor activity. For example, and as shown in the appended examples, the cell viability of HER2- expressing tumor cells may be measured (i) in the presence of the HER2- specific binding molecule of the present invention and (ii) in the presence of mono- specific HER2- binding molecules in combination, wherein one mono- specific HER2- binding molecule comprises or consists of the binding (poly) peptide of (a) and the second mono- specific HER2- binding molecule comprises or consists of the binding (poly) peptide of (b) . A synergistic inhibition of tumor activity is either present (a) when the cell viability of HER2- expressing tumor cells measured in (i) is statistically significant less than the cell viability of HER2- expressing tumor cells measured in (ii) ; in this case the synergistic effect is a superior efficacy; or (b) when the same degree of cell viability of HER2- expressing tumor cell measured in (i) is achieved at a lower dose compared to the sum of doses in (ii) ; in this case the synergistic effects is a superior potency. Preferably, the cell viability of HER2- expressing tumor cells is reduced by at least 10%, such as e.g. at least 20%, more preferably at least 30% and most preferably by 40%. HER2- expressing tumor cells for use in such tests include, without being limiting, NCI- N87 or BT- 474 cells. Means and methods for determining the cell viability of HER2- expressing tumor cells are well known in the art and include, without being limiting, crystal violet staining or XTT proliferation assays, using for example the commercially available XTT proliferation kit II (Roche) ) . Such methods have been described in the art, e.g. in Hudziak et al. (1989) Mol Cell Biol 9 (3) : 1165- 1172 as well as in the appended examples.

The HER2-specific binding molecule of the invention may be produced by any of the methods of producing (poly)peptides known in the art. For example, as described in more detail herein below, one or more nucleic acid molecules encoding the HER2-specific binding molecule of the present invention may be expressed in a suitable host and the thus produced HER2-specific binding molecule can subsequently be isolated. An alternative method for producing the HER2-specific binding molecule of the invention is *in vitro* translation of mRNA. Suitable cell-free expression systems for use in accordance with the present invention include rabbit reticulocyte lysate, wheat germ extract, canine pancreatic microsomal membranes, *E. coli* S30 extract, and coupled transcription/translation systems such as the TNT-system (Promega). These systems allow the expression of recombinant (poly)peptides upon the addition of cloning vectors, DNA fragments, or RNA sequences containing coding regions and appropriate promoter elements.

In addition to recombinant production, the HER2-specific binding molecule of the invention may be produced synthetically, e.g. by direct peptide synthesis using solid-phase techniques (cf Stewart et al. (1969) Solid Phase Peptide Synthesis; Freeman Co, San Francisco; Merrifield, J. Am. Chem. Soc. 85 (1963), 2149-2154). Synthetic protein synthesis may be performed using manual techniques or by automation. Automated synthesis may be achieved, for example, using the Applied Biosystems 431A Peptide Synthesizer (Perkin Elmer, Foster City CA) in accordance with the instructions provided by the manufacturer. Various fragments may be chemically synthesized separately and combined using chemical methods to produce the full length molecule. As indicated above, chemical synthesis, such as the solid phase procedure described by Houghton Proc. Natl. Acad. Sci. USA (82) (1985), 5131-5135, can be used. Furthermore, the HER2-specific binding molecule of the invention may be produced semi-synthetically, for example by a combination of recombinant and synthetic production.

In accordance with the present invention, it was surprisingly found that a binding molecule comprising two binding (poly)peptides having binding specificity to the same antigen, i.e. HER2, but two different epitopes of said antigen resulted in a superior antiproliferative activity on tumor cells. This activity was higher than the activity of the individual binding (poly)peptides alone and, most surprisingly, was also higher than the antiproliferative effect of both compounds given in combination. Accordingly, the generation of the HER2-specific binding molecule of the present invention results in a synergistic effect as compared to the two separate binding (poly)peptides.

In a preferred embodiment of the HER2-specific binding molecule of the invention, the first and the second binding (poly)peptide are independently selected from the group consisting of a Fyn SH3-derived polypeptide and an antibody.

The term "Fyn SH3- derived polypeptide", used interchangeably herein with the term "Fynomer", refers to a non- immunoglobulin- derived binding (poly) peptide (e.g. a so- called scaffold as described above) derived from the human Fyn SH3 domain. Fyn SH3- derived polypeptides are well known in the art and have been described e.g. in Grabulovski et al. (2007) JBC, 282, p. 3196- 3204 or WO 2008/022759, Bertschinger et al (2007) Protein Eng Des Sel 20 (2) : 57- 68, Gebauer and Skerra (2009) Curr Opinion in Chemical Biology 13: 245- 255) .

The SH3 domain of the Fyn kinase (Fyn SH3) comprises 63 residues, namely amino acids 83 to 145 of the sequence reported by Semba et al. (1986) (Proc. Natl. Acad. Sci. U S A 83(15): 5459-63) and Kawakami et al. (1986) (Mol Cell Biol. 6(12): 4195-201), with the sequence GVTLFVALYDYEARTEDDL SFHKGEKFQILNSSEGDWWEARSLTTGETGYIPSNYVAPVDSIQ, as shown in SEQ ID NO:164. Fyn is a 59 kDa member of the Src family of tyrosine kinases. As a result of alternative splicing, the Fyn protein exists in two different isoforms differing in their kinase domains: one form is found in thymocytes, splenocytes and some hematolymphoid cell lines, while a second form accumulates principally in brain (Cooke and Perlmutter (1989),New Biol. 1(1): 66-74). The biological functions of Fyn, which is an intracellular protein, are diverse and include signalling via the T cell receptor, regulation of brain function as well as adhesion mediated signalling (Resh (1998) Int. J. Biochem. Cell Biol. 30(11): 1159-62).

Just as other SH3 domains, the Fyn SH3 is composed of two antiparallel β- sheets and contains two flexible loops (the RT- Src and n- Src- loops) in order to interact with other proteins. The sequences of the two flexible loops (called RT- Src and n- Src- loops) are underlined and double- underlined, respectively. The amino acid sequence of Fyn SH3 is fully conserved among man, mouse, rat and monkey (gibbon) .

As has been shown in the art (WO 2008/022759; Grabulovski et al. (2007) JBC, 282, p. 3196- 3204) , the Fyn SH3 domain is a particularly attractive scaffold for the generation of binding proteins, i.e. Fynomers. The reason for this is because Fynomers (i) can be expressed in bacteria in soluble form in high amounts, (ii) do not aggregate when stored in solution, (iii) are very stable ($T_m$ 70.5 °C) , (iv) lack cysteine residues, and (v) are originally derived from human featuring an amino acid sequence completely conserved from mouse to man, thereby reducing unwanted immunogenic reactions.

The derivation of a specifically binding Fyn SH3-derived polypeptide for a particular target antigen has been described in the art. For example, a library of different Fyn SH3 can be created in which the sequence as shown in SEQ ID NO:164 above has been altered. Preferably, the alteration is carried out (i) in the sequence representing the RT- loop or optionally in a position up to two amino acids adjacent to said sequence (i.e. the sequence DYEARTEDDL as shown above in SEQ ID NO:164), or (ii) in the Scr loop or optionally in a position up to two amino acids adjacent to said sequence (i.e. the sequence LNSSEG as shown double-underlined above in SEQ ID NO:164) or (iii) in both sequences simultaneously. Preferably, the alteration is a substitution, deletion or addition as described in the art (see e.g. WO 2008/022759; Grabulovski et al. (2007) JBC, 282, p. 3196-3204). Means and methods for altering an amino acid sequence are well known in the art and are described in the art, e.g. in Grabulovski et al. (2007) JBC, 282, p. 3196-3204.

Subsequently, this Fyn SH3 library can be cloned into a phagemid vector, such as e.g. pHEN1 (Hoogenboom et al. "Multi- subunit proteins on the surface of filamentous phage: methodologies for displaying antibody (Fab) heavy and light chains", Nucleic Acids Res, 19 (15) : 4133- 7, 1991) and the library is subsequently displayed on phages and subjected to of panning, preferably repeated rounds of panning such as e.g. at least two, more preferably at least three rounds of panning against HER2. Subsequently, screening for binding (poly) peptides can be performed by established techniques, such as e.g. monoclonal phage- ELISA. Sequencing of the thus identified clones may then be employed to reveal the enriched sequences. The thus identified binding (poly) peptide may further be subjected to further maturation steps, such as e.g. by generating additional libraries based on alterations of the identified sequences and repeated phage display and panning steps. Finally, cross- reactivity and immunogenicity of the resulting Fyn SH3- derived polypeptide may be analysed and a Fyn SH3- derived polypeptide specific for HER2 can be selected.

These methods of phage display screening and optimization of binding (poly)peptides are generally known in the art.

The antibody may be a monoclonal or a polyclonal antibody of any class of antibodies. The term "antibody" also comprises antibody fragments or derivatives thereof which still retain the binding specificity of the respective full length or non-modified antibody. The antibody of the invention also includes embodiments such as synthetic, chimeric, single chain and humanized antibodies.

The term "antibody fragment" relates to fragments, such as a (i) Fab fragment, (ii) F $(ab')_2$ fragment, (iii) Fd fragment (consisting of the VHC and CH1 domains) , (iv) a Fv fragment and (v) an isolated complementary determining region (CDR) having sufficient framework to specifically bind, e.g., an antigen binding portion of a variable region. The term "antibody derivative" defines in the context of the invention chemically modified antibodies and antibody fragments. This includes scFv fragments, single domain antibodies etc. Accordingly, antibody derivatives are often (poly) peptides derived from antibody molecules and/or (poly) peptides which are modified by chemical/ biochemical or molecular biological methods. The minimal requirement for the specific interaction of an antibody fragment with its specific epitope is the presence of one or more CDRs from the variable heavy chain ($V_H$) and/or the variable light chain ($V_L$) of the parent antibody in a context which allows for the fitting of the antibody fragment and the epitope. Such a context can be provided by the use of a suitable framework of an antibody. As known in the art the term "framework" defines in the context of an antibody or antibody derivative the amino acid sequence which functions as a spacer between the CDRs as well as extends N- terminally and C- terminally thereof and provides for a structure which allows the formation of the antigen binding site by the CDRs. A modification of the framework or CDR sequences, e.g. to improve the binding affinity by molecular biological methods may comprise modification of the (poly) peptides using conventional techniques known in the art, for example, by using amino acid deletion (s) , insertion (s) , substitution (s) , addition (s) , and/or recombination (s) and/or any other modification (s) (e.g. posttranslational and chemical modifications, such as glycosylation and phosphorylation) known in the art either alone or in combination. Methods for introducing such modifications in the DNA sequence underlying the amino acid sequence of an immunoglobulin chain are well known to the person skilled in the art; see, e.g., Sambrook et al., Molecular Cloning: A Laboratory Manual, Cold Spring Harbor Laboratory Press, 2nd edition 1989 and 3rd edition 2001; Gerhardt et al., Methods for General and Molecular Bacteriology, ASM Press, 1994; Lefkovits, Immunology Methods Manual: The Comprehensive Sourcebook of Techniques, Academic Press, 1997; or Golemis, Protein- Protein Interactions: A Molecular Cloning Manual, Cold Spring Harbor Laboratory Press, 2002.

An antibody in accordance with the invention is capable of specifically binding/interacting with an epitope of HER2. The epitope may be a polypeptide structure as well as compounds which do not comprise amino acids, such as e.g. polysaccharides. The term "specifically binding/interacting with" is as defined herein above.

Preferably, the antibody is a monoclonal antibody. Even more preferably, the (monoclonal) antibody is of the IgG, IgA, IgE, IgD or IgM class (as well as subtypes thereof (e.g., IgG1, IgG2, IgG3 and IgG4) ) .

In another preferred embodiment of the HER2-specific binding molecule of the invention, at least the first binding (poly)peptide is a Fyn SH3-derived polypeptide.

In a more preferred embodiment of the HER2-specific binding molecule of the invention, the Fyn SH3-derived polypeptide comprises or consists of (i) an amino acid sequence selected from the group consisting of SEQ ID NO: 1 to 152 or (ii) an amino acid sequence having at least 65% sequence identity to the amino acid sequence of SEQ ID NO: 1.

In those embodiments where a binding (poly)peptide comprises (rather than consists of) the recited amino acid sequence, additional amino acids extend over the specific sequence either at the N-terminal end or the C-terminal end or both. Preferably, no more than 50 additional amino acids are present at the N- terminal end and no more than 50 additional amino acids are present at the C-terminal end. More preferably no more than 40, such as no more than 30, more preferably no more than 20, such as no more than 10, no more than 9, no more than 8, no more than 7, no more than 6, no more than 5, no more than 4, no more than 3, no more than 2 and even more preferably no more than 1 additional amino acid(s) are/is independently present at either one or both of the N- or C-terminal end. It will be appreciated that it is a prerequisite that the binding capacity of the binding molecule to the two different epitopes of the target antigen is retained or essentially retained as defined herein below in the presence of these additional amino acids. Additional sequences may include for example sequences introduced e.g. for purification. Preferably, the first binding (poly)peptide consists of the amino acid sequence recited in (i) or (ii). More preferably, the first binding (poly)peptide consists of the amino acid sequence of SEQ ID NO:1.

In accordance with the present invention, the term "% sequence identity" describes the number of matches ("hits") of identical amino acids/ nucleotides of two or more aligned amino acid or nucleic acid sequences as compared to the number of amino acid residues or nucleotides making up the overall length of the amino acid sequences or nucleic acid (or the overall compared part thereof) . In other terms, using an alignment, for two or more sequences or subsequences the percentage of amino acid residues or nucleotides that are the same (e.g., 65% or 80% identity) may be determined, when the (sub) sequences are compared and aligned for maximum correspondence over a window of comparison, or over a designated region as measured using a sequence comparison algorithm as known in the art, or when manually aligned and visually inspected. Preferred (poly) peptides in accordance with the invention are those where the described identity exists over a region that is at least about 15 to 25 amino acids in length, more preferably, over a region that is at least about 30 to 50 amino acids in length. More preferred (poly) peptides in accordance with the present invention are those having the described sequence identity over the entire length of the (poly) peptides specifically recited herein. Those having skill in the art will know how to determine percent sequence identity between/ among sequences using, for example, algorithms such as those based on the NCBI BLAST algorithm (Stephen F. Altschul, Thomas L. Madden, Alejandro A. Schäffer, Jinghui Zhang, Zheng Zhang, Webb Miller, and David J. Lipman (1997) , "Gapped BLAST and PSI- BLAST: a new generation of protein database search programs", Nucleic Acids Res. 25: 3389- 3402) , CLUSTALW computer program (Thompson Nucl. Acids Res. 2 (1994) , 4673- 4680) or FASTA (Pearson and Lipman, Proc. Natl. Acad. Sci., 1988, 85; 2444) .

The NCBI BLAST algorithm is preferably employed in accordance with this invention. For amino acid sequences, the BLASTP program uses as default a word length (W) of 3, and an expectation (E) of 10. The BLASTN program for nucleic acid sequences uses as default a word length (W) of 11, an expectation (E) of 10, M=5, N=4, and a comparison of both strands. The BLOSUM62 scoring matrix (Henikoff, Proc. Natl. Acad. Sci., 1989, 89:10915) uses alignments (B) of 50, expectation (E) of 10, M=5, N=4, and a comparison of both strands. Accordingly, all the (poly)peptides having a sequence identity of at least 80% as determined with the NCBI BLAST program fall under the scope of the invention.

In accordance with this embodiment of the present invention, also encompassed are sequences having at least 65% sequence identity, such as at least 70%, at least 80%, at least 85% and at least 90% sequence identity. Even more preferably, the identity is at least 95%, such as at least 98%, at least 99% and most preferably at least 99.5%.

The amino acid sequence having at least 65% sequence identity to the amino acid sequence of SEQ ID NO: 1 preferably has the following formula I:

GVTLFVALYDY$X_1$ $X_2$ $X_3$ $X_4$ $X_5$ $X_6$ $X_7$ $X_8$ $X_9$ $X_{10}$LSFHKGEKFQIL $X_{11}$ $X_{12}$ $X_{13}$ $X_{14}$ $X_{15}$ $X_{16}$G $X_{17}$WW $X_{18}$ARSLTTGE $X_{19}$G $X_{20}$IPS $X_{21}$YVAPVDSIQ (Formula I) (Formula I)

wherein $X_1$ to $X_7$ and $X_{11}$ to $X_{13}$ and $X_{17}$ to $X_{21}$ are each independently selected from G, V, T, L, F, A, Y, D, S, H, K, E, Q, I, W, R, M, P, N and C; more preferably from G, V, T, L, F, A, Y, D, S, H, K, E, Q, I, W, R, M, P and N; and $X_8$ to $X_{10}$ and $X_{14}$ to $X_{16}$ are each independently selected from G, V, T, L, F, A, Y, D, S, H, K, E, Q, I, W, R, M, P, N and C; more preferably from G, V, T, L, F, A, Y, D, S, H, K, E, Q, I, W, R, M, P and N; or wherein one or more of $X_8$ to $X_{10}$ and $X_{14}$ to $X_{16}$ is absent.

More preferably,

$X_1$ is selected from : T, E, D, Q, Y, V, W, N, S, F or K
$X_2$ is selected from: S, A, R or T
$X_3$ is selected from: Y, R, H, T, N, V, W or S
$X_4$ is selected from: N, D, M, Y, R, P, E, L, H, T, G or F
$X_5$ is selected from: T, S, P, Q, R, K, G, Q, A, D, M, N, L, F, Y or E
$X_6$ is selected from: R, M, K, D, F, T, G, H, S, P, N, Q, Y, L, A or P
$X_7$ is selected from: D, G, V, L, H, N, R, F, S or A
$X_8$ is selected from: G, S, E, D , P ,Y or is absent
$X_9$ is selected from: Q, D, S, H or is absent
$X_{10}$ is selected from: D, V or is absent
$X_{11}$ is selected from: R, K, Q, N, S, G, W, M, H, L, F, E, T, P, A, D or V
$X_{12}$ is selected from: M, R, E, G, N, D, S, A, Q, F, P, K, Y, T, H, V, L or W
$X_{13}$ is selected from: E, W, P, R, K, S, V, N, D, H, G, T, Q, A, Y, L or M
$X_{14}$ is selected from: D, R, Q, S, A, N, P, I, H, T, Y, E, L, K, M, V, I, W or is absent
$X_{15}$ is selected from: G, S, I, L, A, V, T, E, D, Q, R, P, K, M, H, Y or is absent
$X_{16}$ is selected from: K, G, R, A, T, V, S, I, E, Q, P, D, N, H or is absent
$X_{17}$ is selected from: V, D, T, I or Y
$X_{18}$ is selected from: E, A, R, T or Q
$X_{19}$ is selected from: T, I or V
$X_{20}$ is selected from: Y, L or F
$X_{21}$ is selected from: N or S.

In a preferred embodiment, the residues X in formula I are independently selected from: $X_1$ to $X_{10}$ is TSYNTRD (i.e. wherein $X_8$ to $X_{10}$ are absent); $X_{11}$ to $X_{16}$ is RMED (i.e. wherein $X_{14}$ to $X_{16}$ are absent); $X_{17}$ is V; $X_{18}$ is E; $X_{19}$ is T; $X_{20}$ is Y and/or $X_{21}$ is N.

Preferably, the binding (poly) peptide comprising or consisting of an amino acid sequence having at least 65% sequence identity to the amino acid sequence of SEQ ID NO: 1 retains or essentially retains the binding capacity of the binding (poly) peptide consisting of SEQ ID NO: 1, i.e. the strength of binding to the respective target epitope is retained or essentially retained.

The Fyn SH3-derived polypeptide C12 (SEQ ID NO: 1) has a dissociation constant for its specific epitope on HER2 of 7x $10^{-8}$ M when determined by surface plasmon resonance (SPR). For this, the Fyn SH3-derived polypeptide is captured, for example by a His-tag specific antibody, which has been immobilized on a BIAcore sensor chip. Upon injection of the antigen containing the specific epitope, formation of the complex is monitored and kinetic association ($k_{on}$) and kinetic dissociation constants ($k_{off}$), or dissociation constants ($K_D$), are obtained by curve fitting using the software BIAcore evaluation software. Accordingly, the binding capacity of the binding (poly)peptide comprising or consisting of an amino acid sequence having at least 65% sequence identity to the amino acid sequence of SEQ ID NO: 1 is essentially retained if a dissociation constant, preferably under the same conditions, for HER2-binding of at least $1x10^{-4}$ M is retained, such as e.g. at least $1x10^{-5}$ M, more preferably at least at least $1x10^{-6}$ M and most preferably at least $1x10^{-7}$ M. Also in accordance with the invention are binding (poly)peptides having an increased binding capacity compared to the binding (poly)peptide consisting of SEQ ID NO:1, i.e. more than 100% activity. For example, envisaged herein are binding (poly)peptides having a dissociation constant of at least $1x10^{-8}$ M, such as e.g. at least $1x10^{-9}$ M, more preferably at least at least $1x10^{-10}$ M, such as e.g. at least $1x10^{-11}$ M, even more preferably at least at least $1x10^{-12}$ M and most preferably at least $1x10^{-13}$ M. Methods of assessing the binding capacity are well known in the art and include, without being limiting, surface Plasmon resonance (SPR) techniques or ELISA.

As is shown in the appended examples, binding molecules comprising the Fyn SH3-derived polypeptide C12 (SEQ ID NO: 1) and a second binding specificity specifically bind to the tumor antigen HER2 and produce an improved, i.e. synergistic, inhibitory effect with the second binding (poly)peptide. Accordingly, it is preferred that the binding molecule of the invention comprises a Fyn SH3-derived polypeptide comprising or consisting of SEQ ID NO: 1.

In another preferred embodiment of the HER2-specific binding molecule of the invention, the second binding (poly)peptide is an antibody.

It will be appreciated that the Fyn SH3-derived polypeptide representing the first binding (poly)peptide may be coupled to the antibody in any possible position, as long as the binding capabilities of the two binding (poly)peptides is retained or essentially retained, as defined herein below. For example, the Fyn SH3-derived polypeptide representing the first binding (poly)peptide may be coupled to the antibody at the N- or C-terminal end of either the heavy chain or light chain where a complete antibody is employed. Preferably, the Fyn SH3-derived polypeptide is coupled to the N-terminal end of the light chain of an antibody.

In a more preferred embodiment of the binding molecule of the invention, the antibody is selected from the group consisting of an antibody, wherein (i) the heavy chain of the antibody comprises or consists of the amino acid sequence of SEQ ID NO: 154 and the light chain of the antibody comprises or consists of the amino acid sequence of SEQ ID NO: 155; (ii) the heavy chain of the antibody comprises or consists of SEQ ID NO: 160 and the light chain of the antibody comprises or consists of the amino acid sequence of SEQ ID NO: 163; (iii) the heavy chain of the antibody comprises or consists of an amino acid sequence having at least 65% sequence identity to the amino acid sequence of SEQ ID NO: 154 and the light chain of the antibody comprises or consists of an amino acid sequence having at least 65% sequence identity to the amino acid sequence of SEQ ID NO: 155; or (iv) the heavy chain of the antibody comprises or consists of an amino acid sequence having at least 65% sequence identity to the amino acid sequence of SEQ ID NO: 160 and the light chain of the antibody comprises or consists of an amino acid sequence having at least 65% sequence identity to the amino acid sequence of SEQ ID NO: 163.

Exemplary nucleic acid molecules encoding the heavy and light chains of SEQ ID NOs: 154, 155, 160 and 163 are shown in SEQ ID NOs: 165, 166, 168 and 169, respectively.

In accordance with this embodiment of the present invention, also encompassed in (iii) and (iv) are sequences having at least 65% sequence identity, such as at least 70%, at least 80%, at least 85% and at least 90% sequence identity to the recited amino acid sequences. Even more preferably, the identity is at least 95%, such as at least 98%, at least 99% and most preferably at least 99.5% to the recited amino acid sequences.

More preferably, the antibody defined in (iii) or (iv) is an antibody wherein the variation in the sequence identity occurs solely in the variable domain of the antibodies, such that the constant region of variant antibodies is identical to the constant region of the antibody as defined in (i) and (ii), respectively. The variable domains of the anti-HER2 antibody 1 used herein are located in amino acids 1 to 119 of SEQ ID NO:154 and amino acids 1 to 107 of SEQ ID NO:155 while the variable domains of the anti-HER2 antibody 2 used herein are located in amino acids 1 to 120 of SEQ ID NO:160 and amino acids 1 to 107 of SEQ ID NO:163. Even more preferably, the variation in the sequence identity occurs solely in the CDR domains of the antibodies, such that the remaining (non-CDR) regions of the variant antibodies is identical to the remaining (non-CDR) regions of the antibody as defined in (i) and (ii), respectively. The CDR domains of the anti-HER2 antibody 1 used herein are located in amino acids 31 to 35 (CDR1), 50 to 66 (CDR2) and 99 to 108 (CDR3) of SEQ ID NO:154 and amino acids 24 to 34 (CDR1), 50 to 56 (CDR2) and 89 to 97 (CDR3) of SEQ ID NO:155 while the CDR domains of the anti-HER2 antibody 2 used herein are located in amino acids 31 to 35 (CDR1), 50 to 66 (CDR2) and 99 to 109 (CDR3) of SEQ ID NO:160 and amino acids 24 to 34 (CDR1), 50 to 56 (CDR2) and 89 to 97 (CDR3) of SEQ ID NO:163.

All of the definition provided above with regard to the first binding (poly) peptide, for example with regard to the term "comprising" and the preferred amounts of sequence identity and methods of determining these, apply *mutatis mutandis* also to this second binding (poly) peptide of the binding molecule of the invention.

Furthermore, the second binding (poly) peptide defined in (iii) preferably retains or essentially retains the binding capacity of the binding (poly) peptide defined in (i) and the second binding (poly) peptide defined in (iv) preferably retains or essentially retains the binding capacity of the binding (poly) peptide defined in (ii) . As defined herein above, the binding capacity of the binding (poly) peptide of (iii) or (iv) is essentially retained if at least 60% of its binding capacity is retained. Preferably, at least 75% or more preferably at least 80% of its binding capacity is retained. More preferred is that at least 90% such as at least 95%, even more preferred at least 98% such as at least 99% of the binding capacity of the binding (poly) peptide defined in (i) or (ii) , respectively, is retained. Most preferred is that the binding capacity is fully, i.e. to 100%, retained. Also in accordance with the invention are binding (poly) peptides having an increased binding capacity compared to the binding (poly) peptides defined in (i) or (ii) , respectively, i.e. more than 100% activity. Preferably, the binding capacity refers to the binding capacity of a binding (poly) peptide to HER2. Methods of assessing the binding capacity have been described herein above.

The antibodies as defined in (i) and (ii) of this embodiment have a dissociation constant for their specific epitope on HER2 of between $2x10^{-9}$ M to $2x10^{-10}$ M when determined by surface plasmon resonance (SPR). For this, the antibodies are captured by a human IgG-specific antibody which has been immobilized on a BIAcore sensor chip. Upon injection of the antigen containing the specific epitope, formation of the complex is monitored and kinetic association ($k_{on}$) and kinetic dissociation constants ($k_{off}$), or dissociation constants ($K_D$), are obtained by curve fitting using the software BIAcore evaluation software. Accordingly, the binding capacity of an antibody having at least 65% sequence identity to the antibody of (i) or (ii) is essentially retained if a dissociation constant, preferably measured under the same conditions, for HER2-binding of at least $1x10^{-5}$ M is retained, such as e.g. at least $1x10^{-6}$ M, more preferably at least at least $1x10^{-7}$ M, even more preferably at least $1x10^{-8}$ M and most preferably at least $1x10^{-9}$ M. Also in accordance with the invention are antibodies having an increased binding capacity compared to the antibodies of (i) or (ii), i.e. more than 100% activity. For example, envisaged herein are antibodies having a dissociation constant of at least $1x10^{-10}$ M, such as e.g. at least $1x10^{-11}$ M, more preferably at least at least $1x10^{-12}$ M and most preferably at least $1x10^{-13}$ M.

Preferably, the second binding (poly) peptide is an antibody wherein the heavy chain of the antibody consists of the amino acid sequence of SEQ ID NO: 154 and the light chain of the antibody consists of the amino acid sequence of SEQ ID NO: 155.

Preferably, the first binding (poly) peptide consists of the amino acid sequence of SEQ ID NO: 1 and the second binding (poly) peptide is an antibody wherein the heavy chain of the antibody consists of the amino acid sequence of SEQ ID NO: 154 and the light chain of the antibody consists of the amino acid sequence of SEQ ID NO: 155. More preferred, the C- terminal end of SEQ ID NO: 1 is linked to the N- terminal end of the light chain of said antibody, i.e. to SEQ ID NO: 155.

In another preferred embodiment of the HER2-specific binding molecule of the present invention, the first and second binding (poly)peptide are linked by a linker.

The term "linker" as used in accordance with the present invention relates to a sequel of amino acids (i.e. peptide linkers) as well as to non-peptide linkers, which separate the binding (poly)peptides of the HER2-specific binding molecule of the invention. It will be appreciated that where the HER2-specific binding molecule of the present is a single polypeptide chain, the linker is a peptide linker.

The nature, i.e. the length and/or composition (such as e.g. amino acid sequence) of the linker may modify or enhance the stability and/or solubility of the molecule, it may enhance the flexibility of the resulting binding molecule and/or may improve the binding to the target antigen by reducing sterical hindrance. The length and composition of a linker depends on the composition of the respective binding (poly)peptides of the binding molecule of the invention. The skilled person is well aware of methods to test the suitability of different linkers. For example, the properties of the binding molecule of the invention can easily be tested by analysing its binding affinity when using different types of linkers. In addition, the respective measurements for each binding (poly)peptide alone may be carried out and compared to the binding affinity of the binding molecule of the invention. The stability of the resulting molecule can be measured by methods known in the art, such as e.g. using an ELISA method to determine the residual binding capacity of the molecule after incubation in human serum at 37°C for several time periods.

Peptide linkers as envisaged by the present invention are (poly) peptide linkers composed of amino acids. Preferably, the linker is 1 to 100 amino acids in length. More preferably, the linker is 5 to 50 amino acids in length and even more preferably, the linker is 10 to 20 amino acids in length. Most preferably, the linker is 15 amino acid in length. In a preferred embodiment, the linker is a flexible linker using e.g. the amino acids alanine and serine or glycine and serine. Preferably the linker sequences are $(Gly_4Ser)_1$, $(Gly_4Ser)_2$, or $(Gly_4Ser)_3$. Most preferably, the linker is $(Gly_4Ser)_3$.

The term "non-peptide linker", as used in accordance with the present invention, refers to linkage groups having two or more reactive groups but excluding peptide linkers as defined above. For example, the non-peptide linker may be a polymer, such as e.g. polyethylene glycol, having reactive groups at both ends, which individually bind to reactive groups of the binding portions of the molecule of the invention, for example, an amino terminus, a lysine residue, a histidine residue or a cysteine residue. The reactive groups of the polymer include a hydroxyl group, an aldehyde group, a propionic aldehyde group, a butyl aldehyde group, a maleimide group, a ketone group, a vinyl sulfone group, a thiol group, a hydrazide group, a carbonyldimidazole (CDI) group, a nitrophenyl carbonate (NPC) group, a trysylate group, an isocyanate group, and succinimide derivatives. Examples of succinimide derivatives include succinimidyl propionate (SPA), succinimidyl butanoic acid (SBA), succinimidyl carboxymethylate (SCM), succinimidyl succinamide (SSA), succinimidyl succinate (SS), succinimidyl carbonate, and N-hydroxy succinimide (NHS). The reactive groups at both ends of the non-peptide polymer may be the same or different. For example, the non-peptide polymer may have a maleimide group at one end and an aldehyde group at another end. Preferably, the polymer is polyethylene glycol.

Most preferably, the linker is a peptide linker.

In an even more preferred embodiment of the binding molecule of the invention, the first binding (poly) peptide consists of the amino acid sequence of SEQ ID NO: 1 linked via a $(Gly_4Ser)_3$ linker to the second binding (poly) peptide, which is an antibody wherein the heavy chain of the antibody consists of the amino acid sequence of SEQ ID NO: 154 and the light chain of the antibody consists of the amino acid sequence of SEQ ID NO: 155 and wherein the linker connects the C- terminal end of SEQ ID NO: 1 with the N- terminal end of the light chain of the antibody, i.e. SEQ ID NO: 155. The amino acid sequence of this fusion protein of SEQ ID NO: 1, the linker $(Gly_4Ser)_3$ and the light chain represented by SEQ ID NO: 155 is shown in SEQ ID NO: 159 (an exemplary nucleic acid molecule encoding this amino acid sequence is shown in SEQ ID NO: 167) . It will be appreciated that where an antibody comprising e.g. two light and two heavy chains is employed as the second binding (poly) peptide and wherein the first binding (poly) peptide is fused to either the light or heavy chain of said antibody, the resulting binding molecule in accordance with the present invention may comprise said one antibody and two first binding (poly) peptides, fused to each one of the two (either light or heavy) chains of the antibody. Examples of such binding molecules of the invention are described in the appended examples and are shown for example in figure 8 below.

As is shown in the appended examples, a binding molecule as defined above (also referred to herein as COVA208) provides superior antitumor activities on HER2-expressing tumors. The antitumor activity is synergistic, i.e. the effect observed using the binding molecule of the invention is higher than the inhibition of tumor activity obtained by the combined binding of two mono-specific binding proteins, wherein the first mono-specific binding protein is the bivalent Fyn SH3-derived polypeptide having SEQ ID NO:153 (i.e. the Fc-fusion of C12 (SEQ ID NO: 1)) and the second mono-specific binding protein is the anti-HER2 antibody 1 wherein the heavy chain of the antibody consists of the amino acid sequence of SEQ ID NO: 154 and the light chain of the antibody consists of the amino acid sequence of SEQ ID NO: 155.

In another preferred embodiment, the HER2-specific binding molecule of the invention further comprises at least one additional (poly)peptide.

Non- limiting examples of such additional (poly) peptides are e.g. pharmaceutically and/or diagnostically active components, including tags or functional (poly) peptides suitable to improve the performance of the HER2- specific binding molecule of the invention.

Pharmaceutically and/or diagnostically active components may for example be selected from cytokines, toxic compounds, chemokines, enzymes, fluorescent dyes and photosensitizers, pro-coagulant factor, preferably a tissue factor, radionuclides or components that modulate the serum half-life of the HER2-specific binding molecule of the invention.

Non- limiting examples of cytokines include e.g. IL- 2, IL- 12, TNF- alpha, IFN alpha, IFN beta, IFN gamma, IL- 10, IL- 15, IL- 24, GM- CSF, IL- 3, IL- 4, IL- 5, IL- 6, IL- 7, IL- 9, IL- 11, IL- 13, LIF, CD80, B70, TNF beta, LT- beta, CD- 40 ligand, Fas- ligand, TGF- beta, IL- 1alpha and IL- 1beta.

Examples of toxic compounds include, without being limiting, calicheamicin, maytansinoid, neocarzinostatin, esperamicin, dynemicin, kedarcidin, maduropeptin, doxorubicin, daunorubicin, auristatin, Ricin-A chain, modeccin, truncated Pseudomonas exotoxin A, diphtheria toxin and recombinant gelonin.

Non- limiting examples of chemokines include IL- 8, GRO alpha, GRO beta, GRO gamma, ENA- 78, LDGF-PBP, GCP- 2, PF4, Mig, IP- 10, SDF- 1alpha/ beta, BUNZO/ STRC33, I- TAC, BLC/BCA- 1, MIP- 1alpha, MIP- 1 beta, MDC, TECK, TARC, RANTES, HCC- 1, HCC- 4, DC- CK1, MIP- 3 alpha, MIP- 3 beta, MCP- 1- 5, Eotaxin, Eotaxin- 2, 1- 309, MPIF- 1, 6Ckine, CTACK, MEC, Lymphotactin and Fractalkine.

Fluorescent dyes include e.g. Alexa Fluor or Cy dyes and photosensitizers include for example phototoxic red fluorescent protein KillerRed or haematoporphyrin.

Non- limiting examples of enzymes include enzymes for pro- drug activation, preferably enzymes selected from the group consisting of carboxy- peptidases, glucuronidases and glucosidases.

Radionuclides may be selected from e.g. the group of gamma- emitting isotopes, preferably $^{99m}$TC, $^{123}$I, $^{111}$In; from the group of positron emitters, preferably $^{18}$F, $^{64}$Cu, $^{68}$Ga, $^{86}$Y, $^{124}$I; from the group of beta- emitters, preferably $^{131}$I, $^{90}$Y, $^{177}$Lu, $^{67}$Cu; or from the group of alpha- emitters, preferably $^{213}$Bi, $^{211}$At.

Examples of components that modulate serum half-life include, without being limiting, polyethylene glycol (PEG), Fc domains of antibodies, albumin-binding proteins and conformationally disordered polypeptide sequences.

Non- limiting examples of tags include Strep- tags, His- tags, Myc- tags, TAP- tags or Flag- tags. Additional functional (poly) peptides are e.g. secretion peptides such as the kappa secretion leader or peptides providing N-glycosylation sites.

As outlined herein above, some of the additional (poly)peptides may have an additional pharmaceutical or diagnostic activity or may enhance stability of the HER2-specific binding molecule of the invention, thereby enhancing its antitumorigenic activity, while other additional (poly)peptides may instead facilitate the preparation and/or purification of the HER2-specific binding molecule. Methods to add the above defined additional (poly)peptides to the HER2-specific binding molecule of the invention are well known to the skilled person and are described e.g. in Sambrook, 2001, loc cit.. It will be appreciated that the additional (poly)peptides may be non-covalently bound to the HER2-specific binding molecule of the invention or may be covalently bound, for example they may form a fusion protein with the HER2-specific binding molecule, e.g. they may form a single polypeptide chain. Such a fusion protein may for example be encoded by a single nucleic acid molecule.

Also encompassed by the present invention are multimers, such as e.g. dimers, trimers, tetramers etc., formed of the binding molecule of the invention, optionally including additional (poly) peptides as defined herein above. Such multimers may be formed by covalent or non- covalent association, preferably by covalent association. Preferably, multimers are formed via linkers as defined herein above, preferably the above defined peptide linkers. More preferably,

the linker is $(Gly_4Ser)_1$, $(Gly_4Ser)_2$, or $(Gly_4Ser)_3$. Most preferably, the linker is $(Gly_4Ser)_3$.

The present invention further relates to a nucleic acid molecule encoding the HER2-specific binding molecule of the invention.

In accordance with the present invention the term "nucleic acid molecule", also referred to as "polynucleotide" or "nucleic acid sequence" herein, defines a linear molecular chain consisting of more than 30 nucleotides. "Nucleic acid molecules", in accordance with the present invention, include DNA, such as for example cDNA or genomic DNA, and RNA, for example mRNA. Further included are nucleic acid mimicking molecules known in the art such as for example synthetic or semisynthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art.

It will be appreciated that the binding molecule of the present invention may be encoded by a single nucleic acid molecule or a plurality of nucleic acid molecules encoding parts of the binding molecule, such as e.g. the individual binding (poly)peptides or different chains of an antibody. Upon expression of these nucleic acid molecules, they form the binding molecule of the invention via non-covalent bonds such as for example hydrogen bonds, ionic bonds, van der Waals forces or hydrophobic interacts or via covalent bonds such as for example disulfide bonds.

For example, where the binding molecule is a Fyn SH3-derived polypeptide bound to an antibody-fragment comprising e.g. of only the scFv fragment or dAb, then the binding molecule may be encoded by a single nucleic acid molecule. Where the binding molecule comprises a Fyn SH3-derived polypeptide bound to a full-length antibody as shown in the appended examples, a first nucleic acid molecule may encode the Fyn SH3-derived polypeptide and the chain of the antibody to which the Fyn SH3-derived polypeptide is bound and a second nucleic acid molecule may encode the remaining chain of the antibody. Alternatively, a single nucleic acid molecule may encode also these separate polypeptide chains, for example when the encoding nucleic acid sequences are included in a vector comprising the respective regulatory elements for each of the encoding sequences. It will be appreciated that where several nucleic acid sequences (or vectors as described below) are employed to encode the binding molecule of the invention, the resulting expressed polypeptides may need to be brought into contact in order to form the binding molecules of the invention.

In accordance with the present invention, the term "nucleic acid molecule of the invention" encompasses both a single nucleic acid molecule as well as a plurality of nucleic acid molecules, as long as all the components of the HER2-specific binding molecule of the invention are encoded thereby.

The present invention further relates to a vector comprising the nucleic acid molecule of the invention.

Preferably, the vector is a plasmid, cosmid, virus, bacteriophage or another vector used conventionally e.g. in genetic engineering.

The nucleic acid molecule of the present invention may be inserted into several commercially available vectors. Non- limiting examples include prokaryotic plasmid vectors, such as pQE- 12, the pUC- series, pBluescript (Stratagene) , the pET- series of expression vectors (Novagen) or pCRTOPO (Invitrogen) , lambda gt11, pJOE, the pBBR1- MCS series, pJB861, pBSMuL, pBC2, pUCPKS, pTACT1 and vectors compatible with expression in mammalian cells like E-027 pCAG Kosak- Cherry (L45a) vector system, pREP (Invitrogen) , pCEP4 (Invitrogen) , pMC1neo (Stratagene) , pXT1 (Stratagene) , pSG5 (Stratagene) , EBO- pSV2neo, pBPV- 1, pdBPVMMTneo, pRSVgpt, pRSVneo, pSV2- dhfr, pIZD35, Okayama- Berg cDNA expression vector pcDV1 (Pharmacia) , pRc/CMV, pcDNA1, pcDNA3 (Invitrogen) , pcDNA3.1, pSPORT1 (GIBCO BRL) , pGEMHE (Promega) , pLXIN, pSIR (Clontech) , pIRES- EGFP (Clontech) , pEAK- 10 (Edge Biosystems) pTriEx- Hygro (Novagen) and pCINeo (Promega) . Examples for plasmid vectors suitable for Pichia pastoris comprise e.g. the plasmids pAO815, pPIC9K and pPIC3.5K (all Invitrogen) . Another vector suitable for expressing proteins in Xenopus embryos, zebrafish embryos as well as a wide variety of mammalian and avian cells is the multipurpose expression vector pCS2+.

The nucleic acid molecule of the present invention referred to above may also be inserted into vectors such that a translational fusion with another nucleic acid molecule is generated. The other nucleic acid molecules may e.g. encode a protein that increases the solubility and/or facilitates the purification of the HER2- specific binding molecule encoded by the nucleic acid molecule of the invention or a protein of interest that is to be observed by fluorescence imaging. Non- limiting examples of such vectors include pET32, pET41, pET43. The vectors may also contain an additional expressible polynucleotide coding for one or more chaperones to facilitate correct protein folding. Suitable bacterial expression hosts comprise e. g. strains derived from BL21 (such as BL21 (DE3) , BL21 (DE3) PlysS, BL21 (DE3) RIL, BL21 (DE3) PRARE) or Rosettaâ.

For vector modification techniques, see Sambrook and Russel, 2001. Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M 13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Such regulatory sequences are well known to those skilled in the art and include, without being limiting, regulatory sequences ensuring the initiation of transcription, internal ribosomal entry sites (IRES) (Owens, Proc. Natl. Acad. Sci. USA 98 (2001) , 1471- 1476) and optionally regulatory elements ensuring termination of transcription and stabilization of the transcript. Non- limiting examples for regulatory elements ensuring the initiation of transcription comprise a translation initiation codon, enhancers such as e.g. the SV40-enhancer, insulators and/or promoters, such as for example the cytomegalovirus (CMV) promoter, SV40- promoter, RSV- promoter (Rous sarcome virus) , the lacZ promoter, chicken beta- actin promoter, CAG- promoter (a combination of chicken beta- actin promoter and cytomegalovirus immediate- early enhancer) , the gai10 promoter, human elongation factor 1$\alpha$- promoter, AOX1 promoter, GAL1 promoter CaM- kinase promoter, the lac, trp or tac promoter, the lacUV5 promoter, the autographa californica multiple nuclear polyhedrosis virus (AcMNPV) polyhedral promoter or a globin intron in mammalian and other animal cells. The lac promoter is a typical inducible promoter, useful for prokaryotic cells, which can be induced using the lactose analogue isopropylthiol- b- D- galactoside ("IPTG") . Non- limiting examples for regulatory elements ensuring transcription termination include the V40- poly- A site, the tk- poly- A site or the SV40, lacZ or AcMNPV polyhedral polyadenylation signals, which are to be included downstream of the nucleic acid sequence of the invention. Additional regulatory elements may include translational enhancers, Kozak sequences and intervening sequences flanked by donor and acceptor sites for RNA splicing, nucleotide sequences encoding secretion signals or, depending on the expression system used, signal sequences capable of directing the expressed polypeptide to a cellular compartment. For example an N- terminal flanking sequence or "leader sequence", which is also referred to as "signal peptide" in the art, may be included. The skilled person can choose suitable leader sequences without further ado. A leader sequence is preferably employed for the expression of any antibody chain (including light chain, heavy chain) or domain but is no longer required in the expressed, mature construct.

Moreover, elements such as origin of replication, drug resistance gene, regulators (as part of an inducible promoter) may also be included.

An expression vector according to this invention is capable of directing the replication, and the expression of the nucleic acid molecule of the invention and the HER2-specific binding molecule encoded thereby.

The co-transfection with a selectable marker such as dhfr, gpt, neomycin, hygromycin allows the identification and isolation of the transfected cells. The transfected nucleic acid can also be amplified to express large amounts of the encoded HER2-binding molecule. The DHFR (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992). Using these markers, the cells are grown in selective medium and the cells with the highest resistance are selected. Expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria.

The nucleic acid molecules of the invention as described herein above may be designed for direct introduction or for introduction via electroporation (using for example Multiporator (Eppendorf) or Genepulser (BioRad) ) , PEI (Polysciences Inc. Warrington, Eppelheim) , $Ca^{2+}$- mediated transfection or via liposomes (for example: "Lipofectamine" (Invitrogen) ) , non- liposomal compounds (for example: "Fugene" (Roche) ) , liposomes, phage vectors or viral vectors (e.g. adenoviral, retroviral, lentiviral) into cells. Additionally, baculoviral systems or systems based on Vaccinia Virus or Semliki Forest Virus can also be used as vector in eukaryotic expression system for the nucleic acid molecules of the invention. Expression vectors derived from viruses such as retroviruses, vaccinia virus, adeno- associated virus, herpes viruses, or bovine papilloma virus, may be used for delivery of the nucleic acid molecules or vector into targeted cell population. Methods which are well known to those skilled in the art can be used to construct recombinant viral vectors; see, for example, the techniques described in Sambrook, 2001 and Ausubel, 2001.

It will be appreciated that where the HER2-specific binding molecule of the invention is encoded by more than one nucleic acid molecule, said plurality of nucleic acid molecules may be comprised in one or in a plurality of vectors. The term "the vector of the invention" encompasses both a single vector as well as a plurality of vectors, as long as all the components of the HER2-specific binding molecule of the invention are encoded thereby.

The present invention further relates to a host cell or a non-human host transformed with the vector of the invention.

Said host or host cell may be produced by introducing the vector of the invention into a host or host cell, which upon its presence mediates the expression of the HER2-specific binding molecule encoded by the vector.

In accordance with the present invention, the host may be a transgenic non-human animal transfected with and/or expressing the vector of the present invention. In a preferred embodiment, the transgenic animal is a mammal, e.g. a hamster, cow, cat, pig, dog or horse.

In a preferred embodiment, the host is a cell, such as an isolated cell which may be part of a cell culture.

Suitable prokaryotic host cells comprise e.g. bacteria of the species Escherichia, *Bacillus, Streptomyces* and *Salmonella typhimurium.* Suitable eukaryotic host cells are e.g. fungal cells, inter alia, yeasts such as *Saccharomyces cerevisiae* or *Pichia pastoris* or insect cells such as Drosophila S2 and Spodoptera Sf9 cells and plant cells as well as mammalian cells. Appropriate culture mediums and conditions for the above-described host cells are known in the art.

Mammalian host cells include without being limiting human Hela, HEK293, H9 and Jurkat cells, mouse NIH3T3 and C127 cells, COS 1, COS 7 and CV1, quail QC1-3 cells, mouse L cells, Chinese hamster ovary (CHO) cells and Bowes melanoma cells. Alternatively, the binding molecule of the invention can be expressed in stable cell lines that contain the gene construct encompassing the nucleic acid molecule or the vector of the invention integrated into a chromosome.

In another preferred embodiment, said cell is a primary cell or primary cell line. Primary cells are cells which are directly obtained from an organism. Suitable primary cells are, for example, mouse embryonic fibroblasts, mouse primary hepatocytes, cardiomyocytes and neuronal cells as well as mouse muscle stem cells (satellite cells) and stable, immortalized cell lines derived thereof.

The present invention also relates to a method for the production of a HER2-specific binding molecule according to the invention comprising culture of the host cell of the invention under suitable conditions and isolation of the HER2-specific binding molecule produced by said host cell.

Suitable conditions for culturing a prokaryotic or eukaryotic host are well known to the person skilled in the art. For example, suitable conditions for culturing bacteria are growing them under aeration in Luria Bertani (LB) medium. To increase the yield and the solubility of the expression product, the medium can be buffered or supplemented with suitable additives known to enhance or facilitate both. *E. coli* can be cultured from 4 to about 37°C, the exact temperature or sequence of temperatures depends on the molecule to be over-expressed. In general, the skilled person is also aware that these conditions may have to be adapted to the needs of the host and the requirements of the HER2-specific binding molecule expressed. In case an inducible promoter controls the nucleic acid molecule of the invention in the vector present in the host cell, expression of the HER2-specific binding molecule can be induced by addition of an appropriate inducing agent. Suitable expression protocols and strategies are known to the skilled person.

Depending on the cell type and its specific requirements, mammalian cell cultures can e.g. be carried out in RPMI or DMEM medium containing 10% (v/v) FCS, 2mM L-glutamine and 100 U/ml penicillin/streptomycine. The cells can be kept at 37 °C in a 5% $CO_2$ water saturated atmosphere. Suitable media for insect cell culture is e.g. TNM + 10% FCS or SF900 medium. Insect cells are usually grown at 27 °C as adhesion or suspension culture.

Suitable expression protocols for eukaryotic cells are well known to the skilled person and can be retrieved e.g. from in Sambrook, 2001, loc cit.

Methods of isolating the HER2-specific binding molecule produced are well-known in the art and comprise without being limiting method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook, 2001, loc. cit.

The present invention also provides a composition comprising at least one of (i) the HER2-specific binding molecule of the invention, (ii) the nucleic acid molecule of the invention; (iii) the vector of the invention or (iv) the host cell of the invention.

The term "composition", as used in accordance with the present invention, relates to a composition which comprises at least one of the recited compounds. It may, optionally, comprise further molecules capable of altering the characteristics of the compounds of the invention thereby, for example, stabilizing, modulating and/or enhancing their function. The composition may be in solid or liquid form and may be, inter alia, in the form of (a) powder(s), (a) tablet(s) or (a) solution(s).

In a preferred embodiment, the composition is a pharmaceutical composition optionally further comprising a pharmaceutically acceptable carrier.

In accordance with the present invention, the term "pharmaceutical composition" relates to a composition for administration to a patient, preferably a human patient. The pharmaceutical composition of the invention comprises the compounds recited above. The pharmaceutical composition of the present invention may, optionally and additionally, comprise a pharmaceutically acceptable carrier. By "pharmaceutically acceptable carrier" is meant a non- toxic solid, semisolid or liquid filler, diluent, encapsulating material or formulation auxiliary of any type. Examples of suitable pharmaceutical carriers are well known in the art and include sodium chloride solutions, phosphate buffered sodium chloride solutions, water, emulsions, such as oil/ water emulsions, various types of wetting agents, sterile solutions, organic solvents etc. Preferably the carrier is a parenteral carrier, more preferably a solution that is isotonic with the blood of the recipient. The term "parenteral" as used herein refers to modes of administration which include intravenous, intramuscular, intraperitoneal, intrasternal, subcutaneous and intraarticular injection and infusion. The carrier suitably contains minor amounts of additives such as substances that enhance isotonicity and chemical stability. Such materials are non- toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly) peptides, e.g., polyarginine or tripeptides; proteins, such as serum albumin, gelatin, or further immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

Compositions comprising such carriers can be formulated by well known conventional methods. Generally, the formulations are prepared by contacting the components of the pharmaceutical composition uniformly and intimately with liquid carriers or finely divided solid carriers or both. Then, if necessary, the product is shaped into the desired formulation.

These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgment of the ordinary clinician or physician. The pharmaceutical composition may be for administration once or for a regular administration over a prolonged period of time. Generally, the administration of the pharmaceutical composition should be in the range of for example 10μg/kg of body weight to 2g/kg of body weight for a single dose. However, a more preferred dosage might be in the range of 100μg /kg to 1.5g/kg of body weight, even more preferably 1 mg/kg to 1g/kg of body weight and even more preferably 5mg/kg to 500mg/kg of body weight for a single dose.

Administration of pharmaceutical compositions of the invention may be effected by different ways, e.g., by intravenous, intraperitoneal, subcutaneous, intramuscular, intradermal, intranasal or intrabronchial administration.

The components of the pharmaceutical composition to be used for therapeutic administration must be sterile. Sterility is readily accomplished by filtration through sterile filtration membranes (e.g., 0.2 micron membranes).

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic water-for-injection. Preservatives and other additives may also be present such as, for example, antimicrobials, anti oxidants, chelating agents, and inert gases and the like. The pharmaceutical composition may comprise further agents depending on the intended use of the pharmaceutical composition.

The pharmaceutical composition may be particularly useful for the treatment of tumors as disclosed below.

In another preferred embodiment, the composition of the invention is a diagnostic composition.

In accordance with the present invention, the term "diagnostic composition" relates to compositions for diagnosing individual patients for their potential response to or curability by the pharmaceutical compositions of the invention. The diagnostic composition of the invention comprises the compounds recited above. The diagnostic composition may further comprise appropriate buffer(s) etc.. The diagnostic compositions may be packaged in a container or a plurality of containers.

The present invention further relates to the HER2-specific binding molecule of the invention, the nucleic acid molecule of the invention or the vector of the invention for use in the treatment of tumors.

The term "tumor", in accordance with the present invention refers to a class of diseases or disorders characterised by uncontrolled division of cells and encompasses all types of tumors, such as e.g. cancerous tumors and benign tumors as well as solid tumors and non-solid tumors. Cancerous tumors are further characterized by the ability of these tumors to spread, either by direct growth into adjacent tissue through invasion, or by implantation into distant sites by metastasis (where tumor cells are transported through the bloodstream or lymphatic system).

Preferably, the tumor is a cancerous tumor selected from the group consisting of breast cancer, ovarian cancer, bladder cancer, salivary gland cancer, endometrium cancer, pancreatic cancer, thyroid cancer, kidney cancer, lung cancer, upper gastrointestinal tract cancers, colon cancer and prostate cancer.

All of the cancer types described herein are well known to the skilled person and are defined in accordance with the pertinent art and the common general knowledge of the skilled person.

The figures show:

**Figure 1:** FACS binding experiments using HER2 overexpressing BT-474 cells.

(A) Binding of Fyn SH3 derived polypeptides C12 (SEQ ID NO: 1) and G10 (SEQ ID NO: 2) on HER2 with or without pre-blocking of the epitope of the anti-HER2 antibody 1 (anti-HER2 mAb 1; wherein the heavy chain has the amino acid sequence of SEQ ID NO: 154 and the light chain has the amino acid sequence of SEQ ID NO: 155; exemplary nucleic acid molecules encoding the heavy and light chain are shown in SEQ ID NO: 165 and 166) and anti-HER2 antibody 2 (anti-HER2 mAb 2; wherein the heavy chain has the amino acid sequence of SEQ ID NO: 160 and the light chain has the amino acid sequence of SEQ ID NO: 163; exemplary nucleic acid molecules encoding the heavy and light chain are shown in SEQ ID NO: 168 and 169). PBS, phosphate buffered saline, represents the negative control.

(B) Binding of biotinylated anti-HER2 antibody 1 and biotinylated anti-HER2 antibody 2 (biotinylated antibodies are indicated with the abbreviation "bt") with or without pre-blocking of the epitope of the anti-HER2 antibody 1 and anti-HER2 antibody 2. PBS, phosphate buffered saline, represents the negative control.

**Figure 2:** *In vitro* proliferation assays with HER2 overexpressing gastric cancer cell line NCI-N87. Fyn SH3-derived polypeptide C12 (SEQ ID NO:1) was fused to the Fc part of a human IgG1 to create the monospecific bivalent protein called Fc-C12 (SEQ ID NO: 153). The combination mixture of Fynomer C12-Fc with the anti-HER2 antibody 1 (anti-HER2 mAb 1) (shown in Fig. 2A) and with the anti-HER2 antibody 2 (anti-HER2 mAb 2) (shown in Fig. 2C) did not reduce proliferation rate of NCI-N87 cells more effectively than the corresponding anti-HER2 antibodies alone. However, the anti-proliferative activity of the binding molecules COVA208 (SEQ ID NO: 154 & 159) (shown in Fig. 2B) and COVA210 (SEQ ID NO: 160 & 161; an exemplary nucleic acid molecule encoding SEQ ID NO:161 is shown in SEQ ID NO: 170) (Fig. 2D) was higher than the activity of the corresponding unmodified antibody. COVA 208 consists of the fusion of C12 (SEQ ID NO:1) to the N-terminus of the light chain of antibody 1 (SEQ ID NO: 154 and 155)) and COVA210 consists of the fusion of C12 (SEQ ID NO:1) to the N-terminus of the light chain of antibody 2 (SEQ ID NO: 160 and 163), see also Figure 8.

**Figure 3:** The anti-proliferative activity of anti-HER2 Fynomer-antibody fusions varies depending on the relative orientation of the Fynomer and the binding site of the antibody.

The anti-proliferative activities of the different Fynomer-antibody fusion proteins in a proliferation cell assay with NCI-N87 gastric cancer cells showed variations (A) and (B), and COVA208 showed the best anti-proliferative effects on this cell line (Fig. 3B). The maximal effects are indicated in the tables and given in percentage of viability. COVA201 (SEQ ID NOs: 156 and 155), COVA202 (SEQ ID NOs: 154 and 157), COVA207 (SEQ ID NOs: 158 and 155) and COVA208 (SEQ ID NOs: 154 and 159) are all fusion proteins of the Fyn SH3 derived polypeptide C12 (SEQ ID NO: 1) and anti-HER2 antibody 1 (anti-HER2 mAb 1) (SEQ ID NOs: 154 and 155). COVA201 consists of the C-terminal heavy chain fusion, COV202 represents the C-terminal light chain fusion, COVA 207 consists of the N-terminal heavy chain fusion and COVA208 represents the N-terminal light chain fusion, see also Figure 8.

**Figure 4:** The anti-proliferative activity of COVA208 (SEQ ID NOs: 154 and 159) (fusion of Fynomer C12 to the N-terminus of the light chain of anti-HER2 antibody 1 (anti-HER2 mAb 1, SEQ ID NOs: 154 and 155)) was determined in a cell assay with the HER2 overexpressing breast cancer cell line BT-474. COVA208 exhibited superior anti-proliferative activity as compared to the unmodified antibody.

**Figure 5:** depicts an animal study with a NCI-N87 gastric cancer xenograft mouse model. NCI-N87 gastric cancer cells were inoculated subcutaneously in CD1 Nude mice (n=6 per treatment group). When tumors reached a size of about 140 $mm^3$, animals were treated with a loading dose of 30 mg/kg COVA208 (SEQ ID NOs: 154 and 159), anti-HER2 antibody 1 (anti-HER2 mAb 1 (SEQ ID NOs: 154 and 155)) or placebo (PBS). Treatment was continued with four weekly i.p. injections (15 mg/kg) (indicated with the arrows) and size of tumors was measured with a caliper. COVA208 was found to inhibit tumor growth significantly better than the monospecific anti-HER2 antibody 1 or placebo (PBS). Mean tumor volumes of 6 mice are shown (relative to day 0 when the treatment was started) ± standard error of the mean (SEM).

**Figure 6:** Serum concentrations of COVA208 (SEQ ID NOs: 154 and 159) and the anti-HER2 antibody 1 (anti-HER2 mAb 1 (SEQ ID NOs: 154 and 155)) at different time-points after a single i.v. injection into C57Bl/6 mice. The six last time-points were used to calculate the terminal half-lives of 247 h (COVA208) and 187 h (anti-HER2 antibody 1). Mean serum concentrations are plotted versus time, error bars represent standard deviations (SD).

**Figure 7:** SDS PAGE of COVA208 (SEQ ID NOs: 154 and 159) and anti-HER2 antibody 1 (anti-HER2 mAb 1 (SEQ ID NO: 154 and 155)) (top) and size exclusion chromatograms of COVA208 after purification and after a storage period of 1 and 2 months at 4° C (*bottom*). Evidently, COVA208 did not form any aggregates.

**Figure 8:** Schematic overview of different formats. COVA201 (SEQ ID NO: 156 & 155), COVA202 (SEQ ID NO: 154 & 157), COVA207 (SEQ ID NO: 158 & 155) and COVA208 (SEQ ID NO: 154 & 159) are all fusion proteins of the Fyn SH3 derived polypeptide C12 (SEQ ID NO: 1) and anti-HER2 antibody 1 (anti-HER2 mAb 1) (SEQ ID NO: 154 and 155). COVA201 consists of the C-terminal heavy chain fusion, COV202 represents the C-terminal light chain fusion, COVA 207 consists of the N-terminal heavy chain fusion and COVA208 represents the N-terminal light chain fusion. COVA210 (SEQ ID NO: 160 & 161) consists of the fusion of C12 (SEQ ID NO:1) to the N-terminus of the light chain of antibody 2 (SEQ ID NO: 160 and 163).

The examples illustrate the invention:

**<u>Example 1</u>: Fyn SH3 derived polypeptides bind to HER2**

Methods

1) Phage ELISA on recombinant HER2 protein

DNA encoding the amino acids shown in SEQ ID NOs: 9 to 121 were cloned into the phagemid vector pHEN1 as described for the Fyn SH3 library in Grabulovski et al. (Grabulovski et al. (2007) JBC, 282, p. 3196- 3204) . Phage production was performed according to standard protocols (Viti, F. et al. (2000) Methods Enzymol. 326, 480- 505) . Monoclonal bacterial supernatants containing phages were used for ELISA: biotinylated extracellular domain of HER2 comprising amino acids 23- 652 of the full- length protein (purchased from Bender Medsystems, or from R&D as fusion to human Fcγ1; biotinylation was performed with sulfo- NHS- LC- biotin (Pierce) according to the manufacturer's instructions) was immobilized on streptavidin- coated wells (StreptaWells, High Bind, Roche) , and after blocking with 2% milk (Rapilait, Migros, Switzerland) in PBS, 20 μl of 10% milk in PBS and 80 μl of phage supernatants were applied. After incubation for 1 hr, unbound phage were washed off, and bound phages were detected with anti- M13- HRP antibody conjugate (GE Healthcare) . The detection of peroxidase activity was done by adding BM blue POD substrate (Roche) and the reaction was stopped by adding 1 M $H_2SO_4$. The phage ELISA positive clones were tested by phage ELISA for the absence of crossreactivity to Streptavidin (StreptaWells, High Bind, Roche) and to human IgG (Sigma) . The DNA sequence of the specific binders was verified by DNA sequencing.

2) FACS experiment on HER2 overexpressing SKOV-3 cells

DNA encoding the polypeptides shown in SEQ ID NOs: 1 to 8 and SEQ ID NOs: 122-152 were subcloned into the bacterial expression vector pQE12 so that the resulting constructs carried a C-terminal myc-hexahistidine tag (SEQ ID NO: 162) as described in Grabulovski et al. (Grabulovski et al. (2007) JBC, 282, p. 3196-3204). The polypeptides were expressed in the cytosol of *E.coli* bacteria, and 1.8 ml of cleared lysate was prepared per ml original culture. 100 μl cleared lysate containing the polypeptides was mixed with 100 μl cell suspension containing 1.25 x $10^5$ SKOV-3 cells in PBS/1% FCS/0.2 % sodium azide. After 60 min incubation on ice, cells were washed, and bound sequences were detected by 10 μg/ml anti-myc mouse antibody 9E10 (Roche), followed by anti-mouse IgG - Alexa488 conjugate (Invitrogen). The stained cells were then analyzed in a FACS analyzer. The DNA sequence of the specific binders was verified by DNA sequencing.

Results:

The amino acid sequences of Fyn SH3 derived HER2 binders is presented in SEQ ID NOs: 1 to 152 as appended in the sequence listing.

**<u>Example 2</u>: Fyn SH3 derived polypeptides bind to other epitopes on HER2 compared to anti-HER2 antibodies**

Methods:

The DNA sequences encoding FynSH3-derived clones C12 (SEQ ID NO: 1) and G10 (SEQ ID NO: 2) were subcloned into the bacterial expression vector pQE12 so that the resulting constructs carried a C-terminal myc-hexahistidine tag (SEQ ID NO: 162), and the two constructs were expressed and purified by means of the hexahistidine tag as described in Grabulovski et al. (Grabulovski et al. (2007) JBC, 282, p. 3196-3204).

The heavy and light chains (SEQ ID NO: 154 and SEQ ID NO: 155) of the anti-HER2 antibody 1 and the anti-HER2 antibody 2 (SEQ ID NO: 160 and SEQ ID NO: 163) were transiently co-expressed in CHO cells. The antibodies were purified from the culture supernatant by affinity chromatography on a MabSelect SuRe column (GE healthcare).

$10^5$ BT- 474 cells (ATCC) were pre- incubated with an excess of 1 μM anti- HER2 antiboy 1, anti- HER2 antiboy 2, or PBS for 60 min on ice. Subsequently, 300 nM C12 or G10 plus 20 nM mouse anti- myc antibody 9E10 (Roche) were added to the cells without washing off the blocking antibodies. After 45 min incubation, cells were washed and bound C12/9E10- and G10/9E10 complexes were detected with anti- mouse IgG- Alexa488 conjugate. The cells were analyzed by FACS. Binding of C12 and G10 to anti- HER2 antiboy 1 or anti- HER2 antiboy 2- blocked cell surface was compared against binding to non- blocked cells. In order to analyze the efficacy of the epitope blockade by anti- HER2 antibody 1 and 2, 25 nM biotinylated antibody (biotinylation was performed with sulfo- NHS-LC- biotin (Pierce) according to the manufacturer's instructions) was added to the pre- blocked cells, followed by detection with Streptavidin- allophycocyanin conjugate.

Results:

The results of the experiments are shown in Figure 1. Preblocking with either of the antibodies drastically reduced binding of the corresponding biotinylated antibodies, indicating that the preblocking step efficiently and specifically blocked the epitopes of the two different antibodies (Figure 1 B).
Binding of C12 and of G10 was not affected by preblocking with anti-HER2 antibody 1 nor with anti-HER2 antibody 2, indicating that both clones bind to an epitope different to anti-HER2 antibody 1 and anti-HER2 antibody 2 (Figure 1A).

**<u>Example 3</u>: The polypeptides of the invention have a stronger antiproliferative effect than the combination of the individual binding proteins**

HER2 targeting molecules were created by fusion of C12 via a glycine-serine $(Gly_4Ser)_3$ linker to the N-terminus of the light chain of anti-HER2 antibody 1 (resulting in the protein termed COVA208) or anti-HER2 antibody 2 (termed COVA21 0).
Methods:

Anti-HER2 antibody 1 (SEQ ID NO: 154 and SEQ ID NO:155), anti-HER2 antibody 2 (SEQ ID NO: 160 and SEQ ID NO: 163), COVA208 (SEQ ID NO: 154 and SEQ ID NO:159) and COVA210 (SEQ ID NO: 160, SEQ ID NO: 161) were transiently co-expressed in CHO cells and purified from the culture supernatant by affinity chromatography on a MabSelect SuRe column (GE healthcare). A bivalent monospecific format of clone C12 was created by fusion via a $(Gly_4Ser)_3$ to the C-terminus of human Fcγ1, resulting in Fc-C12 (SEQ ID NO: 153). The protein was expressed and purified as described above for anti-HER2 antibody 1, anti-HER2 antibody 2, COVA208 and COVA210.

The growth inhibitory effect of the HER2 targeting constructs was investigated *in vitro* on the NCI-N87 tumor cell line (purchased from ATCC). This human HER2 overexpressing gastric cell line was grown in RPMI1640 (Gibco) supplemented with 10 % FBS (Gibco; heat inactivated at 56 °C for 45 min). 7000 cells in 100 μl growth medium per well were seeded into a 96-well plate. After incubation at 37 °C / 5% $CO_2$ for 24 h, 20 μl of the anti-HER2 constructs Fc-C12, COVA208, COVA210, anti-HER2 antibody 1 or anti-HER2 antibody 2, or combinations of the agents, were added. Each condition was performed in triplicate, and the agents were added in three-fold serial dilutions at concentrations between 300 nM and 0.015 nM. For combinations, each agent was used at the indicated concentration (e.g. 300 nM Fc-C12 + 300 nM anti-HER2 antibody 1). After 5 days, the viability of the treated cultures was analyzed with XTT (Roche). The XTT reagent is converted by metabolically active cells into a colored formazan product which absorbs light at 450 nm wavelength. The absorbance directly correlates with the live cell number. The % viability relative to PBS treated cells was calculated according to the formula:

$$\%viability = \left( \frac{OD_{experimental} - OD_{blank}}{OD_{untreated} - OD_{blank}} \right) x100$$

The average % viability was plotted against $\log_{10}$ (concentration) , and the resulting dose- response curves were analyzed by nonlinear regression with the software *Prism,* using the three parameter equation:

$$\%viability = bottom + \frac{top - bottom}{1 + 10^{x - LogIC_{50}}}$$

Results:

The fusion of Fyn SH3 derived binder C12 to the C-terminus of human Fcγ1, Fc-C12, did not have any effect on cell viability (Figure 2A and 2C). When added in combination with anti-HER2 antibody 1 or anti-HER2 antibody 2, Fc-C12 did not increase or decrease the activity of these two antibodies significantly (Figure 2A and 2C). However, when clone C12 was fused to the N-terminus of the light chain of the anti-HER2 antibody 1 (COVA208) or anti-HER2 antibody 2 (COVA210) to generate binding molecules of the invention, it increased the antiproliferative effect of the unmodified corresponding antibodies (Figure 2B and 2D).

In summary, these results show that the molecules COVA208 and COVA210 are superior to the combination of the individual monospecific binding proteins.

**<u>Example 4</u>: The anti-proliferative activity of anti-HER2 Fynomer-antibody fusions is different depending on the relative orientation of the Fynomer and the binding site of the antibody**

Several different C12 - antibody fusions were tested for their ability to inhibit growth of NCI-N87 tumor cells in order to investigate the influence of the fusion site where the Fyn SH3-derived sequence is attached to the antibody.

Methods:

COVA201 (SEQ ID NO:156; SEQ ID NO:155), COVA202 (SEQ ID NO:154; SEQ ID NO:157), COVA207 (SEQ ID NO:158; SEQ ID NO:155) and COVA208 (SEQ ID NO:154; SEQ ID NO:159) are all C12-anti-HER2 antibody 1 fusions in which the clone C12 is fused to either the C-terminus of the heavy chain (COVA201), C-terminus of the light chain (COVA202), N-terminus of the heavy chain (COVA207) and N-terminus of the light chain (COVA208). Expression and purification was performed as described for COVA208 in Example 3. The cell growth inhibition assay was performed on NCI-N87 cells as described in Example 3.

Results:

The different C12-anti-HER2 antibody 1 formats were found to exhibit different activities (Figure 3A and 3B). COVA208 was most efficacious at inhibiting tumor cell growth and reduced the relative viability to 37%. COVA207 and COVA201 showed intermediate activity (viability: 52% and 61%, respectively) while COVA202 was less active and reduced the viability to 67%, but was still better than anti-HER2 antibody 1 (81 - 82 % viability).
These results show that fusions of one pair of a Fyn SH3-derived sequence and an antibody have different activities, depending on the site of fusion and that the N-terminal light chain fusion of C12 to anti-HER2 antibody 1 (=COVA208) showed the strongest anti-proliferative efficacy.

**<u>Example 5</u>: COVA208 inhibits the growth of BT-474 cells with higher efficacy than anti-HER2 antibody 1**

Methods:

The tumor cell growth inhibition of COVA208 (SEQ ID NOs: 154 and 159) was compared to anti-HER2 antibody 1 (SEQ ID NO: 154 and 155) on the human breast tumor cell line BT-474 (purchased from ATCC). This HER2 overexpressing cell line is one of the best characterized models to study the activity of HER2 targeted agents. BT-474 cells were grown in DMEM/F12 medium (Gibco) supplemented with 10% heat-inactivated FBS (Gibco) and 10 μg/ml human recombinant insulin. The assay was performed as described in Example 3 for NCI-N87 cells.

Results:

COVA208 showed better antiproliferative activity than the anti-HER2 antibody 1 (Figure 4).

**Example 6: COVA208 inhibits NCI-N87 tumor growth *in vivo* more efficiently than the anti-HER2 antibody 1**

COVA208 was investigated *in vivo* for tumor growth inhibition and was compared to anti-HER2 antibody 1.

Methods:

$5 \times 10^6$ human gastric tumor cells (ATCC; CRL-5822) were implanted s.c. into athymic CD-1 Nude mice (Charles River). Tumor dimensions and body weights were recorded three times weekly. The tumor volume was calculated according to the formula $volume = (width)^2 \times length \times \pi/6$. When the average tumor size reached about 140 $mm^3$, which was 42 days after tumor inoculation, mice were randomized into four treatment groups comprising six mice each, and the treatment was initiated. COVA208 (SEQ ID NOs: 154 and 159) and anti-HER2 antibody 1 (SEQ ID NOs: 154 and 155) were administered i.p. once a week for four weeks (five injections in total). The first (loading) dose was 30 mg/kg, and each following (maintenance) dose was 15 mg/kg. Mice in the control group were injected with PBS.

Results:

Anti-HER2 antibody 1 treatment resulted in only weak tumor growth inhibition (Figure 5). COVA208 showed improved tumor growth control for the duration of the treatment compared to anti-HER2 antibody 1. On day 32, the tumors in COVA208 treated mice were reduced in volume by 8 % compared to the initial tumor size at the beginning of the treatment (d = 0), whereas the anti-HER2 antibody 1-treated mice showed an increase in volume by 88%.
This result demonstrates that COVA208 shows significant superior efficacy *in vivo* compared to anti-HER2 antibody 1.

**Example 7: COVA208 exhibits an antibody-like PK profile *in vivo***

Methods:

The pharmacokinetic profile of COVA208 in C57BL/ 6 mice (Charles River) was investigated and compared to anti-HER2 antibody 1. Three C57BL/ 6 mice were injected i.v. with 200 μg COVA208 (SEQ ID NOs: 154 and 159) or anti- HER2 antibody 1 (SEQ ID NOs: 154 and 155) . After 10 min, 6, 24, 48, 96, 120, 144 and 168 hours, blood was collected into EDTA coated microvettes (Sarstedt) , centrifuged for 10 min at 9300 g and the serum levels of COVA208 or anti- HER2 antibody 1 were determined by ELISA. Black maxisorp microtiter plates (Nunc) were coated with 50 nM HER2 ECD (Bender MedSystems) . After blocking with 4 % milk (Rapilait, Migros, Switzerland) in PBS, 40 μl of PBS and 10 μl of serum at appropriate dilution were applied. After incubation for 1 hr, wells were washed with PBS, and bound COVA208 or anti- HER2 antibody 1 were detected with protein A- HRP conjugate (Sigma) . The assay was developed with QuantaRed fluorogenic substrate (Pierce) and the fluorescence intensity was measured after 5 to 10 min at 544 nm (excitation) and 590 nm (emission) .
The serum levels of COVA208 and anti-HER2 antibody 1 were determined using a standard curve of COVA208 and anti-HER2 antibody 1 (diluted to 333 - 0.5 ng/ml each). From the concentrations of COVA208 and anti-HER2 antibody 1 determined in serum at different time points and the resulting slope k of the elimination phase (plotted in a semi-logarithmic scale), the half-lives were calculated using to the formula $t^{1/2} = \ln 2/-k$.

Results:

As shown in Figure 6, the half-lives of COVA208 and the anti-HER2 antibody 1 as determined from the elimination phase (beta phase, time-points 24h - 168h) were highly similar (247 and 187 h, respectively). These data demonstrate that COVA208 has drug-like *in vivo* PK properties.

**Example 8: COVA208 is stable and does not aggregate**

The integrity and stability of COVA208 was assessed by SDS-PAGE and by size exclusion chromatography.

Methods

Purified COVA208 (SEQ ID NOs: 154 and 159) and anti- HER2 antibody 1 (SEQ ID NOs: 154 and 155) were analyzed by SDS- PAGE. 4 μg protein were loaded either with reduced or with nonreduced disulphide bonds onto a 4- 12% Bis/ Tris Novex gel in 1x MOPS running buffer (Invitrogen) , together with a molecular weight marker (RPN800e; GE healthcare) . Protein bands were visualized by coomassie staining.
The size exclusion chromatography (SEC) profile of COVA208 was determined immediately after purification as well as after storage of the protein in PBS at 4 °C for one or two months. 100 μl COVA208 at a concentration of 1.75 mg/mL was loaded onto a Superdex 200 10/300 GL column in PBS (GE healthcare) at a flow rate of 0.5 ml/min, and the elution from the column was monitored by reading the $OD_{280}$,

Results:

The results of the SDS-PAGE and the SEC profiles of COVA208 are shown in Figure 7. COVA208 runs in clearly defined bands at the expected molecular weight on an SDS-PAGE (*top*). Of particular interest is the finding that there is no native light chain detectable in COVA208 (MW around 30 kDa), indicating that there is no cleavage of the Fyn SH3-derived clone C12 from the antibody light chain. COVA208 eluted in one main peak form the SEC column with a retention volume of 13.1 ml (*bottom*). Anti-HER2 antibody 1 eluted at 13.2 ml. Most importantly, no aggregates, which would elute at around 8 ml, were detectable in the COVA208 protein preparation.
The SEC profile of COVA208 did not change over two months of storage at 4 °C. The elution peak remained narrow, symmetrical and appeared at the same retention volume. The protein preparation remained free of aggregates after 1 and 2 months of storage. This indicates that COVA208 remains stable over extended periods of storage at 4°C.
In summary, these results support that COVA208 is a stable, monodisperse molecule with optimal biophysical properties.

## Claims

1. A HER2-specific binding molecule that specifically binds to two different epitopes of HER2 comprising:

   (a) a first binding (poly)peptide that specifically binds to a first epitope of HER2; and
   (b) a second binding (poly)peptide that specifically binds to a second epitope of HER2.

2. The HER2-specific binding molecule of claim 1, wherein binding of the HER2-specific binding molecule to tumor cells expressing HER2 on their surface results in a synergistic inhibition of tumor activity that is higher than the inhibition of tumor activity obtained by the combined binding of two mono-specific HER2-binding molecules, wherein the first mono-specific HER2-binding molecule comprises the binding (poly)peptide of (a) and the second mono-specific HER2-binding molecule comprises the binding (poly)peptide of (b)

3. The HER2-specific binding molecule of claim 1 or 2, wherein the first and the second binding (poly)peptide are independently selected from the group consisting of a Fyn SH3-derived polypeptide and an antibody.

4. The HER2-specific binding molecule of any one of claims 1 to 3, wherein at least the first binding (poly)peptide is a Fyn SH3-derived polypeptide.

5. The HER2-specific binding molecule of claim 4, wherein the Fyn SH3-derived polypeptide comprises or consists of

   (i) an amino acid sequence selected from the group consisting of SEQ ID NO: 1 to 152 or
   (ii) an amino acid sequence having at least 65% sequence identity to the amino acid sequence of SEQ ID NO: 1.

6. The HER2-specific binding molecule according to any one of claims 1 to 5, wherein the second binding (poly)peptide is an antibody.

7. The HER2-specific binding molecule of claim 6, wherein the antibody is selected from the group consisting of an antibody, wherein

(i) the heavy chain of the antibody comprises or consists of the amino acid sequence of SEQ ID NO: 154 and the light chain of the antibody comprises or consists of the amino acid sequence of SEQ ID NO: 155;
(ii) the heavy chain of the antibody comprises or consists of SEQ ID NO: 160 and the light chain of the antibody comprises or consists of the amino acid sequence of SEQ ID NO: 163;
(iii) the heavy chain of the antibody comprises or consists of an amino acid sequence having at least 65% sequence identity to the amino acid sequence of SEQ ID NO: 154 and the light chain of the antibody comprises or consists of an amino acid sequence having at least 65% sequence identity to the amino acid sequence of SEQ ID NO: 155; or
(iv) the heavy chain of the antibody comprises or consists of an amino acid sequence having at least 65% sequence identity to the amino acid sequence of SEQ ID NO: 160 and the light chain of the antibody comprises or consists of an amino acid sequence having at least 65% sequence identity to the amino acid sequence of SEQ ID NO: 163.

8. The HER2-specific binding molecule according to any one of claims 1 to 7, wherein the first and second binding (poly)peptide are linked by a linker.

9. The HER2-specific binding molecule according to any one of claims 1 to 8, further comprising at least one additional (poly)peptide.

10. A nucleic acid molecule encoding the HER2-specific binding molecule of any one of claims 1 to 9.

11. A vector comprising the nucleic acid molecule of claim 10.

12. A host cell or a non-human host transformed with the vector of claim 11.

13. A method for the production of a HER2-specific binding molecule comprising culture of the host cell of claim 12 under suitable conditions and isolation of the HER2-specific binding molecule produced.

14. A composition comprising at least one of

(i) the HER2-specific binding molecule of any one of claims 1 to 8;
(ii) the nucleic acid molecule of claim 9;
(iii) the vector of claim 10; or
(iv) the host cell of claim 12.

15. The HER2-specific binding molecule of any one of claims 1 to 9 or the nucleic acid molecule of claim 10 or the vector of claim 11 or the host cell of claim 12 for use in the treatment of tumors.

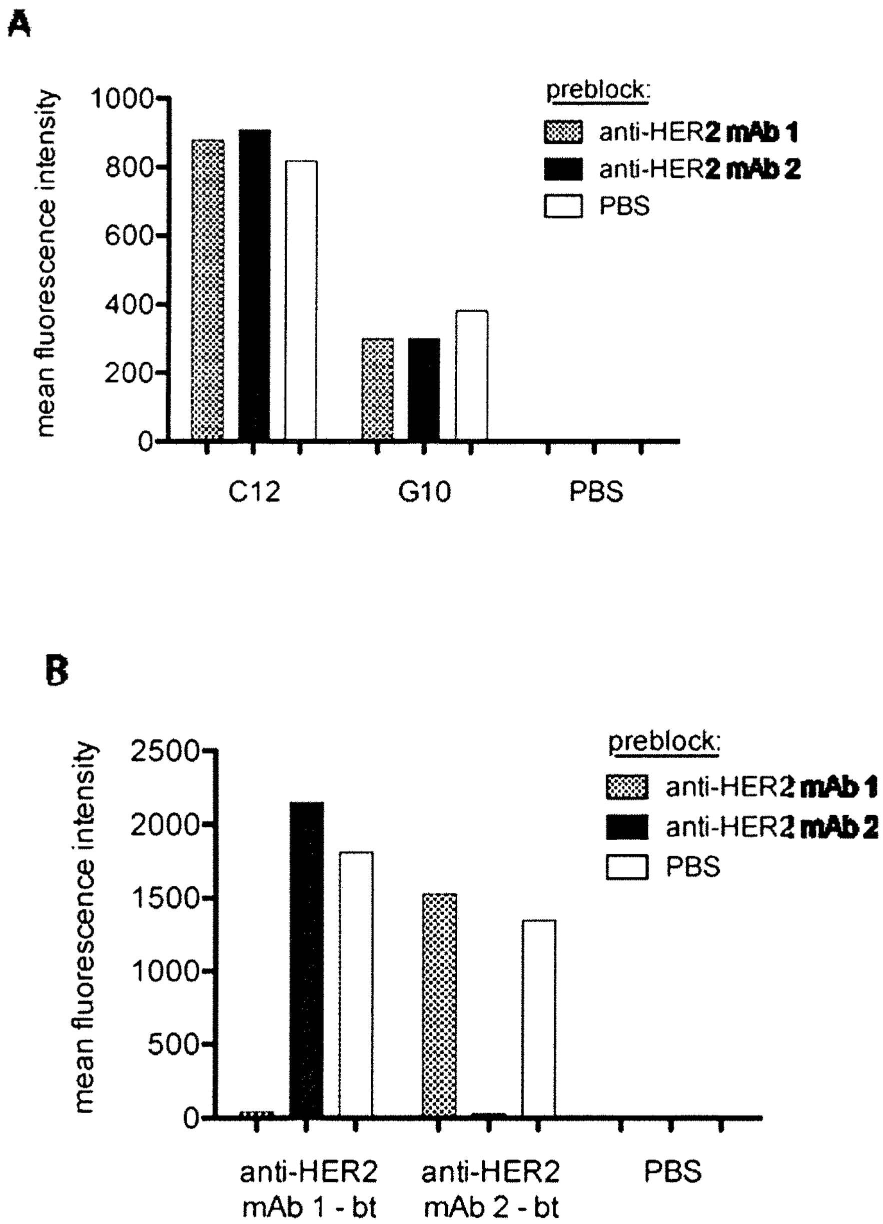
A
mean fluorescence intensity
1000
800
600
400
200
0
preblock:
anti-HER2 mAb 1
anti-HER2 mAb 2
PBS
C12
G10
PBS
B
mean fluorescence intensity
2500
2000
1500
1000
500
0
preblock:
anti-HER2 mAb 1
anti-HER2 mAb 2
PBS
anti-HER2 mAb 1 - bt
anti-HER2 mAb 2 - bt
PBS

**Figure 1**

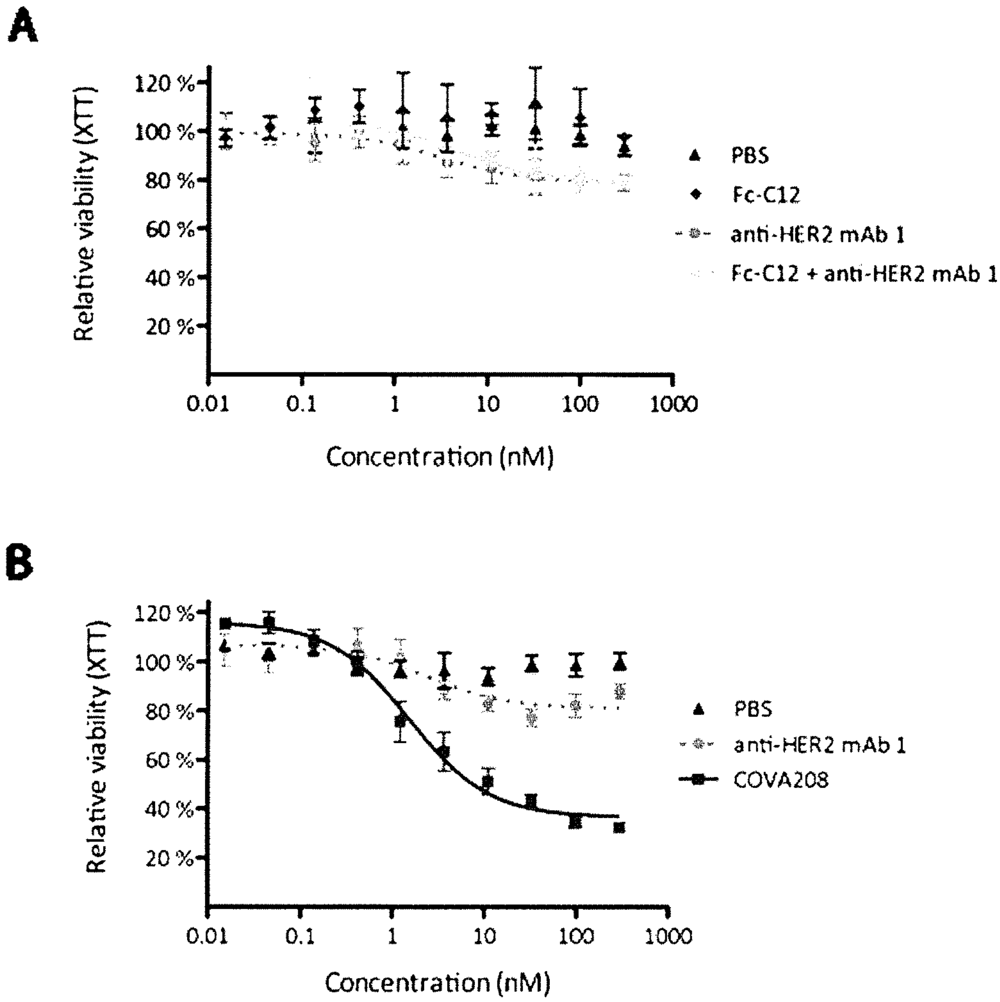
A
120 %
100 %
80 %
60 %
40 %
20 %
Relative viability (XTT)
0.01
0.1
1
10
100
1000
Concentration (nM)
PBS
Fc-C12
anti-HER2 mAb 1
Fc-C12 + anti-HER2 mAb 1
B
120 %
100 %
80 %
60 %
40 %
20 %
Relative viability (XTT)
0.01
0.1
1
10
100
1000
Concentration (nM)
PBS
anti-HER2 mAb 1
COVA208

Figure 2

C

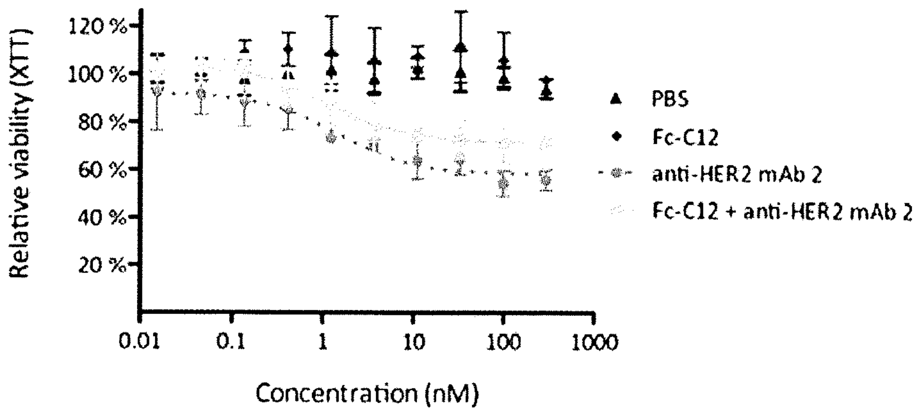


D

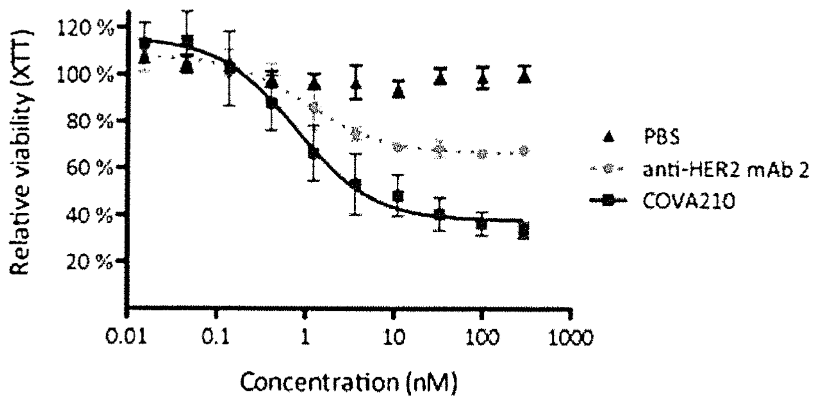


Figure 2 continued

A

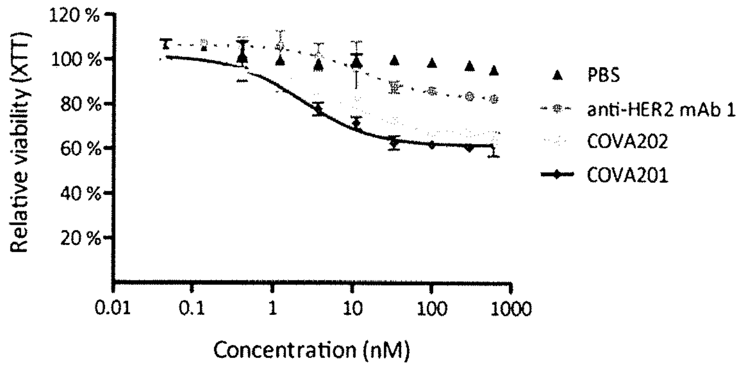


| | maximal effect (% viability) |
|---|---|
| anti-HER2 mAb 1 | 82 |
| COVA201 | 61 |
| COVA202 | 67 |

B

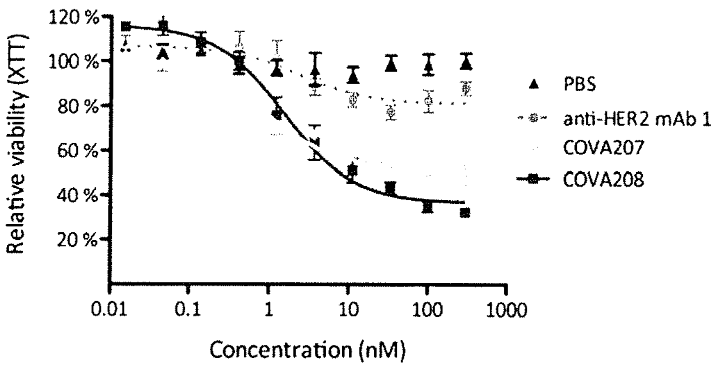


| | maximal effect (% viability) |
|---|---|
| anti-HER2 mAb 1 | 81 |
| COVA207 | 52 |
| COVA208 | 37 |

Figure 3

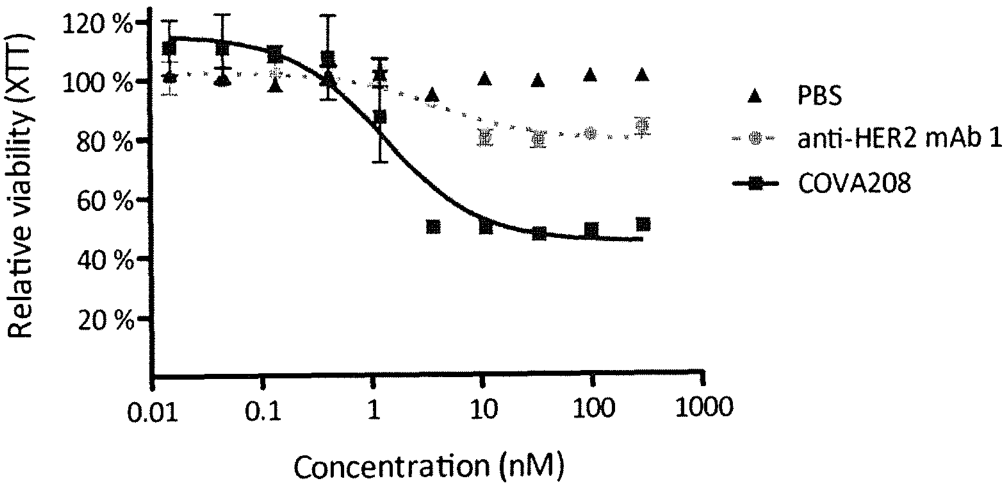
120 %
100 %
80 %
60 %
40 %
20 %
Relative viability (XTT)
0.01
0.1
1
10
100
1000
Concentration (nM)
PBS
anti-HER2 mAb 1
COVA208

**Figure 4**

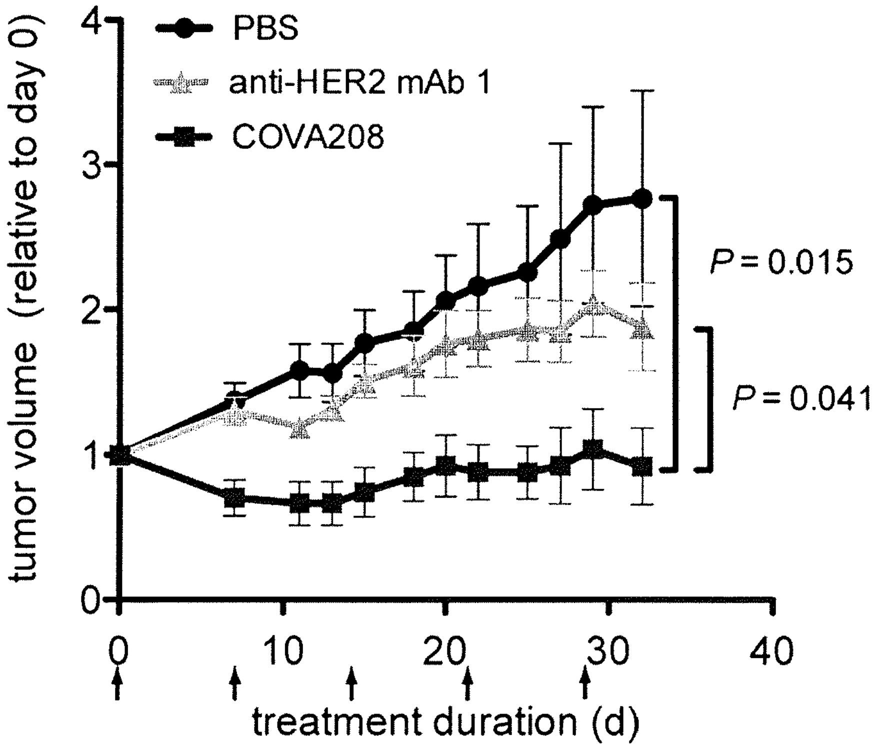
PBS
anti-HER2 mAb 1
COVA208
tumor volume (relative to day 0)
0
1
2
3
4
0
10
20
30
40
treatment duration (d)
P = 0.015
P = 0.041

Figure 5

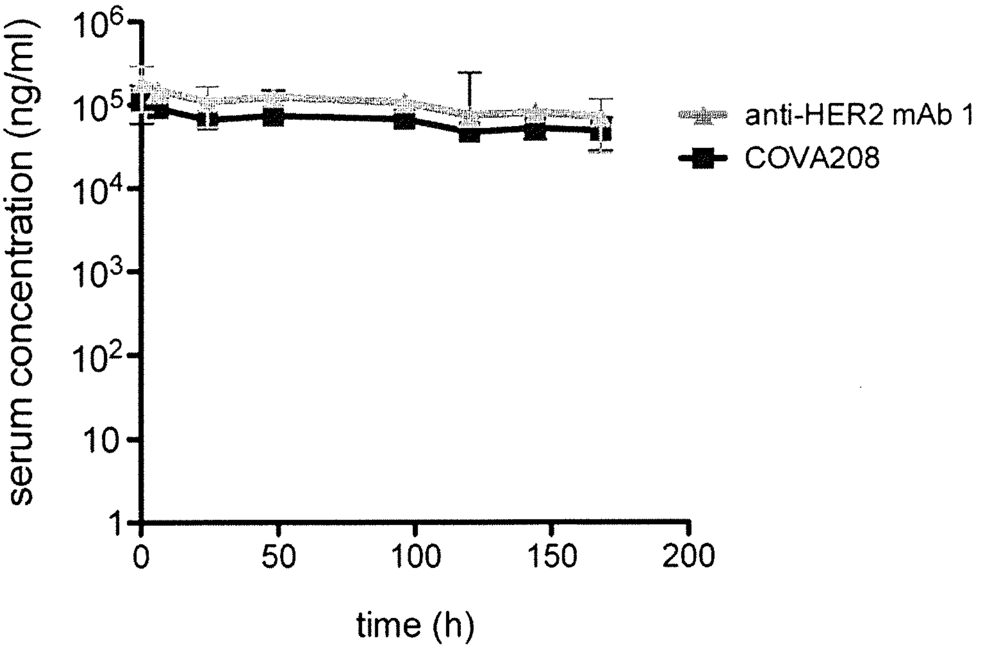
serum concentration (ng/ml)
$10^6$
$10^5$
$10^4$
$10^3$
$10^2$
10
1
0
50
100
150
200
time (h)
anti-HER2 mAb 1
COVA208

Figure 6

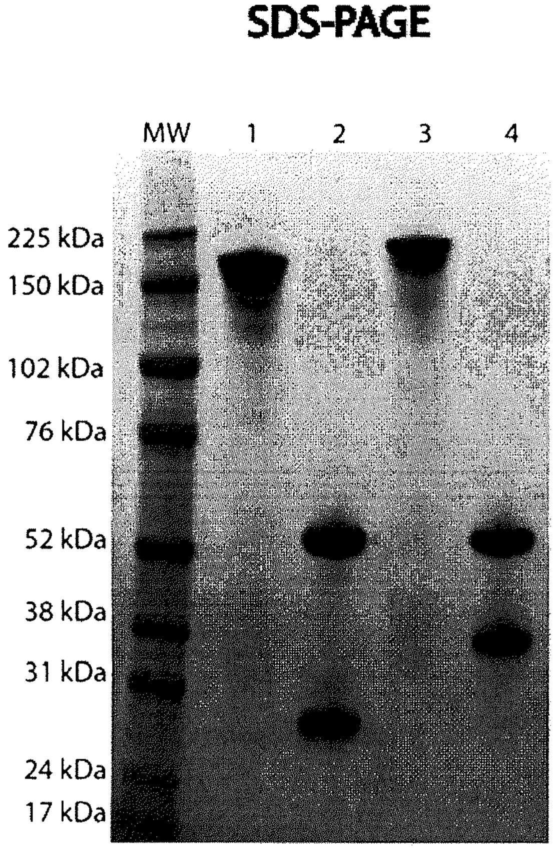


**MW: molecular weight marker**
**lane 1: anti-HER2 mAb 1**
**lane 2: anti-HER2 mAb 1 (reduced)**
**lane 3: COVA208**
**lane 4: COVA208 (reduced)**

Figure 7

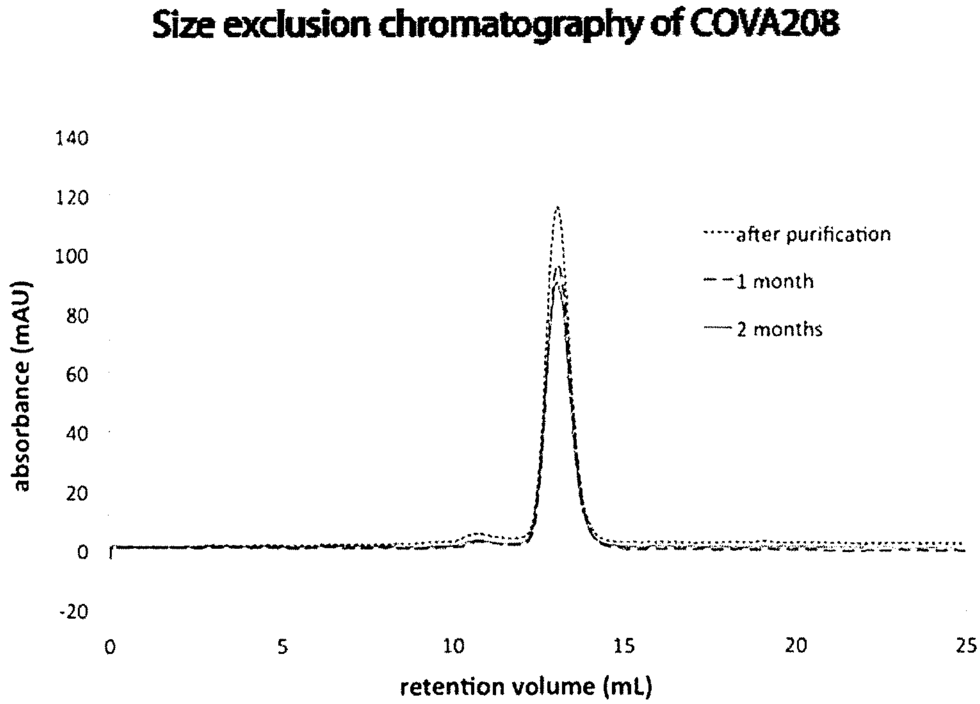


Figure 7 continued

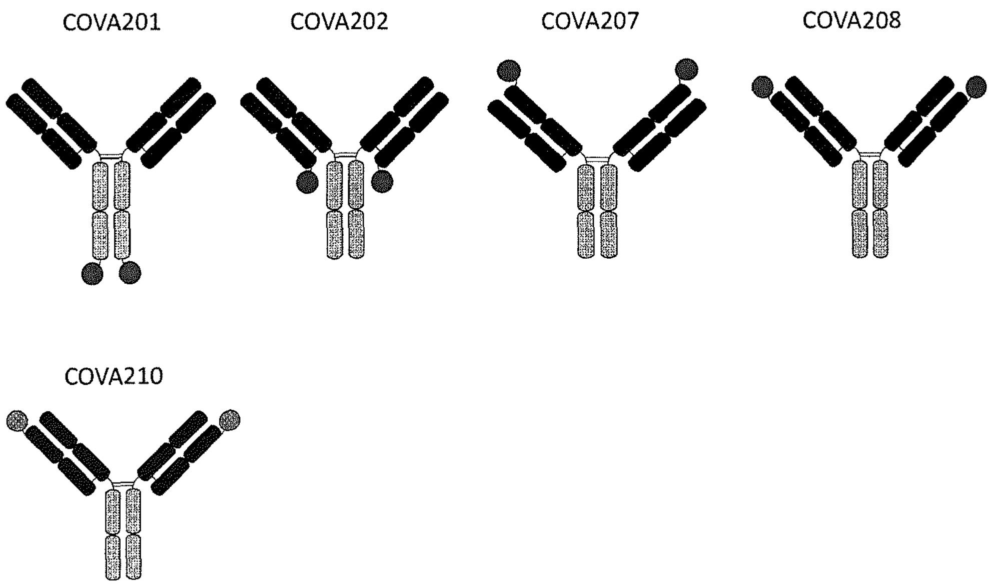


Figure 8

Europäisches Patentamt
European Patent Office
Office européen des brevets

# EUROPEAN SEARCH REPORT

Application Number
EP 12 15 9936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2008/022759 A2 (EIDGENOESS TECH HOCHSCHULE [CH]; GRABULOVSKI DRAGAN [CH]; NERI DARIO [])<br>28 February 2008 (2008-02-28)<br>* the whole document *<br>----- | 3-5 | INV.<br>A61K39/395<br>C07K16/32<br>A61P35/00 |
| Y,D | GRABULOVSKI DRAGAN ET AL: "A novel, non-immunogenic Fyn SH3-derived binding protein with tumor vascular targeting properties",<br>JOURNAL OF BIOLOGICAL CHEMISTRY, THE AMERICAN SOCIETY OF BIOLOGICAL CHEMISTS, INC, US,<br>vol. 282, no. 5,<br>2 February 2007 (2007-02-02), pages 3196-3204, XP002601897,<br>ISSN: 0021-9258, DOI: 10.1074/JBC.M609211200<br>* the whole document *<br>----- | 3-5 | |
| Y | WO 2011/023685 A1 (COVAGEN AG [CH]; GRABULOVSKI DRAGAN [CH]; MELKKO MICHELA SILACCI [CH];) 3 March 2011 (2011-03-03)<br>* the whole document *<br>----- | 3-5 | TECHNICAL FIELDS SEARCHED (IPC)<br>A61K<br>C07K |
| Y,D | J. BERTSCHINGER ET AL: "Selection of single domain binding proteins by covalent DNA display",<br>PROTEIN ENGINEERING DESIGN AND SELECTION,<br>vol. 20, no. 2,<br>1 January 2007 (2007-01-01), pages 57-68, XP055034023,<br>ISSN: 1741-0126, DOI: 10.1093/protein/gzl055<br>* the whole document *<br>-----<br>-/-- | 3-5 | |

The present search report has been drawn up for all claims

1

| Place of search | Date of completion of the search | Examiner |
|---|---|---|
| Munich | 9 August 2012 | Valcárcel, Rafael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 15 9936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/007398 A1 (GENENTECH INC [US]; KELSEY STEPHEN M [US]) 19 January 2006 (2006-01-19) | 1-15 | |
| Y | * the whole document * | 3-5 | |
| X | WO 2009/052249 A1 (GENENTECH INC [US]; MAO WEIGUANG [US]; JUNUTULA JAGATH REDDY [US]; POL) 23 April 2009 (2009-04-23) | 1-15 | |
| Y | * the whole document * | 1-15 | |
| X | WO 2011/069104 A2 (GENENTECH INC [US]; HOFFMANN LA ROCHE [CH]; SCHEER JUSTIN [US]; VANDLE) 9 June 2011 (2011-06-09) | 1-15 | |
| Y | * the whole document * | 1-15 | |
| X | WO 2011/147986 A1 (GENMAB AS [DK]; GOEIJ BART DE [NL]; HAIJ SIMONE DE [NL]; RIEDL THILO [) 1 December 2011 (2011-12-01) | 1,3-15 | |
| Y | * the whole document * | 1-15 | |
| Y | US 2006/099205 A1 (ADAMS GREGORY P [US] ET AL) 11 May 2006 (2006-05-11) * the whole document * | 1-15 | TECHNICAL FIELDS SEARCHED (IPC) |

-/--

The present search report has been drawn up for all claims

1

| Place of search | Date of completion of the search | Examiner |
|---|---|---|
| Munich | 9 August 2012 | Valcárcel, Rafael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 15 9936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br>Y | EUGEN DHIMOLEA AND JANICE M REICHERT: "World Bispecific Antibody Summit, September 27 28, 2011, Boston, MA", MABS, LANDES BIOSCIENCE, US, vol. 4, no. 1, 1 January 2012 (2012-01-01), pages 4-16, XP009156606, ISSN: 1942-0862, DOI: 10.4161/MABS.4.1.18821<br>* For bispecific antibodies binding two different epitopes of HER2, see page 8, right column: discussion of data presented by Max Woisetschläger (f-star).<br>For fusion of a fynomer having hight affinity to IL17A with a heavy chain of a human anti-TNFalpha antibody, see page 16, COVA301. *<br>----- | 1,3-15<br><br><br><br><br><br><br><br>3-5,9 | |
| Y,D | WO 2011/020033 A2 (MASSACHUSETTS INST TECHNOLOGY [US]; WITTRUP K DANE [US]; SPANGLER JAMI)<br>17 February 2011 (2011-02-17)<br>* the whole document *<br>----- | 1-15 | TECHNICAL FIELDS SEARCHED (IPC) |
| Y | WO 2011/073208 A1 (SCIL PROTEINS GMBH [DE]; STEUERNAGEL ARND [DE]; FIEDLER ERIK [DE]; FIE) 23 June 2011 (2011-06-23)<br>* the whole document *<br>-----<br>-/-- | 1-15 | |

The present search report has been drawn up for all claims

1

| Place of search | Date of completion of the search | Examiner |
|---|---|---|
| Munich | 9 August 2012 | Valcárcel, Rafael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 15 9936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PERERA RUSHIKA M ET AL: "Treatment of human tumor xenografts with monoclonal antibody 806 in combination with a prototypical epidermal growth factor receptor-specific antibody generates enhanced antitumor activity", CLINICAL CANCER RESEARCH, THE AMERICAN ASSOCIATION FOR CANCER RESEARCH, US, vol. 11, no. 17, 1 September 2005 (2005-09-01), pages 6390-6399, XP002451509, ISSN: 1078-0432, DOI: 10.1158/1078-0432.CCR-04-2653 * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| Munich | 9 August 2012 | Valcárcel, Rafael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number

EP 12 15 9936

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 12 15 9936

The Search Division considers that the present European patent application does not comply with the requirements of unity of invention and relates to several inventions or groups of inventions, namely:

1. claims: 1, 2, 6-15(all partially)

   HER2 binding molecules that bind to two different epitopes of HER2, including e.g. bispecific antibodies binding to two different epitopes of HER2, and wherein said molecule does not comprise a Fyn SH3-derived polypeptide.

   ---

2. claims: 3-5(completely); 1, 2, 6-15(partially)

   HER2 binding molecules that bind to two different epitopes of HER2, wherein said molecule comprises a Fyn SH3-derived polypeptide.

   ---

## ANNEX TO THE EUROPEAN SEARCH REPORT ON EUROPEAN PATENT APPLICATION NO. EP 12 15 9936

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2012

| Patent document cited in search report | Publication date | Patent family member(s) | Publication date |
|---|---|---|---|
| WO 2008022759 A2 | 28-02-2008 | AU 2007287807 A1 | 28-02-2008 |
| | | CA 2661160 A1 | 28-02-2008 |
| | | CN 101506230 A | 12-08-2009 |
| | | EP 1892248 A1 | 27-02-2008 |
| | | EP 2054432 A2 | 06-05-2009 |
| | | JP 2010500875 A | 14-01-2010 |
| | | KR 20090053926 A | 28-05-2009 |
| | | NZ 574889 A | 25-11-2011 |
| | | RU 2009110180 A | 27-09-2010 |
| | | US 2010119446 A1 | 13-05-2010 |
| | | WO 2008022759 A2 | 28-02-2008 |
| WO 2011023685 A1 | 03-03-2011 | AU 2010288542 A1 | 15-03-2012 |
| | | CA 2772162 A1 | 03-03-2011 |
| | | EP 2470556 A1 | 04-07-2012 |
| | | WO 2011023685 A1 | 03-03-2011 |
| WO 2006007398 A1 | 19-01-2006 | AR 049305 A1 | 12-07-2006 |
| | | AU 2005262459 A1 | 19-01-2006 |
| | | AU 2011200947 A1 | 24-03-2011 |
| | | BR PI0511377 A | 04-12-2007 |
| | | CA 2567808 A1 | 19-01-2006 |
| | | CN 101014366 A | 08-08-2007 |
| | | EP 1755671 A1 | 28-02-2007 |
| | | GT 200500155 A | 15-05-2006 |
| | | JP 2008503476 A | 07-02-2008 |
| | | KR 20070029804 A | 14-03-2007 |
| | | NZ 551376 A | 27-11-2009 |
| | | PA 8637101 A1 | 16-05-2006 |
| | | PE 04612006 A1 | 04-08-2006 |
| | | SV 2006002143 A | 26-01-2006 |
| | | US 2006013819 A1 | 19-01-2006 |
| | | US 2012107391 A1 | 03-05-2012 |
| | | WO 2006007398 A1 | 19-01-2006 |
| | | ZA 200610132 A | 26-08-2009 |
| WO 2009052249 A1 | 23-04-2009 | AR 068941 A1 | 16-12-2009 |
| | | AU 2008312457 A1 | 23-04-2009 |
| | | CA 2698541 A1 | 23-04-2009 |
| | | CN 101835803 A | 15-09-2010 |
| | | CO 6390071 A2 | 29-02-2012 |
| | | EP 2209808 A1 | 28-07-2010 |
| | | JP 2011504460 A | 10-02-2011 |
| | | KR 20100090267 A | 13-08-2010 |
| | | PE 11122009 A1 | 25-07-2009 |
| | | RU 2010119937 A | 27-11-2011 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 12 15 9936

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2012

| Patent document cited in search report | Publication date | Patent family member(s) | Publication date |
|---|---|---|---|
| | | TW 200927172 A | 01-07-2009 |
| | | US 2009117100 A1 | 07-05-2009 |
| | | WO 2009052249 A1 | 23-04-2009 |
| WO 2011069104 A2 | 09-06-2011 | CA 2781682 A1 | 09-06-2011 |
| | | WO 2011069104 A2 | 09-06-2011 |
| WO 2011147986 A1 | 01-12-2011 | NONE | |
| US 2006099205 A1 | 11-05-2006 | AT 530195 T | 15-11-2011 |
| | | AU 2006336279 A1 | 26-07-2007 |
| | | BR PI0611829 A2 | 28-09-2010 |
| | | CA 2612467 A1 | 26-07-2007 |
| | | CN 101355966 A | 28-01-2009 |
| | | DK 1912674 T3 | 13-02-2012 |
| | | EP 1912674 A2 | 23-04-2008 |
| | | EP 2425850 A2 | 07-03-2012 |
| | | ES 2377098 T3 | 22-03-2012 |
| | | JP 4945557 B2 | 06-06-2012 |
| | | JP 2009500005 A | 08-01-2009 |
| | | US 2006099205 A1 | 11-05-2006 |
| | | US 2009010840 A1 | 08-01-2009 |
| | | WO 2007084181 A2 | 26-07-2007 |
| WO 2011020033 A2 | 17-02-2011 | EP 2464663 A2 | 20-06-2012 |
| | | WO 2011020033 A2 | 17-02-2011 |
| WO 2011073208 A1 | 23-06-2011 | CA 2778871 A1 | 23-06-2011 |
| | | CA 2778872 A1 | 23-06-2011 |
| | | CA 2782093 A1 | 23-06-2011 |
| | | EP 2367843 A1 | 28-09-2011 |
| | | EP 2379581 A2 | 26-10-2011 |
| | | KR 20110111304 A | 10-10-2011 |
| | | WO 2011073208 A1 | 23-06-2011 |
| | | WO 2011073209 A1 | 23-06-2011 |
| | | WO 2011073214 A2 | 23-06-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5677171 A
- US 5821337 A
- WO 200100245 A
- WO 2011020033 A
- WO 2008022759 A


### Non-patent literature cited in the description


- **SKERRA.** *J. Mol. Recognit.,* 2000, vol. 13, 167-187
- **GEBAUER ; SKERRA.** *Curr Opinion in Chemical Biology,* 2009, vol. 13, 245-255
- **MUSACCHIO et al.** *Prog. Biophys. Mol. Biol.,* 1994, vol. 61, 283-297
- **MUSACCHIO.** *Advances in Protein Chemistry,* 2003, vol. 61, 211-268
- **REN et al.** *Science,* 1993, vol. 259, 1157-1161
- **KARKKAINEN et al.** *EMBO Rep.,* 2006, vol. 7, 186-191
- **KOYAMA et al.** *Cell,* 1993, vol. 72 (6), 945-952
- **LARSON et al.** *Protein Science,* 2000, vol. 9, 2170-2180
- **SLAMON et al.** *Science,* 1987, vol. 235, 177-182
- **SLAMON et al.** *Science,* 1989, vol. 244, 707-712
- **SCHOLL et al.** *Ann Oncol,* 2001, vol. 12 (1), 81-87
- **ROSS JS.** *Biomark Med,* 2011, vol. 3, 307-318
- **FUKUSHIGE et al.** *Mol Cell Biol,* 1986, vol. 3, 955-958
- **COHEN et al.** *Oncogene,* 1989, vol. 1, 81-88
- **WEINER et al.** *Cancer Res,* 1990, vol. 50, 421-425
- **PARK et al.** *Cancer Res,* 1989, vol. 23, 6605-6609
- **ZHAU et al.** *Mol. Carcinog.,* 1990, vol. 5, 254-257
- **AASLAND et al.** *Br J Cancer,* 1988, vol. 4, 358-363
- **SELIGER et al.** *Int J Cancer,* 2000, vol. 87 (3), 349-359
- **MINNER et al.** *Clin Cancer Res,* 2010, vol. 16 (5), 1553-1560
- **WIKMAN et al.** *Protein Eng Des Sel,* 2004, vol. 17 (5), 455-462
- **ZAHND et al.** *Protein Eng Des Sel,* 2007, vol. 369 (4), 1015-1028
- **HUDZIAK et al.** *Mol Cell Biol,* 1989, vol. 9 (3), 1165-1172
- **AWADA et al.** *Cancer Treat Rev,* 2012, vol. 106 (1), 6-13
- **TSANG et al.** *Br J Cancer,* vol. 106, 6-13
- **NAHTA et al.** *Nat Clin Pract Oncol,* 2006, vol. 3 (5), 269-280
- **TSANG et al.** *Br J Cancer,* 2012, vol. 106, 6-13
- **YAMASHITA-KASHIMA et al.** *Clin Cancer Res,* 2011, vol. 17 (15), 5060-5070
- **SCHEUER et al.** *Cancer Res,* 2009, vol. 69 (24), 9330-9336
- **LEE-HOEFLICH et al.** *Cancer Res,* 2008, vol. 68 (14), 5878-5887
- **KASPRZYK et al.** *Cancer Res,* 1992, vol. 52, 2771-2776
- **BEN-KASUS et al.** *Proc Natl Acad Sci U S A,* 2009, vol. 106 (9), 3294-3299
- **ADAMS C.W. et al.** *Cancer Immunol Immunother,* 2006, vol. 55, 717-727
- **BASELGA et al.** *J Clin Oncol,* 2010, vol. 28, 1138-1144
- **AGUS et al.** *Cancer Cell,* 2002, vol. 2, 127-137
- **FENDLY et al.** *Cancer Res,* 1990, vol. 50 (5), 1550-1558
- **BASELGA et al.** *N Engl J Med,* 2012, vol. 366 (2), 109-119
- **PERERA et al.** *Clin. Cancer Res.,* 2005, vol. 11, 6390-6399
- **WOISETSCHLÄGER M.** *Bispecific Antibody Summit 2011,* 27 September 2011
- **SCHLECHTER et al.** *Nature,* 1984, vol. 312, 513-516
- **ROBINSON et al.** *Oncogene,* 2000, vol. 19, 5548-5557
- **HARLOW ; LANE.** Antibodies: A Laboratory Manual. Cold Spring Harbor Laboratory Press, 1988
- **HARLOW ; LANE.** Using Antibodies: A Laboratory Manual. Cold Spring Harbor Laboratory Press, 1999
- **SELA.** *Science,* 1969, vol. 166, 1365
- **LAVER.** *Cell,* 1990, vol. 61, 553-6

- **STEWART et al.** *Solid Phase Peptide Synthesis,* 1969
- **MERRIFIELD.** *J. Am. Chem. Soc.,* 1963, vol. 85, 2149-2154
- **HOUGHTON.** *Proc. Natl. Acad. Sci. USA,* 1985, vol. 82, 5131-5135
- **GRABULOVSKI et al.** *JBC,* vol. 282, 3196-3204
- **BERTSCHINGER et al.** *Protein Eng Des Sel,* 2007, vol. 20 (2), 57-68
- **SEMBA et al.** *Proc. Natl. Acad. Sci. U S A,* 1986, vol. 83 (15), 5459-63
- **KAWAKAMI et al.** *Mol Cell Biol.,* 1986, vol. 6 (12), 4195-201
- **COOKE ; PERLMUTTER.** *New Biol.,* 1989, vol. 1 (1), 66-74
- **RESH.** *Int. J. Biochem. Cell Biol.,* 1998, vol. 30 (11), 1159-62
- **GRABULOVSKI et al.** *JBC,* 2007, vol. 282, 3196-3204
- **HOOGENBOOM et al.** Multi-subunit proteins on the surface of filamentous phage: methodologies for displaying antibody (Fab) heavy and light chains. *Nucleic Acids Res,* 1991, vol. 19 (15), 4133-7
- **SAMBROOK et al.** Molecular Cloning: A Laboratory Manual. Cold Spring Harbor Laboratory Press, 1989
- **GERHARDT et al.** Methods for General and Molecular Bacteriology. ASM Press, 1994
- **LEFKOVITS.** Immunology Methods Manual: The Comprehensive Sourcebook of Techniques. Academic Press, 1997
- **GOLEMIS.** Protein-Protein Interactions: A Molecular Cloning Manual. Cold Spring Harbor Laboratory Press, 2002
- **STEPHEN F. ALTSCHUL ; THOMAS L. MADDEN ; ALEJANDRO A. SCHÄFFER ; JINGHUI ZHANG ; ZHENG ZHANG ; WEBB MILLER ; DAVID J. LIPMAN.** Gapped BLAST and PSI-BLAST: a new generation of protein database search programs. *Nucleic Acids Res.,* 1997, vol. 25, 3389-3402
- **THOMPSON.** *Nucl. Acids Res.,* 1994, vol. 2, 4673-4680
- **PEARSON ; LIPMAN.** *Proc. Natl. Acad. Sci.,* 1988, vol. 85, 2444
- **HENIKOFF.** *Proc. Natl. Acad. Sci.,* 1989, vol. 89, 10915
- **BRAASCH ; COREY.** *Chem Biol,* 2001, vol. 8, 1
- **OWENS.** *Proc. Natl. Acad. Sci. USA,* 2001, vol. 98, 1471-1476
- **VITI, F. et al.** *Methods Enzymol.,* 2000, vol. 326, 480-505

SEQUENCE LISTING

<110> Covagen AG

<120> Novel bispecific binding molecules with antitumoral activity

<130> U1371 EP

<160> 170

<170> PatentIn version 3.5

<210> 1
<211> 63
<212> PRT
<213> Artificial sequence

<220>
<223> Fynomer #1 C12

<400> 1

```
Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Arg Met
            20                  25                  30

Glu Asp Gly Val Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60
```

<210> 2
<211> 67
<212> PRT
<213> Artificial sequence

<220>
<223> Fynomer #1 G10

<400> 2

```
Gly Val Thr Leu Phe Val Ala Leu Tyr Asp Tyr Glu Ala Arg Asp Ser
1               5                   10                  15

Met Gly Gly Gln Asp Leu Ser Phe His Lys Gly Glu Lys Phe Gln Ile
            20                  25                  30

Leu Lys Arg Trp Arg Gly Gly Asp Trp Trp Glu Ala Arg Ser Leu Thr
        35                  40                  45

Thr Gly Glu Thr Gly Tyr Ile Pro Ser Asn Tyr Val Ala Pro Val Asp
    50                  55                  60

Ser Ile Gln
```

65

<210> 3
<211> 67
<212> PRT
<213> Artificial sequence

<220>
<223> Fynomer #3

<400> 3

```
Gly Val Thr Leu Phe Val Ala Leu Tyr Asp Tyr Glu Ala Arg Asp Ser
1               5                   10                  15

Met Gly Gly Gln Asp Leu Ser Phe His Lys Gly Glu Lys Phe Gln Ile
            20                  25                  30

Leu Thr Arg Trp Ala Gln Gly Asp Trp Trp Glu Ala Arg Ser Leu Thr
        35                  40                  45

Thr Gly Glu Thr Gly Tyr Ile Pro Ser Asn Tyr Val Ala Pro Val Asp
    50                  55                  60

Ser Ile Gln
65
```

<210> 4
<211> 64
<212> PRT
<213> Artificial sequence

<220>
<223> Fynomer #4

<400> 4

```
Gly Val Thr Leu Phe Val Ala Leu Tyr Asp Tyr Gln Ala Tyr Gly Met
1               5                   10                  15

Tyr Asp Leu Ser Phe His Lys Gly Glu Lys Phe Gln Ile Leu Ser Pro
            20                  25                  30

Lys Asp Thr Gly Tyr Trp Trp Glu Ala Arg Ser Leu Thr Thr Gly Glu
        35                  40                  45

Thr Gly Tyr Ile Pro Ser Asn Tyr Val Ala Pro Val Asp Ser Ile Gln
    50                  55                  60
```

<210> 5
<211> 66
<212> PRT
<213> Artificial sequence

<220>

<223> Fynomer #5

<400> 5

Gly Val Thr Leu Phe Val Ala Leu Tyr Asp Tyr Glu Ala Arg Glu Phe
1 5 10 15

Met Gly Gly Gln Asp Leu Ser Phe His Lys Gly Glu Lys Phe Gln Ile
20 25 30

Leu Thr Met Trp Lys Gly Asp Trp Trp Glu Ala Arg Ser Leu Thr Thr
35 40 45

Gly Glu Thr Gly Tyr Ile Pro Ser Asn Tyr Val Ala Pro Val Asp Ser
50 55 60

Ile Gln
65

<210> 6
<211> 67
<212> PRT
<213> Artificial sequence

<220>
<223> Fynomer #6

<400> 6

Gly Val Thr Leu Phe Val Ala Leu Tyr Asp Tyr Glu Ala Arg Pro Tyr
1 5 10 15

Leu Gly Gly Gln Asp Leu Ser Phe His Lys Gly Glu Lys Phe Gln Ile
20 25 30

Leu His Ala Ser Met Leu Gly Asp Trp Trp Glu Ala Arg Ser Leu Thr
35 40 45

Thr Gly Glu Thr Gly Tyr Ile Pro Ser Asn Tyr Val Ala Pro Val Asp
50 55 60

Ser Ile Gln
65

<210> 7
<211> 63
<212> PRT
<213> Artificial sequence

<220>
<223> Fynomer #7

<400> 7

Gly Val Thr Leu Phe Val Ala Leu Tyr Asp Tyr Thr Ser Tyr Asn Gln

1 5 10 15

His Asp Leu Ser Phe His Lys Gly Glu Lys Phe Gln Ile Leu Gly Asp
20 25 30

Asn Ile Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 8
<211> 67
<212> PRT
<213> Artificial sequence

<220>
<223> Fynomer #8

<400> 8

Gly Val Thr Leu Phe Val Ala Leu Tyr Asp Tyr Glu Ala Arg Leu Ser
1 5 10 15

Ser His Pro His Val Leu Ser Phe His Lys Gly Glu Lys Phe Gln Ile
20 25 30

Leu Asn Arg Val Ser Ile Gly Asp Trp Trp Glu Ala Arg Ser Leu Thr
35 40 45

Thr Gly Glu Thr Gly Tyr Ile Pro Ser Asn Tyr Val Ala Pro Val Asp
50 55 60

Ser Ile Gln
65

<210> 9
<211> 67
<212> PRT
<213> Artificial sequence

<220>
<223> Fynomer #9

<400> 9

Gly Val Thr Leu Phe Val Ala Leu Tyr Asp Tyr Glu Ala Arg Pro Tyr
1 5 10 15

Leu Gly Gly Gln Asp Leu Ser Phe His Lys Gly Glu Lys Phe Gln Ile
20 25 30

Leu Asn His Pro Pro Thr Gly Asp Trp Trp Glu Ala Arg Ser Leu Thr
35 40 45

```
Thr Gly Glu Thr Gly Tyr Ile Pro Ser Asn Tyr Val Ala Pro Val Asp
    50                  55                  60

Ser Ile Gln
65

<210>  10
<211>  65
<212>  PRT
<213>  Artificial sequence

<220>
<223>  Fynomer #10

<400>  10

Gly Val Thr Leu Phe Val Ala Leu Tyr Asp Tyr Glu Ala Tyr Asp Leu
1               5                   10                  15

Ser Arg Pro Asp Leu Ser Phe His Lys Gly Glu Lys Phe Gln Ile Leu
            20                  25                  30

Asn Ser Ser Glu Gly Thr Trp Trp Glu Ala Arg Ser Leu Thr Thr Gly
        35                  40                  45

Glu Thr Gly Phe Ile Pro Ser Asn Tyr Val Ala Pro Val Asp Ser Ile
    50                  55                  60

Gln
65

<210>  11
<211>  67
<212>  PRT
<213>  Artificial sequence

<220>
<223>  Fynomer #11

<400>  11

Gly Val Thr Leu Phe Val Ala Leu Tyr Asp Tyr Glu Ala Arg Met Pro
1               5                   10                  15

Lys Val Ser Asp Leu Ser Phe His Lys Gly Glu Lys Phe Gln Ile Leu
            20                  25                  30

Gln Glu Pro Gln Ser Lys Gly Asp Trp Trp Glu Ala Arg Ser Leu Thr
        35                  40                  45

Thr Gly Glu Thr Gly Tyr Ile Pro Ser Asn Tyr Val Ala Pro Val Asp
    50                  55                  60
```

Ser Ile Gln
65

<210> 12
<211> 67
<212> PRT
<213> Artificial sequence

<220>
<223> Fynomer #12

<400> 12

```
Gly Val Thr Leu Phe Val Ala Leu Tyr Asp Tyr Glu Ala Arg Pro Gly
1               5                   10                  15

Arg His Ser Ser Asp Leu Ser Phe His Lys Gly Glu Lys Phe Gln Ile
            20                  25                  30

Leu His Gln Ser Asn Val Gly Asp Trp Trp Glu Ala Arg Ser Leu Thr
        35                  40                  45

Thr Gly Glu Thr Gly Tyr Ile Pro Ser Asn Tyr Val Ala Pro Val Asp
    50                  55                  60

Ser Ile Gln
65
```

<210> 13
<211> 64
<212> PRT
<213> Artificial sequence

<220>
<223> Fynomer #13

<400> 13

```
Gly Val Thr Leu Phe Val Ala Leu Tyr Asp Tyr Gln Thr Thr Arg Pro
1               5                   10                  15

His Asp Leu Ser Phe His Lys Gly Glu Lys Phe Gln Ile Leu Gly Arg
            20                  25                  30

Thr Gln Ile Gly Asp Trp Trp Glu Ala Arg Ser Leu Thr Thr Gly Glu
        35                  40                  45

Thr Gly Tyr Ile Pro Ser Asn Tyr Val Ala Pro Val Asp Ser Ile Gln
    50                  55                  60
```

<210> 14
<211> 63
<212> PRT
<213> Artificial sequence

<220>
<223> Fynomer #14

<400> 14

```
Gly Val Thr Leu Phe Val Ala Leu Tyr Asp Tyr Gln Thr Asn Asn Ser
1               5                   10                  15

Arg Asp Leu Ser Phe His Lys Gly Glu Lys Phe Gln Ile Leu Gly Asn
            20                  25                  30

Thr Gln Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60
```

<210> 15
<211> 63
<212> PRT
<213> Artificial sequence

<220>
<223> Fynomer #15

<400> 15

```
Gly Val Thr Leu Phe Val Ala Leu Tyr Asp Tyr Tyr Ser Tyr Asn Thr
1               5                   10                  15

His Asp Leu Ser Phe His Lys Gly Glu Lys Phe Gln Ile Leu Ser Arg
            20                  25                  30

Ala Asn Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60
```

<210> 16
<211> 63
<212> PRT
<213> Artificial sequence

<220>
<223> Fynomer #16

<400> 16

```
Gly Val Thr Leu Phe Val Ala Leu Tyr Asp Tyr Tyr Ser Tyr Asn Asn
1               5                   10                  15

His Asp Leu Ser Phe His Lys Gly Glu Lys Phe Gln Ile Leu Gln Glu
            20                  25                  30
```

```
Leu Gln Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60


<210>  17
<211>  63
<212>  PRT
<213>  Artificial sequence

<220>
<223>  Fynomer #17

<400>  17

Gly Val Thr Leu Phe Val Ala Leu Tyr Asp Tyr Arg Ser Tyr Asn Thr
1               5                   10                  15

Arg Asp Leu Ser Phe His Lys Gly Glu Lys Phe Gln Ile Leu Thr Lys
            20                  25                  30

Ser Ser Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60


<210>  18
<211>  65
<212>  PRT
<213>  Artificial sequence

<220>
<223>  Fynomer #18

<400>  18

Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Lys Ala
            20                  25                  30

His Ser Leu Ala Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65
```

<210> 19
<211> 65
<212> PRT
<213> Artificial sequence

<220>
<223> Fynomer #19

<400> 19

```
Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Ser Glu
            20                  25                  30

Gln Asp Leu Arg Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65
```

<210> 20
<211> 63
<212> PRT
<213> Artificial sequence

<220>
<223> Fynomer #20

<400> 20

```
Gly Val Thr Leu Phe Val Ala Leu Tyr Asp Tyr Thr Ser Tyr Asn Lys
1               5                   10                  15

Leu Asp Leu Ser Phe His Lys Gly Glu Lys Phe Gln Ile Leu Arg Thr
            20                  25                  30

Asp Ala Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60
```

<210> 21
<211> 63
<212> PRT
<213> Artificial sequence

<220>
<223> Fynomer #21

<400> 21

```
Gly Val Thr Leu Phe Val Ala Leu Tyr Asp Tyr Thr Ser Tyr Asn Gln
1               5                   10                  15

Arg Asp Leu Ser Phe His Lys Gly Glu Lys Phe Gln Ile Leu Gly Pro
            20                  25                  30

Lys Leu Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60


<210>  22
<211>  63
<212>  PRT
<213>  Artificial sequence

<220>
<223>  Fynomer #22

<400>  22

Gly Val Thr Leu Phe Val Ala Leu Tyr Asp Tyr Thr Ser Tyr Asn Asn
1               5                   10                  15

His Asp Leu Ser Phe His Lys Gly Glu Lys Phe Gln Ile Leu Asn Gln
            20                  25                  30

His Asn Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60


<210>  23
<211>  63
<212>  PRT
<213>  Artificial sequence

<220>
<223>  Fynomer #23

<400>  23

Gly Val Thr Leu Phe Val Ala Leu Tyr Asp Tyr Thr Ser Tyr Asn Leu
1               5                   10                  15

Arg Asp Leu Ser Phe His Lys Gly Glu Lys Phe Gln Ile Leu Asn Ser
            20                  25                  30

Ser Glu Gly Asp Trp Trp Glu Ala Arg Ser Leu Thr Thr Gly Glu Thr
        35                  40                  45
```

Gly Tyr Ile Pro Ser Asn Tyr Val Ala Pro Val Asp Ser Ile Gln
50 55 60

<210> 24
<211> 65
<212> PRT
<213> Artificial sequence

<220>
<223> Fynomer #24

<400> 24

Gly Val Thr Leu Phe Val Ala Leu Tyr Asp Tyr Thr Ser Tyr Asn Pro
1 5 10 15

Arg Asp Leu Ser Phe His Lys Gly Glu Lys Phe Gln Ile Leu Gly Ala
20 25 30

Thr Asp Ala Thr Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 25
<211> 63
<212> PRT
<213> Artificial sequence

<220>
<223> Fynomer #25

<400> 25

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Asn Glu
20 25 30

Leu Asn Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 26
<211> 63
<212> PRT
<213> Artificial sequence

<220>
<223> Fynomer #26

<400> 26

Gly Val Thr Leu Phe Val Ala Leu Tyr Asp Tyr Thr Ser Tyr Asn Ser
1 5 10 15

Ser Asp Leu Ser Phe His Lys Gly Glu Lys Phe Gln Ile Leu Arg Glu
20 25 30

Ala Asp Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 27
<211> 67
<212> PRT
<213> Artificial sequence

<220>
<223> Fynomer #27

<400> 27

Gly Val Thr Leu Phe Val Ala Leu Tyr Asp Tyr Glu Ala Arg Arg Asp
1 5 10 15

His Ser Pro His Asp Leu Ser Phe His Lys Gly Glu Lys Phe Gln Ile
20 25 30

Leu Asn Leu Tyr Gln Val Gly Asp Trp Trp Glu Ala Arg Ser Leu Thr
35 40 45

Thr Gly Glu Thr Gly Tyr Ile Pro Ser Asn Tyr Val Ala Pro Val Asp
50 55 60

Ser Ile Gln
65

<210> 28
<211> 66
<212> PRT
<213> Artificial sequence

<220>
<223> Fynomer #28

<400> 28

Gly Val Thr Leu Phe Val Ala Leu Tyr Asp Tyr Glu Ala Arg Glu Ala
1 5 10 15

Leu Gly Gly Gln Asp Leu Ser Phe His Lys Gly Glu Lys Phe Gln Ile
20 25 30

Leu Ser Pro Gln Thr Gly Asp Trp Trp Glu Ala Arg Ser Leu Thr Thr
35 40 45

Gly Glu Thr Gly Tyr Ile Pro Ser Asn Tyr Val Ala Pro Val Asp Ser
50 55 60

Ile Gln
65

<210> 29
<211> 64
<212> PRT
<213> Artificial sequence

<220>
<223> Fynomer #29

<400> 29

Gly Val Thr Leu Phe Val Ala Leu Tyr Asp Tyr Tyr Ser Val His Pro
1 5 10 15

Gly Asp Leu Ser Phe His Lys Gly Glu Lys Phe Gln Ile Leu Ser Asn
20 25 30

Tyr Gln Val Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 30
<211> 63
<212> PRT
<213> Artificial sequence

<220>
<223> Fynomer #30

<400> 30

Gly Val Thr Leu Phe Val Ala Leu Tyr Asp Tyr Tyr Ser Tyr Asn Ser
1 5 10 15

Arg Asp Leu Ser Phe His Lys Gly Glu Lys Phe Gln Ile Leu Ala Gln
20 25 30

His Asp Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln

50 55 60

<210> 31
<211> 63
<212> PRT
<213> Artificial sequence

<220>
<223> Fynomer #31

<400> 31

Gly Val Thr Leu Phe Val Ala Leu Tyr Asp Tyr Thr Ser Tyr Asn Met
1 5 10 15

Arg Asp Leu Ser Phe His Lys Gly Glu Lys Phe Gln Ile Leu Gly Arg
20 25 30

Gly Pro Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 32
<211> 65
<212> PRT
<213> Artificial sequence

<220>
<223> Fynomer #32

<400> 32

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Gly Arg
20 25 30

Pro Arg Asp Val Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 33
<211> 63
<212> PRT
<213> Artificial sequence

<220>

<223> Fynomer #33

<400> 33

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu His Thr
20 25 30

Thr Lys Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 34
<211> 65
<212> PRT
<213> Artificial sequence

<220>
<223> Fynomer #34

<400> 34

Gly Val Thr Leu Phe Val Ala Leu Tyr Asp Tyr Thr Ser Tyr Asn Thr
1 5 10 15

Thr Asp Leu Ser Phe His Lys Gly Glu Lys Phe Gln Ile Leu His Trp
20 25 30

Asn Gly Gly Ser Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 35
<211> 63
<212> PRT
<213> Artificial sequence

<220>
<223> Fynomer #35

<400> 35

Gly Val Thr Leu Phe Val Ala Leu Tyr Asp Tyr Thr Ser Tyr Asn Thr
1 5 10 15

Thr Asp Leu Ser Phe His Lys Gly Glu Lys Phe Gln Ile Leu Asn Asn

20 25 30

Thr Thr Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 36
<211> 65
<212> PRT
<213> Artificial sequence

<220>
<223> Fynomer #36

<400> 36

Gly Val Thr Leu Phe Val Ala Leu Tyr Asp Tyr Trp Ser Tyr Asn Ser
1 5 10 15

Arg Asp Leu Ser Phe His Lys Gly Glu Lys Phe Gln Ile Leu Asn Pro
20 25 30

Glu Glu Thr Ser Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 37
<211> 65
<212> PRT
<213> Artificial sequence

<220>
<223> Fynomer #37

<400> 37

Gly Val Thr Leu Phe Val Ala Leu Tyr Asp Tyr Thr Ser Tyr Asn Pro
1 5 10 15

Arg Asp Leu Ser Phe His Lys Gly Glu Lys Phe Gln Ile Leu Asn Pro
20 25 30

Arg Gln Arg Ile Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 38
<211> 63
<212> PRT
<213> Artificial sequence

<220>
<223> Fynomer #38

<400> 38

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Asn Arg
20 25 30

Pro Met Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 39
<211> 65
<212> PRT
<213> Artificial sequence

<220>
<223> Fynomer #39

<400> 39

Gly Val Thr Leu Phe Val Ala Leu Tyr Asp Tyr Thr Ser Tyr Asn Gln
1 5 10 15

His Asp Leu Ser Phe His Lys Gly Glu Lys Phe Gln Ile Leu Pro Thr
20 25 30

Thr Asp Thr Ala Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 40
<211> 63
<212> PRT

<213> Artificial sequence

<220>
<223> Fynomer #40

<400> 40

Gly Val Thr Leu Phe Val Ala Leu Tyr Asp Tyr Thr Ser Tyr Asn Thr
1 5 10 15

Met Asp Leu Ser Phe His Lys Gly Glu Lys Phe Gln Ile Leu Arg Glu
20 25 30

Thr Thr Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 41
<211> 63
<212> PRT
<213> Artificial sequence

<220>
<223> Fynomer #41

<400> 41

Gly Val Thr Leu Phe Val Ala Leu Tyr Asp Tyr Thr Ser Tyr Asn Thr
1 5 10 15

His Asp Leu Ser Phe His Lys Gly Glu Lys Phe Gln Ile Leu Arg Gln
20 25 30

Asn Asn Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 42
<211> 63
<212> PRT
<213> Artificial sequence

<220>
<223> Fynomer #42

<400> 42

Gly Val Thr Leu Phe Val Ala Leu Tyr Asp Tyr Thr Ser Tyr Asn Ser
1 5 10 15

Thr Asp Leu Ser Phe His Lys Gly Glu Lys Phe Gln Ile Leu Ser Ala
20 25 30

Pro Gln Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 43
<211> 63
<212> PRT
<213> Artificial sequence

<220>
<223> Fynomer #43

<400> 43

Gly Val Thr Leu Phe Val Ala Leu Tyr Asp Tyr Thr Ser Tyr Asn Ala
1 5 10 15

Arg Asp Leu Ser Phe His Lys Gly Glu Lys Phe Gln Ile Leu Ser Ala
20 25 30

Pro Thr Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 44
<211> 65
<212> PRT
<213> Artificial sequence

<220>
<223> Fynomer #44

<400> 44

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Ser Asp
20 25 30

Ala Thr Leu Glu Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 45
<211> 65
<212> PRT
<213> Artificial sequence

<220>
<223> Fynomer #45

<400> 45

Gly Val Thr Leu Phe Val Ala Leu Tyr Asp Tyr Thr Ser Tyr Asn Lys
1 5 10 15

Arg Asp Leu Ser Phe His Lys Gly Glu Lys Phe Gln Ile Leu Ser Glu
20 25 30

Thr Ser Pro Glu Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 46
<211> 65
<212> PRT
<213> Artificial sequence

<220>
<223> Fynomer #46

<400> 46

Gly Val Thr Leu Phe Val Ala Leu Tyr Asp Tyr Val Ser Tyr Asn Thr
1 5 10 15

Thr Asp Leu Ser Phe His Lys Gly Glu Lys Phe Gln Ile Leu Ser His
20 25 30

Thr Thr Ser Gln Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 47
<211> 63
<212> PRT
<213> Artificial sequence

<220>
<223> Fynomer #47

<400> 47

Gly Val Thr Leu Phe Val Ala Leu Tyr Asp Tyr Thr Ser Tyr Asn Thr
1               5                   10                  15

Thr Asp Leu Ser Phe His Lys Gly Glu Lys Phe Gln Ile Leu Ser Met
            20                  25                  30

Ala Val Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210> 48
<211> 65
<212> PRT
<213> Artificial sequence

<220>
<223> Fynomer #48

<400> 48

Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Ser Asn
            20                  25                  30

Gly Pro Asp Pro Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210> 49
<211> 63
<212> PRT
<213> Artificial sequence

<220>
<223> Fynomer #49

<400> 49

Gly Val Thr Leu Phe Val Ala Leu Tyr Asp Tyr Thr Ser Tyr Asn Thr
1               5                   10                  15

Thr Asp Leu Ser Phe His Lys Gly Glu Lys Phe Gln Ile Leu Ser Asn
20 25 30

Pro Pro Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 50
<211> 65
<212> PRT
<213> Artificial sequence

<220>
<223> Fynomer #50

<400> 50

Gly Val Thr Leu Phe Val Ala Leu Tyr Asp Tyr Asn Ser Tyr Asn Lys
1 5 10 15

Arg Asp Leu Ser Phe His Lys Gly Glu Lys Phe Gln Ile Leu Ser Gln
20 25 30

Ala Ala Glu Asp Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 51
<211> 63
<212> PRT
<213> Artificial sequence

<220>
<223> Fynomer #51

<400> 51

Gly Val Thr Leu Phe Val Ala Leu Tyr Asp Tyr Thr Ser Tyr Asn Thr
1 5 10 15

Lys Asp Leu Ser Phe His Lys Gly Glu Lys Phe Gln Ile Leu Ser Gln
20 25 30

Ser Gly Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
    50                  55                  60

<210> 52
<211> 65
<212> PRT
<213> Artificial sequence

<220>
<223> Fynomer #52

<400> 52

Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Ser Tyr
            20                  25                  30

Pro Arg Thr Gln Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210> 53
<211> 65
<212> PRT
<213> Artificial sequence

<220>
<223> Fynomer #53

<400> 53

Gly Val Thr Leu Phe Val Ala Leu Tyr Asp Tyr Thr Ser Tyr Asn Glu
1               5                   10                  15

Arg Asp Leu Ser Phe His Lys Gly Glu Lys Phe Gln Ile Leu Thr Lys
            20                  25                  30

Thr Pro Arg Gln Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210> 54

<211> 65
<212> PRT
<213> Artificial sequence

<220>
<223> Fynomer #54

<400> 54

Gly Val Thr Leu Phe Val Ala Leu Tyr Asp Tyr Val Ser Tyr Asn Thr
1 5 10 15

Asn Asp Leu Ser Phe His Lys Gly Glu Lys Phe Gln Ile Leu Asp Ser
20 25 30

Gln Glu Pro Asn Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 55
<211> 65
<212> PRT
<213> Artificial sequence

<220>
<223> Fynomer #55

<400> 55

Gly Val Thr Leu Phe Val Ala Leu Tyr Asp Tyr Thr Ser Tyr Asn Gln
1 5 10 15

His Asp Leu Ser Phe His Lys Gly Glu Lys Phe Gln Ile Leu Gly Gln
20 25 30

Tyr Pro Lys Thr Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 56
<211> 65
<212> PRT
<213> Artificial sequence

<220>

<223> Fynomer #56

<400> 56

Gly Val Thr Leu Phe Val Ala Leu Tyr Asp Tyr Thr Ser Tyr Asn Asn
1 5 10 15

Thr Asp Leu Ser Phe His Lys Gly Glu Lys Phe Gln Ile Leu Lys Gln
20 25 30

Gln Ala Gly Asp Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 57
<211> 65
<212> PRT
<213> Artificial sequence

<220>
<223> Fynomer #57

<400> 57

Gly Val Thr Leu Phe Val Ala Leu Tyr Asp Tyr Thr Ser Tyr Asn Asn
1 5 10 15

Thr Asp Leu Ser Phe His Lys Gly Glu Lys Phe Gln Ile Leu Asn Ala
20 25 30

His Gln Ser Ala Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 58
<211> 65
<212> PRT
<213> Artificial sequence

<220>
<223> Fynomer #58

<400> 58

Gly Val Thr Leu Phe Val Ala Leu Tyr Asp Tyr Thr Ser Tyr Asn Thr

1               5                   10                  15

Arg Asp Leu Ser Phe His Lys Gly Glu Lys Phe Gln Ile Leu Asn Pro
            20                  25                  30

Gln Ser Arg Thr Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210> 59
<211> 65
<212> PRT
<213> Artificial sequence

<220>
<223> Fynomer #59

<400> 59

Gly Val Thr Leu Phe Val Ala Leu Tyr Asp Tyr Thr Ser Tyr Asn Ser
1               5                   10                  15

His Asp Leu Ser Phe His Lys Gly Glu Lys Phe Gln Ile Leu Pro Gly
            20                  25                  30

Gln Ser Met Thr Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210> 60
<211> 65
<212> PRT
<213> Artificial sequence

<220>
<223> Fynomer #60

<400> 60

Gly Val Thr Leu Phe Val Ala Leu Tyr Asp Tyr Thr Ser Tyr Asn Ala
1               5                   10                  15

His Asp Leu Ser Phe His Lys Gly Glu Lys Phe Gln Ile Leu Pro Arg
            20                  25                  30

Gln Asp Thr Ala Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 61
<211> 63
<212> PRT
<213> Artificial sequence

<220>
<223> Fynomer #61

<400> 61

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Gln Ala
20 25 30

Leu Gly Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 62
<211> 65
<212> PRT
<213> Artificial sequence

<220>
<223> Fynomer #62

<400> 62

Gly Val Thr Leu Phe Val Ala Leu Tyr Asp Tyr Thr Ser Tyr Asn Glu
1 5 10 15

Phe Asp Leu Ser Phe His Lys Gly Glu Lys Phe Gln Ile Leu Gln Gly
20 25 30

Thr Gln Leu Ala Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 63
<211> 63
<212> PRT
<213> Artificial sequence

<220>
<223> Fynomer #63

<400> 63

Gly Val Thr Leu Phe Val Ala Leu Tyr Asp Tyr Thr Ser Tyr Asn Asp
1 5 10 15

Arg Asp Leu Ser Phe His Lys Gly Glu Lys Phe Gln Ile Leu Gln His
20 25 30

Lys Glu Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 64
<211> 63
<212> PRT
<213> Artificial sequence

<220>
<223> Fynomer #64

<400> 64

Gly Val Thr Leu Phe Val Ala Leu Tyr Asp Tyr Tyr Ser Tyr Asn Asn
1 5 10 15

Thr Asp Leu Ser Phe His Lys Gly Glu Lys Phe Gln Ile Leu Gln Lys
20 25 30

Arg Gln Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 65
<211> 63
<212> PRT
<213> Artificial sequence

<220>
<223> Fynomer #65

<400> 65

Gly Val Thr Leu Phe Val Ala Leu Tyr Asp Tyr Thr Ser Tyr Asn Ser
1 5 10 15

Arg Asp Leu Ser Phe His Lys Gly Glu Lys Phe Gln Ile Leu Gln Lys
20 25 30

Ser Gln Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 66
<211> 65
<212> PRT
<213> Artificial sequence

<220>
<223> Fynomer #66

<400> 66

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Gln Pro
20 25 30

Asn Ser Ala Gln Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 67
<211> 63
<212> PRT
<213> Artificial sequence

<220>
<223> Fynomer #67

<400> 67

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Gln Pro
20 25 30

Gln Val Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 68
<211> 63
<212> PRT
<213> Artificial sequence

<220>
<223> Fynomer #68

<400> 68

Gly Val Thr Leu Phe Val Ala Leu Tyr Asp Tyr Tyr Ser Tyr Asn Lys
1 5 10 15

Thr Asp Leu Ser Phe His Lys Gly Glu Lys Phe Gln Ile Leu Gln Gln
20 25 30

His Glu Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 69
<211> 64
<212> PRT
<213> Artificial sequence

<220>
<223> Fynomer #69

<400> 69

Gly Val Thr Leu Phe Val Ala Leu Tyr Asp Tyr Thr Ser Tyr Asn Leu
1 5 10 15

Arg Asp Leu Ser Phe His Lys Gly Glu Lys Phe Gln Ile Leu Gln Gln
20 25 30

Asn Leu Asp Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 70
<211> 63
<212> PRT
<213> Artificial sequence

<220>
<223> Fynomer #70

<400> 70

Gly Val Thr Leu Phe Val Ala Leu Tyr Asp Tyr Thr Ser Tyr Asn Leu
1 5 10 15

Arg Asp Leu Ser Phe His Lys Gly Glu Lys Phe Gln Ile Leu Gln Ser
20 25 30

His Gln Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 71
<211> 64
<212> PRT
<213> Artificial sequence

<220>
<223> Fynomer #71

<400> 71

Gly Val Thr Leu Phe Val Ala Leu Tyr Asp Tyr Thr Ser Tyr Asn Ser
1 5 10 15

Arg Asp Leu Ser Phe His Lys Gly Glu Lys Phe Gln Ile Leu Gln Ser
20 25 30

Arg Ala Ser Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 72
<211> 64
<212> PRT
<213> Artificial sequence

<220>
<223> Fynomer #72

<400> 72

Gly Val Thr Leu Phe Val Ala Leu Tyr Asp Tyr Thr Ser Tyr Asn Ala
1 5 10 15

Arg Asp Leu Ser Phe His Lys Gly Glu Lys Phe Gln Ile Leu Gln Thr
20 25 30

Ser Leu Thr Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 73
<211> 64
<212> PRT
<213> Artificial sequence

<220>
<223> Fynomer #73

<400> 73

Gly Val Thr Leu Phe Val Ala Leu Tyr Asp Tyr Thr Ser Tyr Asn Ser
1 5 10 15

Arg Asp Leu Ser Phe His Lys Gly Glu Lys Phe Gln Ile Leu Gln Thr
20 25 30

Thr Ala Met Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 74
<211> 65
<212> PRT
<213> Artificial sequence

<220>
<223> Fynomer #74

<400> 74

Gly Val Thr Leu Phe Val Ala Leu Tyr Asp Tyr Thr Ser Tyr Asn Arg
1 5 10 15

Arg Asp Leu Ser Phe His Lys Gly Glu Lys Phe Gln Ile Leu Gln Val
20 25 30

Asn Pro Met Asp Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 75
<211> 64
<212> PRT
<213> Artificial sequence

<220>
<223> Fynomer #75

<400> 75

```
Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Arg Gln
            20                  25                  30

Gln Arg Gly Gly Asp Trp Trp Glu Ala Arg Ser Leu Thr Thr Gly Glu
        35                  40                  45

Thr Gly Tyr Ile Pro Ser Asn Tyr Val Ala Pro Val Asp Ser Ile Gln
    50                  55                  60
```

<210> 76
<211> 65
<212> PRT
<213> Artificial sequence

<220>
<223> Fynomer #76

<400> 76

```
Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Arg Val
            20                  25                  30

Pro Gln Asp Asp Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65
```

<210> 77
<211> 64
<212> PRT
<213> Artificial sequence

<220>
<223> Fynomer #77

<400> 77

```
Gly Val Thr Leu Phe Val Ala Leu Tyr Asp Tyr Thr Ser Tyr Asn Pro
1               5                   10                  15

Asn Asp Leu Ser Phe His Lys Gly Glu Lys Phe Gln Ile Leu Ser Gln
            20                  25                  30

Gln Asp Gly Gly Asp Trp Trp Glu Ala Arg Ser Leu Thr Thr Gly Glu
        35                  40                  45

Thr Gly Tyr Ile Pro Ser Asn Tyr Val Ala Pro Val Asp Ser Ile Gln
    50                  55                  60


<210>  78
<211>  65
<212>  PRT
<213>  Artificial sequence

<220>
<223>  Fynomer #78

<400>  78

Gly Val Thr Leu Phe Val Ala Leu Tyr Asp Tyr Thr Ser Tyr Asn Arg
1               5                   10                  15

Arg Asp Leu Ser Phe His Lys Gly Glu Lys Phe Gln Ile Leu Ser Ser
            20                  25                  30

Asn Arg Ala Gln Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65


<210>  79
<211>  65
<212>  PRT
<213>  Artificial sequence

<220>
<223>  Fynomer #79

<400>  79

Gly Val Thr Leu Phe Val Ala Leu Tyr Asp Tyr Thr Ser Tyr Asn Asn
1               5                   10                  15

Arg Asp Leu Ser Phe His Lys Gly Glu Lys Phe Gln Ile Leu Thr Pro
            20                  25                  30
```

Asp Ser Arg Gln Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 80
<211> 64
<212> PRT
<213> Artificial sequence

<220>
<223> Fynomer #80

<400> 80

Gly Val Thr Leu Phe Val Ala Leu Tyr Asp Tyr Thr Ser Tyr Asn Ser
1 5 10 15

Arg Asp Leu Ser Phe His Lys Gly Glu Lys Phe Gln Ile Leu Thr Pro
20 25 30

Pro Gln His Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 81
<211> 65
<212> PRT
<213> Artificial sequence

<220>
<223> Fynomer #081

<400> 81

Gly Val Thr Leu Phe Val Ala Leu Tyr Asp Tyr Thr Ser Tyr Asn Asn
1 5 10 15

Thr Asp Leu Ser Phe His Lys Gly Glu Lys Phe Gln Ile Leu Thr Gln
20 25 30

Asp Pro Leu His Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln

65

<210> 82
<211> 63
<212> PRT
<213> Artificial sequence

<220>
<223> Fynomer #82

<400> 82

```
Gly Val Thr Leu Phe Val Ala Leu Tyr Asp Tyr Thr Ser Tyr Asn Asp
1               5                   10                  15

Arg Asp Leu Ser Phe His Lys Gly Glu Lys Phe Gln Ile Leu Thr Gln
            20                  25                  30

Lys Gln Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60
```

<210> 83
<211> 65
<212> PRT
<213> Artificial sequence

<220>
<223> Fynomer #83

<400> 83

```
Gly Val Thr Leu Phe Val Ala Leu Tyr Asp Tyr Ser Ser Tyr Asn Thr
1               5                   10                  15

Thr Asp Leu Ser Phe His Lys Gly Glu Lys Phe Gln Ile Leu Thr Gln
            20                  25                  30

Pro Pro Leu Ser Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65
```

<210> 84
<211> 65
<212> PRT
<213> Artificial sequence

<220>

<223> Fynomer #84

<400> 84

Gly Val Thr Leu Phe Val Ala Leu Tyr Asp Tyr Thr Ser Tyr Asn Thr
1 5 10 15

Gln Asp Leu Ser Phe His Lys Gly Glu Lys Phe Gln Ile Leu Asp Ser
20 25 30

Glu Thr Gly Lys Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 85
<211> 65
<212> PRT
<213> Artificial sequence

<220>
<223> Fynomer #85

<400> 85

Gly Val Thr Leu Phe Val Ala Leu Tyr Asp Tyr Thr Ser Tyr Asn Gln
1 5 10 15

Arg Asp Leu Ser Phe His Lys Gly Glu Lys Phe Gln Ile Leu Arg Lys
20 25 30

Pro Lys Tyr Asn Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 86
<211> 63
<212> PRT
<213> Artificial sequence

<220>
<223> Fynomer #86

<400> 86

Gly Val Thr Leu Phe Val Ala Leu Tyr Asp Tyr Thr Ser Tyr Asn Gln

1 5 10 15

Arg Asp Leu Ser Phe His Lys Gly Glu Lys Phe Gln Ile Leu Ser Glu
20 25 30

Pro Leu Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 87
<211> 64
<212> PRT
<213> Artificial sequence

<220>
<223> Fynomer #87

<400> 87

Gly Val Thr Leu Phe Val Ala Leu Tyr Asp Tyr Thr Ser Tyr Asn Gln
1 5 10 15

Arg Asp Leu Ser Phe His Lys Gly Glu Lys Phe Gln Ile Leu Val His
20 25 30

Asp Pro Ala Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 88
<211> 62
<212> PRT
<213> Artificial sequence

<220>
<223> Fynomer #88

<400> 88

Gly Val Thr Leu Phe Val Ala Leu Tyr Asp Tyr Val Ser Trp Asn Thr
1 5 10 15

Leu Asp Leu Ser Phe His Lys Gly Glu Lys Phe Gln Ile Leu Arg Met
20 25 30

Pro Gly Asp Trp Trp Glu Ala Arg Ser Leu Thr Thr Gly Glu Thr Gly
35 40 45

Tyr Ile Pro Ser Asn Tyr Val Ala Pro Val Asp Ser Ile Gln
50 55 60

<210> 89
<211> 64
<212> PRT
<213> Artificial sequence

<220>
<223> Fynomer #89

<400> 89

Gly Val Thr Leu Phe Val Ala Leu Tyr Asp Tyr Glu Ala Arg Leu Tyr
1 5 10 15

Ser Leu Asp Leu Ser Phe His Lys Gly Glu Lys Phe Gln Ile Leu Asn
20 25 30

Arg Arg Trp Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 90
<211> 64
<212> PRT
<213> Artificial sequence

<220>
<223> Fynomer #90

<400> 90

Gly Val Thr Leu Phe Val Ala Leu Tyr Asp Tyr Thr Ser Tyr Asn Ser
1 5 10 15

Arg Asp Leu Ser Phe His Lys Gly Glu Lys Phe Gln Ile Leu Ala Pro
20 25 30

Pro Asn Gly Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 91
<211> 64
<212> PRT
<213> Artificial sequence

<220>
<223> Fynomer #91

<400> 91

Gly Val Thr Leu Phe Val Ala Leu Tyr Asp Tyr Thr Ser Tyr Asn Ser

1 5 10 15

Arg Asp Leu Ser Phe His Lys Gly Glu Lys Phe Gln Ile Leu Ala Arg
20 25 30

Met Pro Pro Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 92
<211> 66
<212> PRT
<213> Artificial sequence

<220>
<223> Fynomer #92

<400> 92

Gly Val Thr Leu Phe Val Ala Leu Tyr Asp Tyr Glu Ala Arg Phe Arg
1 5 10 15

Arg Asn Tyr Ser Asp Leu Ser Phe His Lys Gly Glu Lys Phe Gln Ile
20 25 30

Leu Ser Ala Gln Gln Gly Asp Trp Trp Glu Ala Arg Ser Leu Thr Thr
35 40 45

Gly Glu Thr Gly Tyr Ile Pro Ser Asn Tyr Val Ala Pro Val Asp Ser
50 55 60

Ile Gln
65

<210> 93
<211> 65
<212> PRT
<213> Artificial sequence

<220>
<223> Fynomer #93

<400> 93

Gly Val Thr Leu Phe Val Ala Leu Tyr Asp Tyr Asp Arg Arg Tyr Gly
1 5 10 15

Ala Asp Leu Ser Phe His Lys Gly Glu Lys Phe Gln Ile Leu Arg Asp
20 25 30

Glu Ala Val Ser Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 94
<211> 65
<212> PRT
<213> Artificial sequence

<220>
<223> Fynomer #94

<400> 94

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu His Asp
20 25 30

Pro Pro Ser Asn Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 95
<211> 64
<212> PRT
<213> Artificial sequence

<220>
<223> Fynomer #95

<400> 95

Gly Val Thr Leu Phe Val Ala Leu Tyr Asp Tyr Glu Ala Arg Tyr Ala
1 5 10 15

Pro Ala Asp Leu Ser Phe His Lys Gly Glu Lys Phe Gln Ile Leu Asn
20 25 30

His Asp Arg Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 96
<211> 65
<212> PRT
<213> Artificial sequence

<220>
<223> Fynomer #96

<400> 96

Gly Val Thr Leu Phe Val Ala Leu Tyr Asp Tyr Thr Ser Ser Tyr Pro
1 5 10 15

Gly Asp Leu Ser Phe His Lys Gly Glu Lys Phe Gln Ile Leu Ser Asn
20 25 30

Asp Pro Val His Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 97
<211> 65
<212> PRT
<213> Artificial sequence

<220>
<223> Fynomer #97

<400> 97

Gly Val Thr Leu Phe Val Ala Leu Tyr Asp Tyr Gln Thr Trp Thr Pro
1 5 10 15

Gly Asp Leu Ser Phe His Lys Gly Glu Lys Phe Gln Ile Leu Thr Gln
20 25 30

Asp Glu Gln Gln Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 98
<211> 65
<212> PRT
<213> Artificial sequence

<220>
<223> Fynomer #98

<400> 98

Gly Val Thr Leu Phe Val Ala Leu Tyr Asp Tyr Phe Thr Asn Thr Pro
1 5 10 15

Gly Asp Leu Ser Phe His Lys Gly Glu Lys Phe Gln Ile Leu Asn Thr
20 25 30

Ser Tyr Leu Thr Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 99
<211> 63
<212> PRT
<213> Artificial sequence

<220>
<223> Fynomer #99

<400> 99

Gly Val Thr Leu Phe Val Ala Leu Tyr Asp Tyr Lys Thr His Asn Pro
1 5 10 15

Gly Asp Leu Ser Phe His Lys Gly Glu Lys Phe Gln Ile Leu Gln Gly
20 25 30

Arg Val Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 100
<211> 65
<212> PRT
<213> Artificial sequence

<220>
<223> Fynomer #100

<400> 100

Gly Val Thr Leu Phe Val Ala Leu Tyr Asp Tyr Gln Thr Tyr Thr Pro
1 5 10 15

```
Gly Asp Leu Ser Phe His Lys Gly Glu Lys Phe Gln Ile Leu Ser Lys
            20                  25                  30

Pro Pro Gln Thr Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65
```

<210>  101
<211>  65
<212>  PRT
<213>  Artificial sequence

<220>
<223>  Fynomer #101

<400>  101

```
Gly Val Thr Leu Phe Val Ala Leu Tyr Asp Tyr Glu Ala Arg Tyr Gln
1               5                   10                  15

Asp Leu Glu Asp Leu Ser Phe His Lys Gly Glu Lys Phe Gln Ile Leu
            20                  25                  30

Asn Gly Arg Arg Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65
```

<210>  102
<211>  63
<212>  PRT
<213>  Artificial sequence

<220>
<223>  Fynomer #102

<400>  102

```
Gly Val Thr Leu Phe Val Ala Leu Tyr Asp Tyr Asp Arg His Tyr Thr
1               5                   10                  15

Phe Asp Leu Ser Phe His Lys Gly Glu Lys Phe Gln Ile Leu Gln Asn
            20                  25                  30
```

Lys Gln Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 103
<211> 63
<212> PRT
<213> Artificial sequence

<220>
<223> Fynomer #103

<400> 103

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Ser Asp
20 25 30

Ser Asp Gly Val Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 104
<211> 65
<212> PRT
<213> Artificial sequence

<220>
<223> Fynomer #104

<400> 104

Gly Val Thr Leu Phe Val Ala Leu Tyr Asp Tyr Glu Ala Arg Asp Arg
1 5 10 15

Pro Thr Asp Leu Ser Phe His Lys Gly Glu Lys Phe Gln Ile Leu Gly
20 25 30

Asp Glu Gln Ile Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 105

<211> 64
<212> PRT
<213> Artificial sequence

<220>
<223> Fynomer #105

<400> 105

Gly Val Thr Leu Phe Val Ala Leu Tyr Asp Tyr Thr Thr Tyr Arg Lys
1 5 10 15

Gly Asp Leu Ser Phe His Lys Gly Glu Lys Phe Gln Ile Leu Asn Arg
20 25 30

Val Ser Ile Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 106
<211> 64
<212> PRT
<213> Artificial sequence

<220>
<223> Fynomer #106

<400> 106

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Asn Arg
20 25 30

Val Ser Ile Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 107
<211> 63
<212> PRT
<213> Artificial sequence

<220>
<223> Fynomer #107

<400> 107

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Gly Asp
20 25 30

Asn Ile Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 108
<211> 63
<212> PRT
<213> Artificial sequence

<220>
<223> Fynomer #108

<400> 108

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Asn Ser
20 25 30

Ser Asp Gly Thr Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 109
<211> 64
<212> PRT
<213> Artificial sequence

<220>
<223> Fynomer #109

<400> 109

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Trp Ser
20 25 30

Asp Ala Leu Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 110

<211> 63
<212> PRT
<213> Artificial sequence

<220>
<223> Fynomer #110

<400> 110

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Met Ala
20 25 30

Trp Ala Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 111
<211> 65
<212> PRT
<213> Artificial sequence

<220>
<223> Fynomer #111

<400> 111

Gly Val Thr Leu Phe Val Ala Leu Tyr Asp Tyr Glu Ala Arg Glu Gly
1 5 10 15

Gly Asn Asp Leu Ser Phe His Lys Gly Glu Lys Phe Gln Ile Leu Asn
20 25 30

Arg Val Ser Ile Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 112
<211> 66
<212> PRT
<213> Artificial sequence

<220>
<223> Fynomer #112

<400> 112

Gly Val Thr Leu Phe Val Ala Leu Tyr Asp Tyr Glu Ala His Asp Gln
1               5                   10                  15

His Arg Pro Asp Leu Ser Phe His Lys Gly Glu Lys Phe Gln Ile Leu
            20                  25                  30

Asn Arg Val Ser Ile Gly Asp Trp Trp Glu Ala Arg Ser Leu Thr Thr
        35                  40                  45

Gly Glu Thr Gly Tyr Ile Pro Ser Asn Tyr Val Ala Pro Val Asp Ser
    50                  55                  60

Ile Gln
65

<210> 113
<211> 66
<212> PRT
<213> Artificial sequence

<220>
<223> Fynomer #113

<400> 113

Gly Val Thr Leu Phe Val Ala Leu Tyr Asp Tyr Glu Ala Arg Leu Ser
1               5                   10                  15

Ser His Pro His Val Leu Ser Phe His Lys Gly Glu Lys Phe Gln Ile
            20                  25                  30

Leu Asn Ser Ser Ala Gly Asp Trp Trp Glu Ala Arg Ser Leu Thr Thr
        35                  40                  45

Gly Glu Thr Gly Tyr Ile Pro Ser Asn Tyr Val Ala Pro Val Asp Ser
    50                  55                  60

Ile Gln
65

<210> 114
<211> 64
<212> PRT
<213> Artificial sequence

<220>
<223> Fynomer #114

<400> 114

Gly Val Thr Leu Phe Val Ala Leu Tyr Asp Tyr Thr Ser Tyr Asn Ser
1               5                   10                  15

Arg Asp Leu Ser Phe His Lys Gly Glu Lys Phe Gln Ile Leu Leu Met

20 25 30

His Pro Gly Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 115
<211> 64
<212> PRT
<213> Artificial sequence

<220>
<223> Fynomer #115

<400> 115

Gly Val Thr Leu Phe Val Ala Leu Tyr Asp Tyr Glu Ala Arg His Ala
1 5 10 15

Pro Val Asp Leu Ser Phe His Lys Gly Glu Lys Phe Gln Ile Leu Gly
20 25 30

Asp Asn Ile Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 116
<211> 64
<212> PRT
<213> Artificial sequence

<220>
<223> Fynomer #116

<400> 116

Gly Val Thr Leu Phe Val Ala Leu Tyr Asp Tyr Glu Ala Arg Thr Lys
1 5 10 15

His Phe Asp Leu Ser Phe His Lys Gly Glu Lys Phe Gln Ile Leu Ser
20 25 30

Gln Pro His Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 117
<211> 64

<212> PRT
<213> Artificial sequence

<220>
<223> Fynomer #117

<400> 117

Gly Val Thr Leu Phe Val Ala Leu Tyr Asp Tyr Glu Ala Arg His Glu
1 5 10 15

Asn Phe Asp Leu Ser Phe His Lys Gly Glu Lys Phe Gln Ile Leu Asn
20 25 30

Arg Gly Ala Gly Asp Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 118
<211> 65
<212> PRT
<213> Artificial sequence

<220>
<223> Fynomer #118

<400> 118

Gly Val Thr Leu Phe Val Ala Leu Tyr Asp Tyr Glu Ala Arg Pro Asp
1 5 10 15

Ser His Asp Leu Ser Phe His Lys Gly Glu Lys Phe Gln Ile Leu Asn
20 25 30

Arg Val Ser Ile Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 119
<211> 63
<212> PRT
<213> Artificial sequence

<220>
<223> Fynomer #119

<400> 119

Gly Val Thr Leu Phe Val Ala Leu Tyr Asp Tyr Glu Ala Arg Arg Thr

1 5 10 15

His Asp Leu Ser Phe His Lys Gly Glu Lys Phe Gln Ile Leu Ser Gln
20 25 30

Pro His Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 120
<211> 65
<212> PRT
<213> Artificial sequence

<220>
<223> Fynomer #120

<400> 120

Gly Val Thr Leu Phe Val Ala Leu Tyr Asp Tyr Glu Ala Arg Pro Met
1 5 10 15

Ser Ser Asp Leu Ser Phe His Lys Gly Glu Lys Phe Gln Ile Leu Asn
20 25 30

Arg Val Ser Ile Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 121
<211> 63
<212> PRT
<213> Artificial sequence

<220>
<223> Fynomer #121

<400> 121

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Arg Phe
20 25 30

Asn Pro Gly Asp Trp Trp Glu Ala Arg Ser Leu Thr Thr Gly Glu Thr
35 40 45

Gly Tyr Ile Pro Ser Ser Tyr Val Ala Pro Val Asp Ser Ile Gln
    50              55              60

<210> 122
<211> 63
<212> PRT
<213> Artificial sequence

<220>
<223> Fynomer #122

<400> 122

Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Phe Pro
            20                  25                  30

Asp Asp Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210> 123
<211> 63
<212> PRT
<213> Artificial sequence

<220>
<223> Fynomer #123

<400> 123

Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Ser Gln
            20                  25                  30

Pro His Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210> 124
<211> 63
<212> PRT
<213> Artificial sequence

<220>
<223> Fynomer #124

<400> 124

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Gly Lys
20 25 30

Gly Ser Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 125
<211> 63
<212> PRT
<213> Artificial sequence

<220>
<223> Fynomer #125

<400> 125

Gly Val Thr Leu Phe Val Ala Leu Tyr Asp Tyr Thr Ser Tyr Asn Ser
1 5 10 15

Arg Asp Leu Ser Phe His Lys Gly Glu Lys Phe Gln Ile Leu Ser Asp
20 25 30

Gln His Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 126
<211> 63
<212> PRT
<213> Artificial sequence

<220>
<223> Fynomer #126

<400> 126

Gly Val Thr Leu Phe Val Ala Leu Tyr Asp Tyr Thr Ser Tyr Asn Asp
1 5 10 15

Arg Asp Leu Ser Phe His Lys Gly Glu Lys Phe Gln Ile Leu Ser Ala
20 25 30

Pro Gln Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 127
<211> 63
<212> PRT
<213> Artificial sequence

<220>
<223> Fynomer #127

<400> 127

Gly Val Thr Leu Phe Val Ala Leu Tyr Asp Tyr Thr Ser Tyr Asn Asp
1 5 10 15

Arg Asp Leu Ser Phe His Lys Gly Glu Lys Phe Gln Ile Leu Glu Tyr
20 25 30

Thr Thr Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 128
<211> 65
<212> PRT
<213> Artificial sequence

<220>
<223> Fynomer #128

<400> 128

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Thr Thr
20 25 30

Glu Ala Thr Asp Gly Asp Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 129
<211> 63
<212> PRT

<213> Artificial sequence

<220>
<223> Fynomer #129

<400> 129

Gly Val Thr Leu Phe Val Ala Leu Tyr Asp Tyr Thr Ser Tyr Asn Ser
1 5 10 15

Arg Asp Leu Ser Phe His Lys Gly Glu Lys Phe Gln Ile Leu Gln Asn
20 25 30

Ser Gln Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 130
<211> 63
<212> PRT
<213> Artificial sequence

<220>
<223> Fynomer #130

<400> 130

Gly Val Thr Leu Phe Val Ala Leu Tyr Asp Tyr Thr Ser Tyr Asn Ser
1 5 10 15

Arg Asp Leu Ser Phe His Lys Gly Glu Lys Phe Gln Ile Leu Gly Asn
20 25 30

Thr Gln Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 131
<211> 63
<212> PRT
<213> Artificial sequence

<220>
<223> Fynomer #131

<400> 131

Gly Val Thr Leu Phe Val Ala Leu Tyr Asp Tyr Thr Ser Tyr Asn Ala
1 5 10 15

Gln Asp Leu Ser Phe His Lys Gly Glu Lys Phe Gln Ile Leu Gly Ala
20 25 30

Arg Tyr Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 132
<211> 63
<212> PRT
<213> Artificial sequence

<220>
<223> Fynomer #132

<400> 132

Gly Val Thr Leu Phe Val Ala Leu Tyr Asp Tyr Thr Ser Tyr Asn Ala
1 5 10 15

Arg Asp Leu Ser Phe His Lys Gly Glu Lys Phe Gln Ile Leu Gly His
20 25 30

His Ser Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 133
<211> 63
<212> PRT
<213> Artificial sequence

<220>
<223> Fynomer #133

<400> 133

Gly Val Thr Leu Phe Val Ala Leu Tyr Asp Tyr Thr Ser Tyr Asn Met
1 5 10 15

Arg Asp Leu Ser Phe His Lys Gly Glu Lys Phe Gln Ile Leu Asn Asn
20 25 30

Ser Asp Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 134
<211> 65
<212> PRT

<213> Artificial sequence

<220>
<223> Fynomer #134

<400> 134

```
Gly Val Thr Leu Phe Val Ala Leu Tyr Asp Tyr Thr Ser Tyr Asn Asp
1               5                   10                  15

Arg Asp Leu Ser Phe His Lys Gly Glu Lys Phe Gln Ile Leu Ser Lys
            20                  25                  30

Asp Ser Ala Leu Gly Asp Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65
```

<210> 135
<211> 63
<212> PRT
<213> Artificial sequence

<220>
<223> Fynomer #135

<400> 135

```
Gly Val Thr Leu Phe Val Ala Leu Tyr Asp Tyr Thr Ser Tyr Asn Ser
1               5                   10                  15

Arg Asp Leu Ser Phe His Lys Gly Glu Lys Phe Gln Ile Leu Asn Arg
            20                  25                  30

Gly Ala Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60
```

<210> 136
<211> 63
<212> PRT
<213> Artificial sequence

<220>
<223> Fynomer #136

<400> 136

```
Gly Val Thr Leu Phe Val Ala Leu Tyr Asp Tyr Thr Ser Tyr Asn Thr
1               5                   10                  15
```

His Asp Leu Ser Phe His Lys Gly Glu Lys Phe Gln Ile Leu Lys Met
20 25 30

Gln Ser Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 137
<211> 63
<212> PRT
<213> Artificial sequence

<220>
<223> Fynomer #137

<400> 137

Gly Val Thr Leu Phe Val Ala Leu Tyr Asp Tyr Thr Ser Tyr Asn Arg
1 5 10 15

Arg Asp Leu Ser Phe His Lys Gly Glu Lys Phe Gln Ile Leu Glu Thr
20 25 30

Gln Asn Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 138
<211> 63
<212> PRT
<213> Artificial sequence

<220>
<223> Fynomer #138

<400> 138

Gly Val Thr Leu Phe Val Ala Leu Tyr Asp Tyr Thr Ser Tyr Asn Ala
1 5 10 15

Arg Asp Leu Ser Phe His Lys Gly Glu Lys Phe Gln Ile Leu Gly Ser
20 25 30

Thr Ala Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 139
<211> 67
<212> PRT
<213> Artificial sequence

<220>
<223> Fynomer #139

<400> 139

Gly Val Thr Leu Phe Val Ala Leu Tyr Asp Tyr Glu Ala Arg Asp Ser
1 5 10 15

Met Gly Gly Gln Asp Leu Ser Phe His Lys Gly Glu Lys Phe Gln Ile
20 25 30

Leu Arg Ser Trp Pro Glu Gly Asp Trp Trp Glu Ala Arg Ser Leu Thr
35 40 45

Thr Gly Glu Thr Gly Tyr Ile Pro Ser Asn Tyr Val Ala Pro Val Asp
50 55 60

Ser Ile Gln
65

<210> 140
<211> 67
<212> PRT
<213> Artificial sequence

<220>
<223> Fynomer #140

<400> 140

Gly Val Thr Leu Phe Val Ala Leu Tyr Asp Tyr Glu Ala Arg Asp Ser
1 5 10 15

Met Gly Gly Gln Asp Leu Ser Phe His Lys Gly Glu Lys Phe Gln Ile
20 25 30

Leu Lys Thr Trp Glu Gly Gly Asp Trp Trp Glu Ala Arg Ser Leu Thr
35 40 45

Thr Gly Glu Thr Gly Tyr Ile Pro Ser Asn Tyr Val Ala Pro Val Asp
50 55 60

Ser Ile Gln
65

<210> 141
<211> 67
<212> PRT
<213> Artificial sequence

<220>
<223> Fynomer #141

<400> 141

Gly Val Thr Leu Phe Val Ala Leu Tyr Asp Tyr Glu Ala Arg Asp Ser
1               5                   10                  15

Met Gly Gly Gln Asp Leu Ser Phe His Lys Gly Glu Lys Phe Gln Ile
            20                  25                  30

Leu Gln Ala Trp Gln Glu Gly Asp Trp Trp Glu Ala Arg Ser Leu Thr
        35                  40                  45

Thr Gly Glu Thr Gly Tyr Ile Pro Ser Asn Tyr Val Ala Pro Val Asp
    50                  55                  60

Ser Ile Gln
65

<210> 142
<211> 63
<212> PRT
<213> Artificial sequence

<220>
<223> Fynomer #142

<400> 142

Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Arg Met
            20                  25                  30

Glu Asp Gly Val Trp Trp Ala Ala Arg Ser Leu Thr Thr Gly Glu Ile
        35                  40                  45

Gly Tyr Ile Pro Ser Asn Tyr Val Ala Pro Val Asp Ser Ile Gln
    50                  55                  60

<210> 143
<211> 63
<212> PRT
<213> Artificial sequence

<220>
<223> Fynomer #143

<400> 143

Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Arg Met
20 25 30

Glu Asp Gly Val Trp Trp Arg Ala Arg Ser Leu Thr Thr Gly Glu Ile
35 40 45

Gly Leu Ile Pro Ser Asn Tyr Val Ala Pro Val Asp Ser Ile Gln
50 55 60

<210> 144
<211> 63
<212> PRT
<213> Artificial sequence

<220>
<223> Fynomer #144

<400> 144

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Arg Met
20 25 30

Glu Asp Gly Val Trp Trp Thr Ala Arg Ser Leu Thr Thr Gly Glu Val
35 40 45

Gly Phe Ile Pro Ser Asn Tyr Val Ala Pro Val Asp Ser Ile Gln
50 55 60

<210> 145
<211> 63
<212> PRT
<213> Artificial sequence

<220>
<223> Fynomer #145

<400> 145

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Arg Met
20 25 30

Glu Asp Gly Ile Trp Trp Gln Ala Arg Ser Leu Thr Thr Gly Glu Thr
35 40 45

Gly Phe Ile Pro Ser Asn Tyr Val Ala Pro Val Asp Ser Ile Gln
50 55 60

<210> 146
<211> 65
<212> PRT
<213> Artificial sequence

<220>
<223> Fynomer #146

<400> 146

Gly Val Thr Leu Phe Val Ala Leu Tyr Asp Tyr Gln Ala Tyr Gly Met
1 5 10 15

Tyr Asp Leu Ser Phe His Lys Gly Glu Lys Phe Gln Ile Leu Pro Pro
20 25 30

Tyr Pro Thr Gly Gly Tyr Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 147
<211> 64
<212> PRT
<213> Artificial sequence

<220>
<223> Fynomer #147

<400> 147

Gly Val Thr Leu Phe Val Ala Leu Tyr Asp Tyr Gln Ala Tyr Gly Met
1 5 10 15

Tyr Asp Leu Ser Phe His Lys Gly Glu Lys Phe Gln Ile Leu Gln Val
20 25 30

Leu Asp Asn Gly Tyr Trp Trp Glu Ala Arg Ser Leu Thr Thr Gly Glu
35 40 45

Thr Gly Tyr Ile Pro Ser Asn Tyr Val Ala Pro Val Asp Ser Ile Gln
50 55 60

<210> 148
<211> 65
<212> PRT
<213> Artificial sequence

<220>
<223> Fynomer #148

<400> 148

Gly Val Thr Leu Phe Val Ala Leu Tyr Asp Tyr Gln Ala Tyr Gly Met
1               5                   10                  15

Tyr Asp Leu Ser Phe His Lys Gly Glu Lys Phe Gln Ile Leu Thr Ala
            20                  25                  30

Leu Pro Asp Arg Gly Tyr Trp Trp Glu Ala Arg Ser Leu Thr Thr Gly
        35                  40                  45

Glu Thr Gly Tyr Ile Pro Ser Asn Tyr Val Ala Pro Val Asp Ser Ile
    50                  55                  60

Gln
65

<210> 149
<211> 63
<212> PRT
<213> Artificial sequence

<220>
<223> Fynomer #149

<400> 149

Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Arg Asp
            20                  25                  30

Asp Asp Gly Val Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60

<210> 150
<211> 65
<212> PRT
<213> Artificial sequence

<220>
<223> Fynomer #150

<400> 150

Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Ser Phe
            20                  25                  30

Gln Ser Ala Gly Gly Val Trp Trp Glu Ala Arg Ser Leu Thr Thr Gly
35 40 45

Glu Thr Gly Tyr Ile Pro Ser Asn Tyr Val Ala Pro Val Asp Ser Ile
50 55 60

Gln
65

<210> 151
<211> 63
<212> PRT
<213> Artificial sequence

<220>
<223> Fynomer #151

<400> 151

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Ala Arg
20 25 30

Asp Asn Gly Val Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 152
<211> 66
<212> PRT
<213> Artificial sequence

<220>
<223> Fynomer #152

<400> 152

Gly Val Thr Leu Phe Val Ala Leu Tyr Asp Tyr Glu Ala Arg Asp Ser
1 5 10 15

Met Gly Gly Gln Asp Leu Ser Phe His Lys Gly Glu Lys Phe Gln Ile
20 25 30

Leu Trp Asn Thr Gly Gly Asp Trp Trp Glu Ala Arg Ser Leu Thr Thr
35 40 45

Gly Glu Thr Gly Tyr Ile Pro Ser Asn Tyr Val Ala Pro Val Asp Ser
50 55 60

Ile Gln

65

<210> 153
<211> 305
<212> PRT
<213> Artificial sequence

<220>
<223> Fc fusion of Fynomer C12 (Fynomer #1; SEQ ID NO:1)

<400> 153

```
Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Glu Glu Met Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205
```

His Glu Ala Leu His Asn His Tyr Thr Gln Lys Ser Leu Ser Leu Ser
    210                 215                 220

Pro Gly Lys Gly Gly Gly Gly Ser Gly Gly Gly Gly Ser Gly Gly Gly
225                 230                 235                 240

Gly Ser Gly Val Thr Leu Phe Val Ala Leu Tyr Asp Tyr Thr Ser Tyr
                245                 250                 255

Asn Thr Arg Asp Leu Ser Phe His Lys Gly Glu Lys Phe Gln Ile Leu
            260                 265                 270

Arg Met Glu Asp Gly Val Trp Trp Glu Ala Arg Ser Leu Thr Thr Gly
        275                 280                 285

Glu Thr Gly Tyr Ile Pro Ser Asn Tyr Val Ala Pro Val Asp Ser Ile
    290                 295                 300

Gln
305

<210> 154
<211> 449
<212> PRT
<213> Artificial sequence

<220>
<223> anti HER2 antibody 1, heavy chain

<400> 154

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Thr Asp Tyr
            20                  25                  30

Thr Met Asp Trp Val Arg Gln Ala Pro Gly Lys Gly Leu Glu Trp Val
        35                  40                  45

Ala Asp Val Asn Pro Asn Ser Gly Gly Ser Ile Tyr Asn Gln Arg Phe
    50                  55                  60

Lys Gly Arg Phe Thr Leu Ser Val Asp Arg Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asn Leu Gly Pro Ser Phe Tyr Phe Asp Tyr Trp Gly Gln Gly
            100                 105                 110

```
Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365
```

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 155
<211> 214
<212> PRT
<213> Artificial sequence

<220>
<223> anti HER2 antibody 1, light chain

<400> 155

Asp Ile Gln Met Thr Gln Ser Pro Ser Ser Leu Ser Ala Ser Val Gly
1 5 10 15

Asp Arg Val Thr Ile Thr Cys Lys Ala Ser Gln Asp Val Ser Ile Gly
20 25 30

Val Ala Trp Tyr Gln Gln Lys Pro Gly Lys Ala Pro Lys Leu Leu Ile
35 40 45

Tyr Ser Ala Ser Tyr Arg Tyr Thr Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Tyr Ile Tyr Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 156
<211> 527
<212> PRT
<213> Artificial sequence

<220>
<223> C-terminal heavy chain fusion protein of Fynomer C12 (Fynomer #1; SEQ ID NO:1) with anti HER2 antibody 1, heavy chain (SEQ ID NO:154)

<400> 156

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Thr Asp Tyr
            20                  25                  30

Thr Met Asp Trp Val Arg Gln Ala Pro Gly Lys Gly Leu Glu Trp Val
        35                  40                  45

Ala Asp Val Asn Pro Asn Ser Gly Gly Ser Ile Tyr Asn Gln Arg Phe
    50                  55                  60

Lys Gly Arg Phe Thr Leu Ser Val Asp Arg Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asn Leu Gly Pro Ser Phe Tyr Phe Asp Tyr Trp Gly Gln Gly
            100                 105                 110

```
Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
```

370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys Gly Gly Gly Gly Ser Gly Gly Gly Gly Ser Gly Gly Gly Gly Ser
450 455 460

Gly Val Thr Leu Phe Val Ala Leu Tyr Asp Tyr Thr Ser Tyr Asn Thr
465 470 475 480

Arg Asp Leu Ser Phe His Lys Gly Glu Lys Phe Gln Ile Leu Arg Met
485 490 495

Glu Asp Gly Val Trp Trp Glu Ala Arg Ser Leu Thr Thr Gly Glu Thr
500 505 510

Gly Tyr Ile Pro Ser Asn Tyr Val Ala Pro Val Asp Ser Ile Gln
515 520 525

<210> 157
<211> 292
<212> PRT
<213> Artificial sequence

<220>
<223> C-terminal light chain fusion protein of Fynomer C12 (Fynomer #1; SEQ ID NO:1) with anti HER2 antibody 1, light chain (SEQ ID NO:155)

<400> 157

Asp Ile Gln Met Thr Gln Ser Pro Ser Ser Leu Ser Ala Ser Val Gly
1 5 10 15

Asp Arg Val Thr Ile Thr Cys Lys Ala Ser Gln Asp Val Ser Ile Gly
20 25 30

Val Ala Trp Tyr Gln Gln Lys Pro Gly Lys Ala Pro Lys Leu Leu Ile
35 40 45

Tyr Ser Ala Ser Tyr Arg Tyr Thr Gly Val Pro Ser Arg Phe Ser Gly
50 55 60

```
Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Tyr Ile Tyr Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Gly Gly Gly Gly Ser Gly Gly Gly Gly Ser
    210                 215                 220

Gly Gly Gly Gly Ser Gly Val Thr Leu Phe Val Ala Leu Tyr Asp Tyr
225                 230                 235                 240

Thr Ser Tyr Asn Thr Arg Asp Leu Ser Phe His Lys Gly Glu Lys Phe
                245                 250                 255

Gln Ile Leu Arg Met Glu Asp Gly Val Trp Trp Glu Ala Arg Ser Leu
            260                 265                 270

Thr Thr Gly Glu Thr Gly Tyr Ile Pro Ser Asn Tyr Val Ala Pro Val
        275                 280                 285

Asp Ser Ile Gln
    290


<210>  158
<211>  527
<212>  PRT
```

<213> Artificial sequence

<220>
<223> N-terminal heavy chain fusion of Fynomer C12 (Fynomer #1; SEQ ID NO:1) with anti HER2 antibody 1, heavy chain (SEQ ID NO:154)

<400> 158

```
Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Arg Met
            20                  25                  30

Glu Asp Gly Val Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln Gly
    50                  55                  60

Gly Gly Gly Ser Gly Gly Gly Gly Ser Gly Gly Gly Gly Ser Glu Val
65                  70                  75                  80

Gln Leu Val Glu Ser Gly Gly Gly Leu Val Gln Pro Gly Gly Ser Leu
                85                  90                  95

Arg Leu Ser Cys Ala Ala Ser Gly Phe Thr Phe Thr Asp Tyr Thr Met
            100                 105                 110

Asp Trp Val Arg Gln Ala Pro Gly Lys Gly Leu Glu Trp Val Ala Asp
        115                 120                 125

Val Asn Pro Asn Ser Gly Gly Ser Ile Tyr Asn Gln Arg Phe Lys Gly
    130                 135                 140

Arg Phe Thr Leu Ser Val Asp Arg Ser Lys Asn Thr Leu Tyr Leu Gln
145                 150                 155                 160

Met Asn Ser Leu Arg Ala Glu Asp Thr Ala Val Tyr Tyr Cys Ala Arg
                165                 170                 175

Asn Leu Gly Pro Ser Phe Tyr Phe Asp Tyr Trp Gly Gln Gly Thr Leu
            180                 185                 190

Val Thr Val Ser Ser Ala Ser Thr Lys Gly Pro Ser Val Phe Pro Leu
        195                 200                 205

Ala Pro Ser Ser Lys Ser Thr Ser Gly Gly Thr Ala Ala Leu Gly Cys
    210                 215                 220

Leu Val Lys Asp Tyr Phe Pro Glu Pro Val Thr Val Ser Trp Asn Ser
```

```
225                 230                 235                 240

Gly Ala Leu Thr Ser Gly Val His Thr Phe Pro Ala Val Leu Gln Ser
                245                 250                 255

Ser Gly Leu Tyr Ser Leu Ser Ser Val Val Thr Val Pro Ser Ser Ser
            260                 265                 270

Leu Gly Thr Gln Thr Tyr Ile Cys Asn Val Asn His Lys Pro Ser Asn
        275                 280                 285

Thr Lys Val Asp Lys Lys Val Glu Pro Lys Ser Cys Asp Lys Thr His
    290                 295                 300

Thr Cys Pro Pro Cys Pro Ala Pro Glu Leu Leu Gly Gly Pro Ser Val
305                 310                 315                 320

Phe Leu Phe Pro Pro Lys Pro Lys Asp Thr Leu Met Ile Ser Arg Thr
                325                 330                 335

Pro Glu Val Thr Cys Val Val Val Asp Val Ser His Glu Asp Pro Glu
            340                 345                 350

Val Lys Phe Asn Trp Tyr Val Asp Gly Val Glu Val His Asn Ala Lys
        355                 360                 365

Thr Lys Pro Arg Glu Glu Gln Tyr Asn Ser Thr Tyr Arg Val Val Ser
    370                 375                 380

Val Leu Thr Val Leu His Gln Asp Trp Leu Asn Gly Lys Glu Tyr Lys
385                 390                 395                 400

Cys Lys Val Ser Asn Lys Ala Leu Pro Ala Pro Ile Glu Lys Thr Ile
                405                 410                 415

Ser Lys Ala Lys Gly Gln Pro Arg Glu Pro Gln Val Tyr Thr Leu Pro
            420                 425                 430

Pro Ser Arg Glu Glu Met Thr Lys Asn Gln Val Ser Leu Thr Cys Leu
        435                 440                 445

Val Lys Gly Phe Tyr Pro Ser Asp Ile Ala Val Glu Trp Glu Ser Asn
    450                 455                 460

Gly Gln Pro Glu Asn Asn Tyr Lys Thr Thr Pro Pro Val Leu Asp Ser
465                 470                 475                 480

Asp Gly Ser Phe Phe Leu Tyr Ser Lys Leu Thr Val Asp Lys Ser Arg
                485                 490                 495
```

Trp Gln Gln Gly Asn Val Phe Ser Cys Ser Val Met His Glu Ala Leu
500 505 510

His Asn His Tyr Thr Gln Lys Ser Leu Ser Leu Ser Pro Gly Lys
515 520 525

<210> 159
<211> 292
<212> PRT
<213> Artificial sequence

<220>
<223> N-terminal light chain fusion protein of Fynomer C12 (Fynomer #1; SEQ ID NO:1) with anti HER2 antibody 1, light chain (SEQ ID NO:155)

<400> 159

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Arg Met
20 25 30

Glu Asp Gly Val Trp Trp Glu Ala Arg Ser Leu Thr Thr Gly Glu Thr
35 40 45

Gly Tyr Ile Pro Ser Asn Tyr Val Ala Pro Val Asp Ser Ile Gln Gly
50 55 60

Gly Gly Gly Ser Gly Gly Gly Gly Ser Gly Gly Gly Gly Ser Asp Ile
65 70 75 80

Gln Met Thr Gln Ser Pro Ser Ser Leu Ser Ala Ser Val Gly Asp Arg
85 90 95

Val Thr Ile Thr Cys Lys Ala Ser Gln Asp Val Ser Ile Gly Val Ala
100 105 110

Trp Tyr Gln Gln Lys Pro Gly Lys Ala Pro Lys Leu Leu Ile Tyr Ser
115 120 125

Ala Ser Tyr Arg Tyr Thr Gly Val Pro Ser Arg Phe Ser Gly Ser Gly
130 135 140

Ser Gly Thr Asp Phe Thr Leu Thr Ile Ser Ser Leu Gln Pro Glu Asp
145 150 155 160

Phe Ala Thr Tyr Tyr Cys Gln Gln Tyr Tyr Ile Tyr Pro Tyr Thr Phe
165 170 175

Gly Gln Gly Thr Lys Val Glu Ile Lys Arg Thr Val Ala Ala Pro Ser
180 185 190

Val Phe Ile Phe Pro Pro Ser Asp Glu Gln Leu Lys Ser Gly Thr Ala
195 200 205

Ser Val Val Cys Leu Leu Asn Asn Phe Tyr Pro Arg Glu Ala Lys Val
210 215 220

Gln Trp Lys Val Asp Asn Ala Leu Gln Ser Gly Asn Ser Gln Glu Ser
225 230 235 240

Val Thr Glu Gln Asp Ser Lys Asp Ser Thr Tyr Ser Leu Ser Ser Thr
245 250 255

Leu Thr Leu Ser Lys Ala Asp Tyr Glu Lys His Lys Val Tyr Ala Cys
260 265 270

Glu Val Thr His Gln Gly Leu Ser Ser Pro Val Thr Lys Ser Phe Asn
275 280 285

Arg Gly Glu Cys
290

<210> 160
<211> 450
<212> PRT
<213> Artificial sequence

<220>
<223> anti HER2 antibody 2, heavy chain

<400> 160

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val
35 40 45

Ala Arg Ile Tyr Pro Thr Asn Gly Tyr Thr Arg Tyr Ala Asp Ser Val
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

```
Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350
```

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

<210> 161
<211> 292
<212> PRT
<213> Artificial sequence

<220>
<223> N-terminal light chain fusion protein of Fynomer C12 (Fynomer #1; SEQ ID NO:1) with anti HER2 antibody 2, light chain (SEQ ID NO:163)

<400> 161

Gly Val Thr Leu Phe Val Ala Leu Tyr Asp Tyr Thr Ser Tyr Asn Thr
1 5 10 15

Arg Asp Leu Ser Phe His Lys Gly Glu Lys Phe Gln Ile Leu Arg Met
20 25 30

Glu Asp Gly Val Trp Trp Glu Ala Arg Ser Leu Thr Thr Gly Glu Thr
35 40 45

Gly Tyr Ile Pro Ser Asn Tyr Val Ala Pro Val Asp Ser Ile Gln Gly
50 55 60

Gly Gly Gly Ser Gly Gly Gly Gly Ser Gly Gly Gly Gly Ser Asp Ile
65 70 75 80

Gln Met Thr Gln Ser Pro Ser Ser Leu Ser Ala Ser Val Gly Asp Arg
85 90 95

Val Thr Ile Thr Cys Arg Ala Ser Gln Asp Val Asn Thr Ala Val Ala
100 105 110

Trp Tyr Gln Gln Lys Pro Gly Lys Ala Pro Lys Leu Leu Ile Tyr Ser
115 120 125

Ala Ser Phe Leu Tyr Ser Gly Val Pro Ser Arg Phe Ser Gly Ser Arg
130 135 140

Ser Gly Thr Asp Phe Thr Leu Thr Ile Ser Ser Leu Gln Pro Glu Asp
145 150 155 160

Phe Ala Thr Tyr Tyr Cys Gln Gln His Tyr Thr Thr Pro Pro Thr Phe
165 170 175

Gly Gln Gly Thr Lys Val Glu Ile Lys Arg Thr Val Ala Ala Pro Ser
180 185 190

Val Phe Ile Phe Pro Pro Ser Asp Glu Gln Leu Lys Ser Gly Thr Ala
195 200 205

Ser Val Val Cys Leu Leu Asn Asn Phe Tyr Pro Arg Glu Ala Lys Val
210 215 220

Gln Trp Lys Val Asp Asn Ala Leu Gln Ser Gly Asn Ser Gln Glu Ser
225 230 235 240

Val Thr Glu Gln Asp Ser Lys Asp Ser Thr Tyr Ser Leu Ser Ser Thr
245 250 255

Leu Thr Leu Ser Lys Ala Asp Tyr Glu Lys His Lys Val Tyr Ala Cys
260 265 270

Glu Val Thr His Gln Gly Leu Ser Ser Pro Val Thr Lys Ser Phe Asn
275 280 285

Arg Gly Glu Cys
290

<210> 162
<211> 17
<212> PRT
<213> Artificial sequence

<220>
<223> myc-hexahistidine tag (SEQ ID NO: 162)

<400> 162

Gly Glu Gln Lys Leu Ile Ser Glu Glu Asp Leu His His His His His
1 5 10 15

His

<210> 163
<211> 214
<212> PRT
<213> Artificial sequence

<220>
<223> anti HER2 antibody 2, light chain

<400> 163

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205
```

Phe Asn Arg Gly Glu Cys
210

<210> 164
<211> 63
<212> PRT
<213> Artificial sequence

<220>
<223> wildt-type SH3 domain of the Fyn kinase (Fyn SH3)

<400> 164

Gly Val Thr Leu Phe Val Ala Leu Tyr Asp Tyr Glu Ala Arg Thr Glu
1 5 10 15

Asp Asp Leu Ser Phe His Lys Gly Glu Lys Phe Gln Ile Leu Asn Ser
20 25 30

Ser Glu Gly Asp Trp Trp Glu Ala Arg Ser Leu Thr Thr Gly Glu Thr
35 40 45

Gly Tyr Ile Pro Ser Asn Tyr Val Ala Pro Val Asp Ser Ile Gln
50 55 60

<210> 165
<211> 1362
<212> DNA
<213> Artificial sequence

<220>
<223> Anti-HER2 antibody 1 (heavy chain) (coding for amino acids shown in SEQ ID NO: 154)

<400> 165
gaggtgcagc tggtcgaatc tggtgggggc ctggtgcagc ctgggggctc cctgagactg 60

tcctgtgccg catccggttt tacatttacc gactacacaa tggattgggt gcgacaggca 120

cccgggaagg gtctggagtg ggtggctgac gtgaacccta attccggcgg aagcatctac 180

aaccagaggt tcaagggccg gtttactctg tctgtggaca ggagtaaaaa caccctgtat 240

ctgcagatga attccctgag agccgaagat acagctgtct actattgcgc tcgcaatctg 300

ggtccatcat tctactttga ctattggggg cagggaactc tggtgactgt ctcatccgct 360

agcacaaagg gccctagtgt gtttcctctg gctccctctt ccaaatccac ttctggtggc 420

actgctgctc tgggatgcct ggtgaaggat tactttcctg aacctgtgac tgtctcatgg 480

aactctggtg ctctgacttc tggtgtccac actttccctg ctgtgctgca gtctagtgga 540

ctgtactctc tgtcatctgt ggtcactgtg ccctcttcat ctctgggaac ccagacctac 600

atttgtaatg tgaaccacaa accatccaac actaaagtgg acaaaaaagt ggaacccaaa 660

tcctgtgaca aaacccacac ctgcccacct tgtcctgccc ctgaactgct gggaggacct 720

tctgtgtttc tgttcccccc caaaccaaag gataccctga tgatctctag aacccctgag 780
gtgacatgtg tggtggtgga tgtgtctcat gaggaccctg aggtcaaatt caactggtac 840
gtggatggag tggaagtcca caatgccaaa accaagccta gagaggaaca gtacaattca 900
acctacagag tggtcagtgt gctgactgtg ctgcatcagg attggctgaa tggcaaggaa 960
tacaagtgta aagtctcaaa caaggccctg cctgctccaa ttgagaaaac aatctcaaag 1020
gccaagggac agcctaggga accccaggtc tacaccctgc caccttcaag agaggaaatg 1080
accaaaaacc aggtgtccct gacatgcctg gtcaaaggct tctacccttc tgacattgct 1140
gtggagtggg agtcaaatgg acagcctgag aacaactaca aaacaacccc ccctgtgctg 1200
gattctgatg gctctttctt tctgtactcc aaactgactg tggacaagtc tagatggcag 1260
caggggaatg tcttttcttg ctctgtcatg catgaggctc tgcataacca ctacactcag 1320
aaatccctgt ctctgtctcc tggcaaatga tagtaaaagc tt 1362

<210> 166
<211> 657
<212> DNA
<213> Artificial sequence

<220>
<223> Anti-HER2 antibody 1 (light chain) (coding for amino acids shown in SEQ ID NO:155)

<400> 166
gatatccaga tgacccagag ccctagtagt ctgagcgcaa gcgtcgggga ccgtgtgacc 60
attacctgta aagcaagcca ggatgtgtct atcggtgtgg catggtatca gcagaagccc 120
ggcaaagccc ctaagctgct gatctactct gctagttaca gatatactgg agtcccaagt 180
cggttctcag gctccggaag cgggaccgac tttaccctga caatctcctc cctgcaaccc 240
gaggatttcg ccacatacta ttgccagcag tactacatct atccttatac attcgggcag 300
gggacaaaag tggaaatcaa acggactgtg gcggcgcctt ctgtgttcat tttcccccca 360
tctgatgaac agctgaaatc tggcactgct tctgtggtct gtctgctgaa caacttctac 420
cctagagagg ccaaagtcca gtggaaagtg gacaatgctc tgcagagtgg gaattcccag 480
gaatctgtca ctgagcagga ctctaaggat agcacatact ccctgtcctc tactctgaca 540
ctgagcaagg ctgattacga gaaacacaaa gtgtacgcct gtgaagtcac acatcagggg 600
ctgtctagtc ctgtgaccaa atccttcaat aggggagagt gctgatagta aaagctt 657

<210> 167
<211> 891
<212> DNA
<213> Artificial sequence

<220>
<223> N-terminal fusion of C12 to light chain of anti-HER2 antibody 1 (coding for amino acids shown in SEQ ID NO: 159)

<400> 167
ggggtgactc tgttcgtcgc tctgtatgat tacacttcct ataacaccag agacctgagc 60

ttccacaagg gcgagaaatt tcagatcctg aggatggagg atggagtgtg gtgggaagcc 120

cggtctctga ccacagggga gacaggttac attccttcaa actacgtcgc tcccgtggac 180

agcattcagg gtggtggggg atccggcgga ggaggaagtg gcggaggagg aagtgatatc 240

cagatgaccc agagccctag tagtctgagc gcaagcgtcg gggaccgtgt gaccattacc 300

tgtaaagcaa gccaggatgt gtctatcggt gtggcatggt atcagcagaa gcccggcaaa 360

gcccctaagc tgctgatcta ctctgctagt tacagatata ctggagtccc aagtcggttc 420

tcaggctccg gaagcgggac cgactttacc ctgacaatct cctccctgca acccgaggat 480

ttcgccacat actattgcca gcagtactac atctatcctt atacattcgg gcaggggaca 540

aaagtggaaa tcaaacggac tgtggcggcg ccttctgtgt tcatttcccc cccatctgat 600

gaacagctga aatctggcac tgcttctgtg gtctgtctgc tgaacaactt ctaccctaga 660

gaggccaaag tccagtggaa agtggacaat gctctgcaga gtgggaattc ccaggaatct 720

gtcactgagc aggactctaa ggatagcaca tactccctgt cctctactct gacactgagc 780

aaggctgatt acgagaaaca caaagtgtac gcctgtgaag tcacacatca ggggctgtct 840

agtcctgtga ccaaatcctt caatagggga gagtgctgat agtaaaagct t 891

<210> 168
<211> 1365
<212> DNA
<213> Artificial sequence

<220>
<223> Anti-HER2 antibody 2 (heavy chain) (coding for amino acids shown in SEQ ID NO: 160)

<400> 168
gaagtccagc tggtcgaatc tggtggtggc ctggtccagc ctggtggatc actgagactg 60

tcctgtgctg cttctggttt caacatcaag gacacctaca tccattgggt cagacaggca 120

cctggcaagg gactggaatg ggtcgcccga atctacccta caaacggcta cactcgctac 180

gccgactccg tcaagggacg ctttaccatc tccgccgaca cctctaaaaa caccgcctac 240

ctgcagatga atagtctgag ggccgaggat actgctgtgt actactgctc acgatgggga 300

ggcgacggct tttacgctat ggattactgg ggacagggaa ctctggtcac tgtgtctagc 360

gctagcacaa agggccctag tgtgtttcct ctggctccct cttccaaatc cacttctggt 420

ggcactgctg ctctgggatg cctggtgaag gattactttc ctgaacctgt gactgtctca 480

tggaactctg gtgctctgac ttctggtgtc cacactttcc ctgctgtgct gcagtctagt 540

ggactgtact ctctgtcatc tgtggtcact gtgccctctt catctctggg aacccagacc 600

tacatttgta atgtgaacca caaaccatcc aacactaaag tggacaaaaa agtggaaccc 660

aaatcctgtg acaaaaccca cacctgccca ccttgtcctg cccctgaact gctgggagga 720

ccttctgtgt ttctgttccc ccccaaacca aaggataccc tgatgatctc tagaacccct 780

gaggtgacat gtgtggtggt ggatgtgtct catgaggacc ctgaggtcaa attcaactgg 840

tacgtggatg gagtggaagt ccacaatgcc aaaaccaagc ctagagagga acagtacaat 900

tcaacctaca gagtggtcag tgtgctgact gtgctgcatc aggattggct gaatggcaag 960

gaatacaagt gtaaagtctc aaacaaggcc ctgcctgctc caattgagaa aacaatctca 1020

aaggccaagg gacagcctag ggaaccccag gtctacaccc tgccaccttc aagagaggaa 1080

atgaccaaaa accaggtgtc cctgacatgc ctggtcaaag gcttctaccc ttctgacatt 1140

gctgtggagt gggagtcaaa tggacagcct gagaacaact acaaaacaac ccccccctgtg 1200

ctggattctg atggctcttt ctttctgtac tccaaactga ctgtggacaa gtctagatgg 1260

cagcagggga atgtcttttc ttgctctgtc atgcatgagg ctctgcataa ccactacact 1320

cagaaatccc tgtctctgtc tcctggcaaa tgatagtaaa agctt 1365

<210> 169
<211> 657
<212> DNA
<213> Artificial sequence

<220>
<223> Anti-HER2 antibody 2 (light chain) (coding for amino acids shown in SEQ ID NO: 163)

<400> 169
gacatccaga tgacacagtc tccctcttcc ctgtccgctt ctgtgggcga tcgagtgaca 60

atcacctgta gggctagtca ggatgtgaat actgctgttg cttggtacca gcagaaacca 120

ggaaaagccc ctaaactgct gatctactct gcctcattcc tgtactctgg ggtgccttct 180

cgattcagtg gttctagatc tggcaccgat ttcacactga ccatttcttc actgcaacct 240

gaggattttg ccacctacta ctgtcagcag cactacacaa cacctcccac atttggccag 300

ggcacaaaag tggagatcaa acggaccgtg gcggcgcctt ctgtgttcat tttcccccca 360

tctgatgaac agctgaaatc tggcactgct tctgtggtct gtctgctgaa caacttctac 420

cctagagagg ccaaagtcca gtggaaagtg gacaatgctc tgcagagtgg gaattcccag 480

gaatctgtca ctgagcagga ctctaaggat agcacatact ccctgtcctc tactctgaca 540

ctgagcaagg ctgattacga gaaacacaaa gtgtacgcct gtgaagtcac acatcagggg 600

ctgtctagtc ctgtgaccaa atccttcaat aggggagagt gctgatagta aaagctt 657

<210> 170
<211> 891
<212> DNA
<213> Artificial sequence

<220>
<223> N-terminal C12 fusion to anti-HER2 antibody 2 light chain (coding

for amino acids shown in SEQ ID NO: 161)

<400> 170
ggggtgactc tgttcgtcgc tctgtatgat tacacttcct ataacaccag agacctgagc 60
ttccacaagg gcgagaaatt tcagatcctg aggatggagg atggagtgtg gtgggaagcc 120
cggtctctga ccacagggga gacaggttac attccttcaa actacgtcgc tcccgtggac 180
agcattcagg gtggtggggg atccggcgga ggaggaagtg gcggaggagg aagtgacatc 240
cagatgacac agtctccctc ttccctgtcc gcttctgtgg gcgatcgagt gacaatcacc 300
tgtagggcta gtcaggatgt gaatactgct gttgcttggt accagcagaa accaggaaaa 360
gcccctaaac tgctgatcta ctctgcctca ttcctgtact ctggggtgcc ttctcgattc 420
agtggttcta gatctggcac cgatttcaca ctgaccattt cttcactgca acctgaggat 480
tttgccacct actactgtca gcagcactac acaacacctc ccacatttgg ccagggcaca 540
aaagtggaga tcaaacggac cgtggcggcg ccttctgtgt tcattttccc cccatctgat 600
gaacagctga aatctggcac tgcttctgtg gtctgtctgc tgaacaactt ctaccctaga 660
gaggccaaag tccagtggaa agtggacaat gctctgcaga gtgggaattc ccaggaatct 720
gtcactgagc aggactctaa ggatagcaca tactccctgt cctctactct gacactgagc 780
aaggctgatt acgagaaaca caaagtgtac gcctgtgaag tcacacatca ggggctgtct 840
agtcctgtga ccaaatcctt caatagggga gagtgctgat agtaaaagct t 891